# EUROPEAN PATENT SPECIFICATION

(11) **EP 2 081 600 B1**
(45) Date of publication and mention of the grant of the patent: **05.12.2012**
(21) Application number: 07820281.9
(22) Date of filing: 18.09.2007
(51) Int. Cl.: A61K 45/06, A61P 3/04, A61P 3/10, A61P 1/10, A61P 9/00, A61P 25/16, A61P 25/28, A61P 37/02, A61K 31/13, A61K 31/4045

(54) **Combination of the NMDA- receptor ligand memantine and a compound with 5-HT6 receptor affinity**
Kombination des NMDA-rezeptor-ligand Memantine und einer Verbindung mit 5-HT6-rezeptor-Affinität
Combinaison du ligand de récepteur NMDA memantine et d'un composé présentant une affinité au récepteur 5-HT6

(30) Priority: 19.09.2006 EP 06384014
(43) Date of publication of application: 29.07.2009
(73) Proprietor: Laboratorios del. Dr. Esteve, S.A., 08041 Barcelona (ES)
(72) Inventor: BUSCHMANN, Helmut Heinrich, 08960 Sant Just Desvern - Barcelona (ES); CODONY-SOLER, Xavier, 08301 Mataró - Barcelona (ES)
(74) Representative: ABG Patentes, S.L.
(86) International application number: PCT/EP2007/059816
(87) International publication number: WO 2008/034815

(56) References cited:
- EP-A- 1 676 841
- WO-A-03/042175
- WO-A-03/084952
- WO-A-2006/069807
- KING M V ET AL: "5-HT6 receptor antagonists reverse delay-dependent deficits in novel object discrimination by enhancing consolidation - an effect sensitive to NMDA receptor antagonism" NEUROPHARMACOLOGY, vol. 47, no. 2, August 2004 (2004-08), pages 195-204, XP002423399 ISSN: 0028-3908
- "S.28.05 The 5-HT6 receptor: memories are made of this" EUROPEAN NEUROPSYCHOPHARMACOLOGY, ELSEVIER SCIENCE PUBLISHERS BV, AMSTERDAM, NL, vol. 15, 2005, page S357, XP005556554 ISSN: 0924-977X

## Description

### FIELD OF THE INVENTION

The present invention relates to an active substance combination comprising at least one compound with 5-HT₆ receptor affinity, and at least one N-methyl-D-aspartate-receptor ligand (NMDA-receptor ligand), a medicament comprising said active substance combination, and the use of said active substance combination for the manufacture of a medicament.

### BACKGROUND

Cognitive and/or degenerative brain disorders are characterized clinically by progressive loss of memory, cognition, reasoning, judgement and emotional stability that gradually leads to profound mental deterioration and ultimately death. In an example of such disorders, Alzheimer's disease is a common cause of progressive mental failure (dementia) in aged humans and is believed to represent the fourth most common medical cause of death in the United States. In particular, Alzheimer's disease is associated with degeneration of cholinergic neurons in the basal forebrain that play a fundamental role in cognitive functions, including memory. Cognitive and/or degenerative brain disorders have been observed in varied races and ethnic groups world-wide and present a major public health problem. These diseases are currently estimated to affect about two to three million individuals in the United States alone and the occurrence will increase world-wide as the human life span increases.

Cognitive and/or degenerative brain disorders are incurable with presently used medications, however, the symptoms of these disorders seem to be possibly alleviated by using compounds such as memantine.

Whereas known compounds which act as NMDA-receptor ligands are generally effective for treating disorders related to NMDA-receptors such as cognitive disorders, in particular for treating Alzheimer's disease, in some instances they show undesirable side effects. Specifically, many of these compounds that have been tested in humans can cause potentially serious side effects such as gastrointestinal complications including insomnia, restlessness, headache, akathisia, fatigue, nausea, emesis, ulcers, constipation, flatulence, diarrhea, hypertension, respiratory depression and psychological and physical dependence.

Therefore, there is a need to provide a medicament suitable for the prophylaxis and/or treatment of disorders related to NMDA-receptors and to 5-HT₆ receptors, which preferably does not show the undesired side effects of the conventional compounds which act as NMDA-receptor ligands, or at least less frequent and/or less pronounced.

### BRIEF DESCRIPTION OF THE INVENTION

The authors of the present invention have developed a medicament suitable for the prophylaxis and/or treatment of cognitive disorders, in particular for treating Alzheimer's disease, which does not show, or at least reduced significantively, the undesired side effects mentioned above of the conventional medicaments.

Therefore, a first aspect of the present invention relates to an active substance combination comprising:
(A) at least one compound with 5-HT₆ receptor affinity selected from the group consisting of a compound of formula (If), a compound of formula (Ih) and a compound of formula (Ik) as defined here with, and
(B) at least one NMDA-receptor ligand, which is memantine.

It has surprisingly been found that the compounds with 5-HT₆ receptor affinity and the compounds which act as NMDA-receptor ligands show a synergistic effect in their pharmacological activities. Consequently, the dose of the corresponding compounds may be reduced in comparison to the dose necessary for an individual administration of said compounds.

According to the invention it has also been found that the action of a NMDA-receptor antagonist potentiates the action of the compound with 5-HT₆ receptor affinity, so the combination of a NMDA-receptor antagonist and a compound with 5-HT₆ receptor affinity for use in the treatment of disorders that are related to NMDA-receptors, and to 5-HT₆ receptors may result in a faster onset of action and an increased success rate. The invention therefore particularly resides in the combined action of a NMDA-receptor compound, particularly an antagonist, and a compound with 5-HT₆ receptor affinity, or the dual action of a substance possessing both NMDA-receptor antagonist activity and 5-HT₆ receptor affinity, for the treatment of disorders that are related to NMDA-receptors, and/or to 5-HT₆ receptors.

In another aspect, the present invention relates to a medicament comprising the active substance combination as defined above and optionally one or more pharmacologically acceptable adjuvants.

A third aspect of the invention refers to a medicament as defined above, for simultaneous NMDA-receptor inhibition and 5-HT6-receptor regulation.

Also, another aspect of the invention relates to the use of the active substance combination in the manufacture of a medicament for simultaneous NMDA-receptor inhibition and 5-HT6-receptor regulation.

Another aspect of the invention refers to a pharmaceutical formulation which comprises the active substance combination and optionally one or more pharmacologically acceptable adjuvants.

### BRIEF DESCRIPTION OF THE DRAWINGS

Figure 1 shows the results obtained for the novel object discrimination paradigm trial in rats, when they have been administered intraperitoneally with different doses of memantine (0, 5, 10, 15 and 20 mg/kg).
Figure 2 shows the results obtained for the novel object discrimination paradigm trial in rats, when they have been administered intraperitoneally with the compound 841 alone, with memantine alone, or with a combination of compound 841 and memantine.

### DETAILED DESCRIPTION OF THE INVENTION

In the treatment of cognitive disorders, the effect on e.g. memory or novel object discrimination is significantly greater in the group that is treated with a combination of at least one NMDA-receptor antagonist and at least one compound with 5-HT₆ receptor affinity than in the group that is treated with at least one NMDA-receptor antagonist or at least one compound with 5-HT₆ receptor affinity exclusively.

Also there is an indication that in the treatment of depression, the effect on the symptoms - in an animal model - is more pronounced in the group that is treated with a combination of at least one NMDA-receptor antagonist and at least one compound with 5-HT₆ receptor affinity than in the group that is treated with at least one NMDA-receptor antagonist or at least one compound with 5-HT₆ receptor affinity exclusively.

In one embodiment of the present invention the binding of compounds present as component (A) to the 5-HT₆-receptor is determined by a Kᵢ value of less than 7000 nM, particularly preferably of less than 6500 nM, more particularly preferably of less than 200 nM, more Particularly preferably of less than 100 nM.

In one embodiment of the present invention, the compounds present as component (B) act as NMDA-receptor antagonists. The NMDA-receptor antagonist of the invention may be any ligand that binds to and inhibits the NMDA-receptor, thereby resulting in a biological response. The potential of a given substance to act as a NMDA-receptor antagonist may be determined using standard in vitro binding assays and/or standard in vivo functionality tests.

In one embodiment of the present invention the binding of compounds present as component (B) to the NMDA-receptor is determined by an EC₅₀ or IC₅₀ value of less than 300 µM, preferably less than 100 µM, when determined in a standard functionality assay using a mouse, rat, or human NMDA-receptor ion channel.

In one embodiment the NMDA-receptor antagonist blocks the NMDA receptor at the PCP binding site

In an embodiment of the present invention as component (A) at least one compound is present, which is selected from the group consisting of compounds of general formula (Ih) 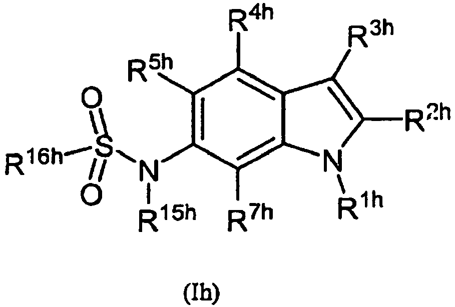 wherein
R^{1h} represents a hydrogen atom or a -(CH₂)ᵢₙₕ-NR^{13h}R^{14h} radical,
R^{2h}, and R^{7h} each represent hydrogen,
R^{3h} represents a hydrogen atom, 1-methyl-piperidin-4-yl or a -(CH₂)ₙₕ-NR^{13h}R^{14h} moiety with nh = 0, 1 or 2,
R^{4h} represents chlorine, bromine or a hydrogen atom,
R^{5h} represents -C(=O)-O-C₂H₅ or a hydrogen atom,
R^{15h} represents hydrogen or a -S(=O)₂-R^{16h} moiety,
R^{13h} and R^{14h}, identical or different, each represent methyl, ethyl, isopropyl or n-propyl, more preferably methyl,
   or
R^{13h} and R^{14h}, together with the bridging nitrogen atom form a 5- or 6-membered heterocyclic ring, more preferably form a pyrrolidine ring or a piperidine ring
   and
R^{16h} represents an aryl or heteroaryl radical selected from the group consisting of phenyl, naphthyl, thiophenyl, benzo[b]furanyl, benzo[b]thiophenyl and imidazo[2,1-b]thiazolyl which may be substituted by 1, 2 or 3 substituents selected from the group consisting of chlorine, methyl, phenyl and -O-phenyl and/or which may be bonded via a C₁₋₂ alkylene group,
and mh is 0, 1 or 2,
optionally in form of one of its stereoisomers, preferably enantiomers or diastereomers, a racemate or in form of a mixture of at least two of its stereoisomers, preferably enantiomers and/or diastereomers, in any mixing ratio, or a salt thereof, preferably a corresponding, physiologically acceptable salt thereof, or a corresponding solvate thereof.

More particularly preferred compounds of general formula (Ih) are those selected from the group consisting of:
[788] N-[1-(2-Dimethylaminoethyl)-1H-indol-6-yl]-5-chloro-3-methylbenzo[b]thiophene-2-sulfonamide,
[789] N-[1-(2-Dimethylaminoethyl)-1H-indol-6-yl]-naphthalene-2-sulfonamide,
[790] N-[1-(2-Dimethylaminoethyl)-1H-indol-6-yl]-naphthalene-1-sulfonamide,
[791] N-[1-(2-Dimethylaminoethyl)-1H-indol-6-yl]-6-chloroimidazo[2,1-b]thiazole-5-sulfonamide,
[792] N-[1-(2-Dimethylaminoethyl)-1H-indol-6-yl]-4-phenylbenzenesulfonamide,
[793] N-[1-(2-Dimethylaminoethyl)-1H-indol-6-yl]-2-(naphthalene-1-yl)-ethanesulfonamide,
[794] N-[1-(2-Dimethylaminoethyl)-1H-indol-6-yl]-4-phenoxybenzenesulfonamide,
[795] N-[1-(2-Dimethylaminoethyl)-1H-indol-6-yl]-3,5-dichlorobenzenesulfonamide,
[796] 5-Chloro-3-methyl-N-[1-[2-(pyrrolidin-1-yl)ethyl-1H-indol-6-yl]-benzo[b]thiophene-2-sulfonamide,
[797] N-(1-[2-(Pyrrolidin-1-yl)ethyl]-1H-indol-6-yl]-napthyl-2-sulfonamide,
[798] N-[1-[2-Pyrrolidin-1-yl]ethyl]-1H-indol-6-yl]-naphthalene-1-sulfonamide,
[799] 6-Chloro-N-[1-[2-(pyrrolidin-1-yl)ethyl]-1H-indol-6-yl]-imidazo[2,1-b]thiazole-5-sulfonamide,
[800] 4-Phenyl-N-(1-(2-(pyrrolidin-1-yl)ethyl)-1H-indol-6-yl)-benzenesulfonamide
[801] 2-(Naphth-1-yl)-N-(1-(2-(pyrrolidin-1-yl)ethyl)-1H-indol-6-yl)-ethansulfonamide,
[802] 4-Phenoxy-N-(1-(2-(pyrrolidin-1-yl)ethyl)-1H-indol-6-yl)-benzenesulfonamide
[803] 3,5-Dichloro-N-(1-(2-(pyrrolidin-1-yl)-1H-indol-6-yl)-benzenesulfonamide,
[804] 5-chloro-N-(3-(2-(diethylamino)ethyl)-1H-indol-6-yl)-3-methylbenzo[b]thiophene-2-sulfonamide,
[805] N-(3-(2-(diethylamino)ethyl)-1H-indol-6-yl)naphthalene-2-sulfonamide,
[806] N-(3-(2-(diethylamino)ethyl)-1H-indol-6-yl)naphthalene-1-sulfonamide,
[807] 6-chloro-N-(3-(2-(diethylamino)ethyl)-1H-indol-6-yl)imidazo[2,1-b]thiazole-5-sulfonamide,
[808] N-(3-(2-(diethylamino)ethyl)-1H-indol-6-yl)-4-phenylbenzenesulfonamide,
[809] N-(3-(2-(diethylamino)ethyl)-1H-indol-6-yl)-4-phenoxybenzenesulfonamide,
[810] 3,5-dichloro-N-(3-(2-(diethylamino)ethyl)-1H-indol-6-yl)benzenesulfonamide,
[811] 4,5-dichloro-N-(3-(2-(diethylamino)ethyl)-1H-indol-6-yl)thiophene-2-sulfonamide,
[812] 5-chloro-N-(3-(2-(diethylamino)ethyl)-1H-indol-6-yl)naphthalene-1-sulfonamide,
[813] 5-chloro-N-(3-(2-(dimethylamino)ethyl)-1H-indol-6-yl)-3-methylbenzo[b]thiophene-2-sulfonamide,
[814] N-(3-(2-(dimethylamino)ethyl)-1H-indol-6-yl)naphthalene-2-sulfonamide,
[815] N-(3-(2-(dimethylamino)ethyl)-1H-indol-6-yl)naphthalene-1-sulfonamide,
[816] 6-chloro-N-(3-(2-(dimethylamino)ethyl)-1H-indol-6-yl)imidazo[2,1-b]thiazole-5-sulfonamide,
[817] N-(3-(2-(dimethylamino)ethyl)-1H-indol-6-yl)-4-phenylbenzenesulfonamide,
[818] N-(3-(2-(dimethylamino)ethyl)-1H-indol-6-yl)-2-(naphthalen-1-yl)ethanesulfonamide.
[819] N-(3-(2-(dimethylamino)ethyl)-1H-indol-6-yl)-4-phenoxybenzenesulfonamide,
[820] 3,5-dichloro-N-(3-(2-(dimethylamino)ethyl)-1H-indol-6-yl)benzenesulfonamide,
[821] 4,5-dichloro-N-(3-(2-(dimethylamino)ethyl)-1H-indol-6-yl)thiophene-2-sulfonamide,
[822] 5-chloro-N-(3-(2-(dimethylamino)ethyl)-1H-indol-6-yl)naphthalene-1-sulfonamide,
[823] 6-bis(6-chloroimidazo[2,1-b]thiazol-5-ylsulfonyl)amino-3-(2-(dimethylamino)ethyl)-1H-indole,
[824] 6-bis(3,5-dichlorobenzenesulfonyl)amino-3-(2-(dimethylamino)ethyl)-1H-indole,
[825] 6-bis(4,5-dichlorothiophene-2-sulfonyl)amino-3-(2-(dimethylamino)ethyl)-1H-indole,
[826] 6-bis(5-chloro-3-methylbenzo[b]thiophene-2-sulfonyl)amino-3-(2-(dimethylamino)-1-ethoxyethyl)-1H-indole
[827] Ethyl 6-(5-chloro-3-methylbenzo[b]thiophene-2-sulfonamido)-3-(1-methylpiperidin-4-yl)-1H-indole-5-carboxylate,
[828] N-(4-bromo-3-(1-methylpiperidin-4-yl)-1H-indol-6-yl)naphthalene-1-sulfonamide,
[829] N-(7-bromo-3-(2-(dimethylamino)ethyl)-1H-indol-5-yl)benzofuran-2-sulfonamide,
[830] N-(7-methoxy-3-(2-(pyrrolidin-1-yl)ethyl)-1H-indol-5-yl)benzo[c][1,2,5]thiadiazole-4-sulfonamide,
[831] N-(7-methoxy-3-(2-(pyrrolidin-1-yl)ethyl)-1H-indol-5-yl)naphthalene-2-sulfonamide and
[832] 6-chloro-N-(7-methoxy-3-(2-(pyrrolidin-1-yl)ethyl)-1H-indol-5-yl)imidazo[2,1-b]thiazole-5-sulfonamide;
and their corresponding salts and solvates.

The compounds of general formula (Ih) may be prepared according to the disclosure of WO 2005/013976 and WO 2006/024535.

In another embodiment of the present invention component (A) is selected from the group consisting of compounds of general formula (Ik) 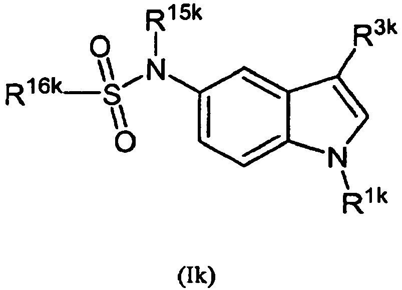 wherein
nk represents 0, 1, 2, 3 or 4;
R^{1k} represents hydrogen,
R^{3k} represents a -NR^{13k}R^{14k} moiety or a moiety selected from the group consisting of 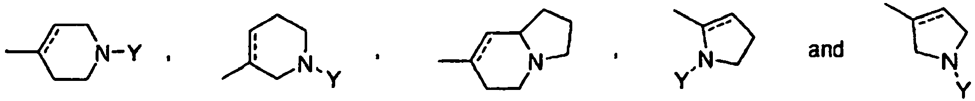 wherein, if present, the dotted line represents an optional chemical bond and Y represents hydrogen, a methyl group or an ethyl group,
R^{15k} represents hydrogen, a methyl group or an ethyl group,
R^{13k} and R^{14k}, identical or different, represent a methyl group, an ethyl group, an n-propyl group, an isopropyl group, an n-butyl group, an isobutyl group, a sec-butyl group, or a tert-butyl group, or
R^{13k} and R^{14k} together with the bridging nitrogen atom form a moiety selected from the group consisting of 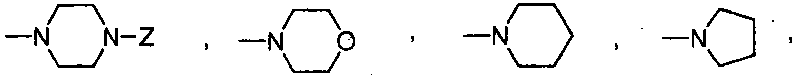 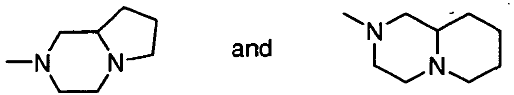 wherein Z represents hydrogen, a methyl group or an ethyl group,
R^{16k} represents a moiety selected from the group consisting of 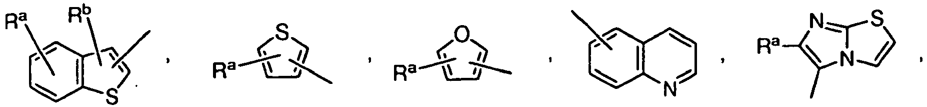 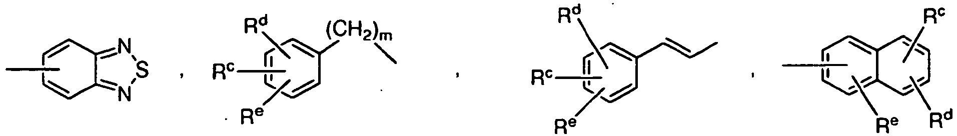 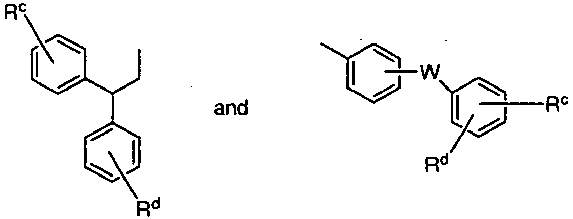 wherein
R^{a} and R^{b} are each independently selected from the group consisting of hydrogen, fluorine, chlorine, bromine, methyl, ethyl, pyridinyl, thiophenyl and furyl,
R^{c}, R^{d} and R^{e} are each independently selected from the group consisting of hydrogen, fluorine, chlorine, bromine, methyl, ethyl, methoxy, ethoxy and -CF₃,
W represents a single chemical bond between the two rings, a CH₂-group, O, S or a NR^{f}-moiety, wherein R^{f} is hydrogen, methyl or ethyl,
m is 0, 1, 2, 3 or 4;
optionally in form of one of its stereoisomers, preferably enantiomers or diastereomers, a racemate or in form of a mixture of at least two of its stereoisomers, preferably enantiomers and/or diastereomers, in any mixing ratio, or a salt thereof, preferably a corresponding, physiologically acceptable salt thereof, or a corresponding solvate thereof.

Particularly preferred compounds of general formula (Ik) are those selected from the group consisting of:
[833] N-[3-(2-diethylaminoethyl)-1H-indol-5-yl]-5-chloro-3-methylbenzo[b]thiophene-2-sulphonamide,
[834] N-[3-(2-diethylaminoethyl)-1H-indol-5-yl]naphthalene-1-sulphonamide,
[835] Hydrochloride N-[3-(2-diethylaminoethyl)-1*H*-indol-5-yl]naphthalene-1-sulphonamide,
[836] N-[3-(2-diethylaminoethyl)-1*H*-indol-5-yl]-3,5-dichlorobenzenesulphonamide,
[837] N-[3-(2-diethylaminoethyl)-1*H*-indol-5-yl]-4-phenylbenzenesulphonamide,
[838] N-[3-(2-diethylaminoethyl)-1*H*-indol-5-yl]-5-chlorothiophene-2-sulphonamide,
[839] N-[3-(2-dimethylaminoethyl)-1*H*-indol-5-yl]-5-chloro-3-methylbenzo[b]thiophene-2-sulphonamide,
[840] N-[3-(2-dimethylaminoethyl)-1*H*-indol-5-yl]naphthalene-1-sulphonamide,
[841] N-[3-(2-dimethylamino-ethyl)-1*H*-indol-5-yl]-6-chloroimidazo[2,1-b]thiazol-5-sulphonamide,
[842] N-[3-(1-methylpiperidin-4-yl)-1*H*-indol-5-yl]-5-chloro-3-methylbenzo[b]thiophene-2-sulphonamide,
[843] N-[3-(1-methylpiperidin-4-yl)-1*H*-indol-5-yl]-5-chloro-3-methylbenzo[b]thiophene-2-sulphonamide hydrochloride,
[844] N-[3-(1-methylpiperidin-4-yl)-1*H*-indol-5-yl]naphthalene-1-sulphonamide,
[845] N-[3-(1-methylpiperidin-4-yl)-1*H*-indol-5-yl]naphthalene-1-sulphonamide hydrochloride,
[846] N-[3-(1-methylpiperidin-4-yl)-1*H*-indol-5-yl]-5-chlorothiophene-2-sulphonamide,
[847] N-[3-(1-methylpiperidin-4-yl)-1*H*-indol-5-yl]-4-phenylbenzenesulphonamide,
[848] N-[3-(1-methylpiperidin-4-yl)-1*H*-indol-5-yl]quinoline-8-sulphonamide,
[849] N-[3-(2-diethylaminoethyl)-1*H*-indol-5-yl]naphthalene-2-sulphonamide,
[850] N-[3-(1-methyl-1,2,3,6-tetrahydropylidin-4-yl)-1*H*-indol-5-yl]naphthalene-1-sulphonamide,
[851] N-[3-(4-methylpiperazin-1-yl)methyl-1*H*-indol-5-yl]-5-chloro-3-methylbenzo[b]thiophene-2-sulphonamide,
[852] N-[3-(2-dimethylaminoethyl)-1*H*-indol-5-yl]-5-(2-pyridil)thiophene-2-sulphonamide,
[853] N-[3-(2-dimethylaminoethyl)-1*H*-indol-5-yl]-2,1,3-benzothiadiazol-4-sulphonamide,
[854] N-[3-(2-dimethylaminoethyl)-1*H*-indol-5-yl]quinoline-8-sulphonamide,
[855] N-[3-(2-dimethylaminoethyl)-1*H*-indol-5-yl]-5-chloronaphthalene-2-sulphonamide,
[856] N-[3-(2-dimethylaminoethyl)-1*H*-indol-5-yl]-4-phenoxybenzenesulphonamide,
[857] N-[3-(2-dimethylaminoethyl)-1*H*-indol-5-yl]-4-phenylbenzenesulphonamide,
[858] N-[3-(2-diethylaminoethyl)-1*H*-indol-5-yl]-N-ethyl-naphthalene-2-sulphonamide,
[859] N-{3-[2-(morpholin-4-yl)ethyl]-1H-indol-5-yl}-5-chloro-3-methylbenzo[b]thiophene-2-sulphonamide,
[860] N-{3-[2-(morpholin-4-yl)ethyl]-1H-indol-5-yl}naphthalene-1-sulphonamide,
[861] N-[3-(2-diethylaminoethyl)-1H-indol-5-yl]naphthalene-2-sulphonamide,
[862] N-[3-dimethylaminomethyl-1H-indol-5-yl]-5-chloro-3-methylbenzo[b]thiophene-2-sulphonamide,
[863] N-[3-(2-dipropylaminoethyl)-1H-indol-5-yl]naphthalene-1-sulphonamide,
[864] N-[3-(2-dipropylaminoethyl)-1H-indol-5-yl]-5-chloro-3-methylbenzo[b]thiophene-2-sulphonamide,
[865] N-[3-(2-dibutylaminoethyl)-1H-indol-5-yl]-5-chloro-3-methylbenzo[b]thiophene-2-sulphonamide,
[866] N-[3-(2-dibutylaminoethyl)-1H-indol-5-yl]naphthalene-1-sulphonamide,
[867] N-[3-(2-diethylaminoethyl)-1H-indol-5-yl]-5-chloronaphthalene-1-sulphonamide,
[868] N-[3-(2-diethylaminoethyl)-1H-indol-5-yl]-trans-β-styrenesulphonamide,
[869] N-[3-(4-methylpiperazin-1-yl)methy]-1*H*-indol-5-yl]-trans-β-styrenesulphonamide,
[870] N-[3-(octahydroindolizin-7-yl)-1H-indol-5-yl]-5-chloro-3-methylbenzo[b]thiophene-2-sulphonamide,
[871] N-[3-(2-diethylaminoethyl)-1*H*-indol-5-yl]-6-chloroimidazo[2,1-b]thiazol-5-sulphonamide,
[872] N-{3-[2-(morpholin-4-yl)ethyl]-1H-indol-5-yl}naphthalene-2-sulphonamide,
[873] N-[3-(4-methylpiperazin-1-yl)methyl-1*H*-indol-5-yl]-a-toluenesulphonamide,
[874] N-[3-(3-diethylaminopropyl)-1H-indol-5-yl]naphthalene-2-sulphonamide,
[875] N-[3-(3-diethylaminopropyl)-1H-indol-5-yl]-5-chloro-3-methylbenzo[b]thiophene-2-sulphonamide,
[876] N-{3-[2-(pyrrolidin-1-yl)ethyl]-1H-indol-5-yl}-5-chloro-3-methylbenzo[b]thiophene-2-sulphonamide,
[877] N-{3-[2-(pyrrolidin-1-yl)ethyl]-1H-indol-5-yl}naphthalene-1-sulphonamide,
[878] N-{3-[2-(pyrrolidin-1-yl)ethyl]-1H-indol-5-yl}naphthalene-2-sulphonamide,
[879] N-[3-(2-dipropylaminoethyl)-1H-indol-5-yl]naphthalene-2-sulphonamide,
[880] N-[3-(2-dimethylaminoethyl)-1H-indol-5-yl]-5-chloronaphthalene-1-sulphonamide,
[881] N-[3-(2-dimethylaminoethyl)-1H-indol-5-yl]naphthalene-2-sulphonamide,
[882] N-{3-[2-(morpholin-4-yl)ethyl]-1H-indol-5-yl}quinoline-8-sulphonamide,
[883] N-{3-[2-(morpholin-4-yl)ethyl]-1H-indol-5-yl}-4-phenylbenzenesulphonamide,
[884] N-[3-(4-methylpiperazin-1-yl)ethyl-1*H*-indol-5-yl]naphthalene-2-sulphonamide and
[885] N-[3-(4-methylpiperazin-1-yl)ethyl-1*H*-indol-5-yl]-5-chloronaphthalene-1-sulphonamide;
and their corresponding salts and solvates.

The compounds of general formula (Ik) may be prepared according to the disclosure of WO 2004/098588.

In another embodiment of the present invention as component (A) at least one compound is present which is selected from the group consisting of tetrahydroisoquinoline-derived sulfonamide compounds of general formula (If) 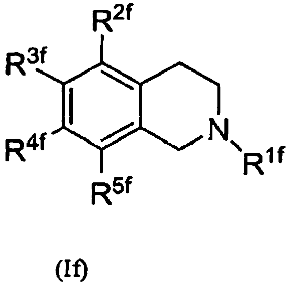
R^{1f} represents a hydrogen atom; a -C(=O)-OR^{37f} moiety; a radical selected from the group consisting of methyl, ethyl, n-propyl, isopropyl, n-butyl, sec-butyl, isobutyl, tert-butyl, n-pentyl, n-hexyl, -CH₂-NH₂, -CH₂-NH-CH₃, -CH₂-N(CH₃)₂, -CH₂-N(C₂H₅)₂, -CH₂-NH-C₂H₅, -CH₂-CH₂-NH₂, -CH₂-CH₂-NH-CH₃, -CH₂-CH₂-N(CH₃)₂, - CH₂-CH₂-N(C₂H₅)₂, -CH₂-CH₂-NH-C₂H₅, -CH₂-CH₂-CH₂-NH-CH₃, -CH₂-CH₂-CH₂-N(CH₃)₂, -CH₂-CH₂-CH₂-N(C₂H₅)₂ and -CH₂-CH₂-CH₂-NH-C₂H₅; or a (hetero)cycloaliphatic radical selected from the group consisting of imidazolidinyl, aziridinyl, azetidinyl, pyrrolidinyl, piperidinyl, morpholinyl, thiomorpholinyl, piperazinyl, pyrazolidinyl and azepanyl, which may be bonded via a -(CH₂)_{1,2 or 3}- group and which may be unsubstituted or optionally substituted with 1, 2, 3, 4 or 5 substituent(s) independently selected from the group consisting of methyl, ethyl, n-propyl, isopropyl, n-butyl, tert-butyl, sec-butyl and isobutyl;
R^{2f}, R^{3f}, R^{4f} and R^{5f}, independently of one another, each represent a hydrogen atom; F, Cl, Br, I, -NO₂; -O-CH₃; -O-C₂H₅; -O-CF₃; -O-CFH₂; -O-CF₂H; -O-CH₂-CF₃; -O-CF₂-CF₃; -S-CH₃; -S-C₂H₅; -S-CF₃; -S-CFH₂; -S-CF₂H; -S-CH₂-CF₃; -S-CF₂-CF₃; - N(R^{11f})-S(=O)₂-R^{12f}; or a radical selected from the group consisting of methyl, ethyl, n-propyl, isopropyl, tert-butyl, -CF₃, -CFH₂, -CF₂H, -CH₂-CF₃ and -CF₂-CF₃;
with the proviso that at least one of the substituents R^{2f}, R^{3f}, R^{4f} and R^{5f} represents a - N(R^{11f})-S(=O)₂-R^{12f} moiety;
R^{11f} represents a hydrogen atom, -S(=O)₂-R^{12f} or an alkyl radical selected from the group consisting of methyl, ethyl, n-propyl, isopropyl, n-butyl, sec-butyl, isobutyl and tert-butyl;
R^{12f} represents a phenyl radical of general formula (Af), 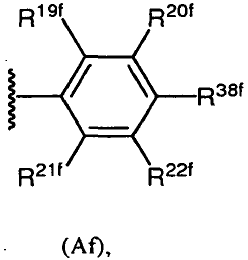 wherein
R^{19f}, R^{20f}, R^{21f}, R^{22f} and R^{38f}, independently of one another, each represent a hydrogen atom; F, Cl, Br, I, -NO₂; -NH₂; -SH; -OH; -CN; -C(=O)-OH; -C(=O)-H; -C(=O)-CH₃; - C(=O)-C₂H₅; -O-CH₃; -O-C₂H₅; -O-CF₃; -O-CFH₂; -O-CF₂H; -O-CH₂-CF₃; -O-CF₂-CF₃; -S-CH₃; -S-C₂H₅; -S-CF₃; -S-CFH₂; -S-CF₂H; -S-CH₂-CF₃; -S-CF₂-CF₃; -C(=O)-OCH₃; -C(=O)-OC₂H₅; methyl, ethyl, n-propyl, isopropyl, n-butyl, isobutyl, sec-butyl, tert-butyl, -CF₃, -CF₂H, -CFH₂, -CH₂-CF₃ and -CF₂-CF₃;
a radical selected from the group consisting of naphthyl, [1,3]-benzodioxolyl, [1,4]-benzodioxanyl, benzo[b]furanyl, benzo[b]thiophenyl, benzo[2,1,3]thiadiazolyl, [1,2,3]-benzothiadiazolyl, [2,1,3]-benzoxadiazolyl, [1,2,3]-benzoxadiazolyl, benzoxazolyl, benzothiazolyl, benzisoxazolyl, benzisothiazolyl and imidazo[2,1-b]thiazolyl, which may be unsubstituted or optionally substituted with 1, 2, 3, 4 or 5 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, F, Cl, Br, I, -CN, - CF₃, -OCF₃, -SCF₃, -CF₂H and -CFH₂;
or a radical selected from the group consisting of pyridinyl, pyrrolyl, imidazolyl, pyrazolyl, triazolyl, pyridazinyl, pyrimidinyl and pyrazinyl, which may be unsubstituted or optionally substituted with 1, 2, 3, 4 or 5 substituent(s) independently selected from the group consisting of F, Cl, Br, I, -NO₂; methyl, ethyl, n-propyl, isopropyl, n-butyl, isobutyl, sec-butyl, tert-butyl, -CF₃, -CF₂H, -CFH₂, -CH₂-CF₃ and -CF₂-CF₃;
and R^{37f} represents a radical selected from the group consisting of methyl, ethyl, n-propyl, isopropyl, n-butyl, sec-butyl, isobutyl, tert-butyl, n-pentyl, n-hexyl, fluorenyl, fluorenylmethyl, phenyl, benzyl and naphthyl;
optionally in form of one of its stereoisomers, preferably enantiomers or diasteromers, a racemate or in form of a mixture of at least two of its stereoisomers, preferably enantiomers and/or diastereomers, in any mixing ratio, or a salt thereof, or a corresponding solvate thereof.

Particularly preferred compounds of general formula (If) are those selected from the group consisting of
[1114] N-(1,2,3,4-tetrahydroisoquinolin-6-yl)naphthalene-1-sulfonamide hydrochloride,
[1115] 2,2-dimethyl-6-(N-methylnaphthalene-1-sulfonamido)-1,2,3,4-tetrahydroisoquinolinium iodide,
[1116] N-(2-methyl-1,2,3,4-tetrahydroisoquinolin-6-yl)naphthalene-1-sulfonamide hydrochloride,
[1117] 5-chloro-3-methyl-N-(1,2,3,4-tetrahydroisoquinolin-6-yl)benzo[b]thiophene-2-sulfonamide hydrochloride,
[1118] 5-chloro-3-methyl-N-(2-methyl-1,2,3,4-tetrahydroisoquinolin-6-yl)benzo[b]thiophene-2-sulfonamide hydrochloride,
[1119] 4-methyl-N-(1,2,3,4-tetrahydroisoquinolin-6-yl)naphthalene-1-sulfonamide hydrochloride,
[1120] 4-methyl-N-(2-methyl-1,2,3,4-tetrahydroisoquinolin-6-yl)naphthalene-1-sulfonamide hydrochloride,
[1121] N-(1,2,3,4-tetrahydroisoquinolin-6-yl)naphthalene-2-sulfonamide hydrochloride,
[1122] N-(2-methyl-1,2,3,4-tetrahydroisoquinolin-6-yl)naphthalene-2-sulfonamide hydrochloride,
[1123] 6-chloro-N-(1,2,3,4-tetrahydroisoquinolin-6-yl)imidazo[2,1-b]thiazole-5-sulfonamide hydrochloride,
[1124] 2-methoxy-5-methyl-N-(1,2,3,4-tetrahydroisoquinolin-6-yl)benzenesulfonamide hydrochloride,
[1125] N-(1,2,3,4-tetrahydroisoquinolin-6-yl)pyridine-3-sulfonamide dihydrochloride,
[1126] 6-(naphthalene-1-sulfonylamino)-3,4-dihydro-1H-isoquinoline-2-carboxylic acid tert-butyl ester,
[1127] 6-(5-chloro-3-methyl-benzo[b]thiophene-2-sulfonylamino)-3,4-dihydro-1H-isoquinoline-2-carboxylic acid tert-butyl ester,
[1128] 6-(4-methyl-naphthalene-1-sulfonylamino)-3,4 -dihydro-1H-isoquinoline-2-carboxylic acid tert-butyl ester,
[1129] 6-(naphthalene-2-sulfonylamino)-3,4-dihydro-1H-isoquinoline-2-carboxylic acid tert-butyl ester,
[1130] 6-(2-methoxy-5-methyl-benzenesulfonylamino) -3,4-dihydro-1H-isoquinoline-2-carboxylic acid tert-butyl ester and
[1131] 6-(pyridine-3-sulfonylamino)-3,4-dihydro-1H-isoquinoline-2carboxylic acid tert-butyl ester;
optionally in form of one of its stereoisomers, preferably enantiomers or diasteromers, a racemate or in form of a mixture of at least two of its stereoisomers, preferably enantiomers and/or diastereomers, in any mixing ratio, or a salt thereof, or a corresponding solvate thereof.

The compounds of general formula If are prepared by a process, wherein at least one compound of general formula IV, 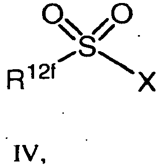 wherein R^{12f} has the meaning given above and X represents a leaving group, preferably a halogen atom, particularly preferably a chlorine atom, is reacted with at least one compound of general formula V, 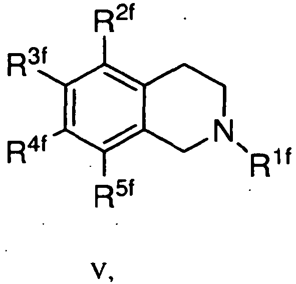 wherein R^{1f} to R^{5f} have the meaning given above, with the proviso that at least one substituent of the group consisting of R^{2f}, R^{3f}, R^{4f} and R^{5f} represents a -N(H)(R^{11f}) moiety, wherein R^{11f} has the meaning given above, or a protected derivative thereof, in a reaction medium, preferably in a reaction medium selected from the group consisting of pyridine, chloroform, dichloromethane, tetrahydrofurane and mixtures thereof, preferably in the presence of at least one base, more preferably in the presence of at least one base selected from the group consisting of triethylamine, diisopropylethylamine and diethylisopropylamine, preferably at a temperature between 0 °C and 30 °C.

If the substituted tetrahydroisoquinoline compounds of general formula If are obtained in form of a mixture of stereoisomers, particularly enantiomers or diastereomers, said mixtures may be separated by standard procedures known to those skilled in the art, e.g. chromatographic methods or crystallization with chiral reagents.

The substituted tetrahydroisoquinoline compounds of general formula If and in each case stereoisomers thereof may be obtained in form of a corresponding salt according to methods well known to those skilled in the art, e.g. by reacting said compound with at least one inorganic and/or organic acid, preferably in a suitable reaction medium. Suitable reaction media include, for example, any of the ones given above.

Compounds of general formula IV are in most cases commercially available or may be prepared by processes known to those skilled in the art.

Compounds of general formula V are in most cases commercially available or may be prepared by processes known to those skilled in the art.

In particular, 1,2,3,4-tetrahydroisoquinoline compounds with an amino group in position 5 can be prepared starting from 5-nitro-1,2,3,4-tetrahydroisoquinoline compounds. A process for the preparation of the latter compounds is described in K. V. Rao et al., Journal of Heterocyclic Chemistry, .1973, 10, 213 to 215.

In particular, 1,2,3,4-tetrahydroisoquinoline compounds with an amino group in position 6 are commercially available or can be prepared starting from 6-nitro-1,2,3,4-tetrahydroisoquinoline compounds. A process for the preparation of the latter compounds is described in G. J. Quallich, Journal of Organic Chemistry, 1998, 63, 4116 to 4119.

1,2,3,4-tetrahydroisoquinoline compounds with a nitro group in position 6 or 8 may be prepared by established procedures described in M. Tercel, Journal of Medicinal Chemistry, 1996, 39, 1084 to 1094.

In particular, 1,2,3,4-tetrahydroisoquinoline compounds with an amino group in position 7 are commercially available or can be prepared starting from 7-nitro-1,2,3,4-tetrahydroisoquinoline compounds. A process for the preparation of the latter compounds is described in J. F. Ajao et al., Journal of Heterocyclic Chemistry, 1985, 22, 329 to 331.

The N-methyl-8-amino-substituted 1,2,3,4-tetrahydroisoquinoline compounds were prepared by bromination and nitration of the corresponding 1,2,3,4-tetrahydroisoquinolines followed by two-step standard reduction conditions as described in M. Rey, Helvetica Chimica Acta, 1985, 66, 1828 to 1834.

If any of the substituents in any of the above defined formulae represents or comprises a (hetero)cycloaliphatic radical, preferably a C₃₋₉ cycloalkyl radical or a C₄₋₉ cycloalkenyl radical, or a heterocyclic ring, preferably a 3- to 8-membered heterocyclic ring, said (hetero)cycloaliphatic radical, heterocyclic ring, C₃₋₉ cycloalkyl radical or C₄₋₉ cycloalkenyl radical may - if not defined otherwise - be unsubstituted or substituted by one or more substituents, preferably unsubstituted or optionally substituted with 1, 2, 3, 4 or 5 substituent(s). Said substituent(s) may preferably be selected independently from the group consisting of oxo (=O), thioxo (=S), C₁₋₅-alkyl, -O-C₁₋₅-alkyl, -S-C₁₋₅-alkyl, - C(=O)-OH, -C(=O)-C₁₋₅-alkyl, -C(=O)-O-C₁₋₅-alkyl, -O-C(=O)-C₁₋₅-alkyl, F, Cl, Br, I, - CN, -CF₃, -OCF₃, -SCF₃, -OH, -SH, -NH₂, -NH(C₁₋₅-alkyl), -N(C₁₋₅-alkyl)₂, -NO₂, - CHO, -CF₂H, -CFH₂, -C(=O)-NH₂, -C(=O)-NH(C₁₋₅-alkyl), -C(=O)-N(C₁₋₅-alkyl)₂, - S(=O)₂-C₁₋₅-alkyl, -S(=O)₂-phenyl, phenyl, phenoxy and benzyl; whereby in each occurrence C₁₋₅-alkyl may be linear or branched and whereby said cyclic substituents may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃, -OCF₃, -SCF₃, -OH, -SH, -NH₂ and -NO₂.

More preferably said substituents may be selected independently from the group consisting of oxo (=O), thioxo (=S), methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₃-CH₃-CH₃, -C(=O)-O-CH(CH₃)₂, - C(=O)-O-C(CH₃)₃, -C(=O)-CH₃, -C(=O)-C₂H₅, -C(=O)-CH₃-CH₃-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, - NH-CH₃, -NH-C₂H₅, -NH-CH₂-CH₂-CH₃, -NH-CH(CH₃)₂, -NH-C(CH₃)₃, -N(CH₃)₂, - N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂, -S(=O)₂-CH₃, -S(=O)₂-phenyl, phenyl, phenoxy and benzyl; whereby in each occurrence said cyclic substituents may be unsubstituted or substituted by 1, 2 or 3 substituent(s) independently selected from the group consisting of methyl, ethyl, n-propyl, isopropyl, methoxy, ethoxy, F, Cl, Br,-CN, -CF₃, -OCF₃, -SCF₃, -OH, -SH, -NH₂ and -NO₂.

If any of the substituents in any of the above defined formulae represents or comprises a cycloaliphatic radical, C₃₋₉ cycloalkyl radical or C₄₋₉ cycloalkenyl radical which contains one or more, preferably 1, 2 or 3 heteroatom(s) as ring member(s), unless defined otherwise, each of these heteroatom(s) may preferably be selected independently from the group consisting of N, O and S.

If any of the substituents in any of the above defined formulae represents or comprises a heterocyclic ring which contains at least one further, preferably I or 2 further heteroatom(s) as ring member(s), unless defined otherwise, each of these heteroatom(s) may preferably be selected independently from the group consisting of N, O and S.

Suitable saturated or unsaturated, optionally at least one heteroatom as ring member containing cycloaliphatic radicals, C₃₋₉ cycloalkyl radicals or C₄₋₉ cycloalkenyl radicals may preferably be selected from the group consisting of cyclopropyl, cyclobutyl, cyclopentyl, cyclohexyl, cycloheptyl, cyclooctyl, cyclononyl, cyclopentenyl, cyclohexenyl, cycloheptenyl, cyclooctenyl, pyrrolidinyl, piperidinyl, piperazinyl, homopiperazinyl, morpholinyl, aziridinyl, azetidinyl, imidazolidinyl, thiomorpholinyl, pyrazolidinyl, tetrahydrofuranyl, tetrahydrothiophenyl, azepanyl, diazepanyl, azocanyl, (2,5)-dihydrofuranyl, (2,5)-dihydrothiophenyl, (2,3)-dihydrofuranyl, (2,3)-dihydrofuranyl, (2,5)-dihydro-1H-pyrrolyl, (2,3)-dihydro-1H-pyrrolyl, tetrahydrothiopyranyl, tetrahydropyranyl, (3,4)-dihydro-2H-pyranyl, (3,4)-dihydro-2H-thiopyranyl, (1,2,3,6)-tetrahydropyridinyl, (1,2,3,4)-tetrahydropyridinyl, (1,2,5,6)-tetrahydropyridinyl, [1,3]-oxazinanyl, hexahydropyrimidinyl, (5,6)-dihydro-4H-pyrimidinyl, oxazolidinyl, (1,3)-dioxanyl, (1,4)-dioxanyl and (1,3)-dioxolanyl.

Suitable saturated or unsaturated heterocyclic rings which may be condensed with an unsubstituted or at least mono-substituted mono- or bicyclic ring system may preferably be selected from the group consisting of pyrrolidinyl, piperidinyl, piperazinyl, homopiperazinyl, morpholinyl, aziridinyl, azetidinyl, imidazolidinyl, thiomorpholinyl, pyrazolidinyl, azepanyl, diazepanyl, azocanyl, (1,2,3,6)-tetrahydropyridinyl, (1,2,3,4)-tetrahydropyridinyl, (1,2,5,6)-tetrahydropyridinyl, hexahydropyrimidinyl, (5,6)-dihydro-4H-pyrimidinyl, pyridazin-3(2H)-on-yl, phthalazin-1(2H)-on-yl, indolinyl, isoindolinyl, decahydronaphthyl, (1,2,3,4)-tetrahydroquinolinyl, (1,2,3,4)-tetrahydroisoquinolinyl, (1,2,3,4)-tetrahydronaphthyl, octahydro-cyclopenta[c]pyrrolyl, (1,3,4,7,9a)-hexahydro-2H-quinolizinyl, (1,2,3,5,6,8a)-hexahydro-indolizinyl, decahydroquinolinyl, octahydropyrrolo[1,2-a]pyrazinyl, octahydro-1H-pyrido[1,2-a]pyrazinyl, dodecahydrocarbazolyl, 9H-carbazolyl, decahydroisoquinolinyl and (2,3)-dihydro-1H-benzo[de]isoquinolinyl.

Suitable aromatic heterocyclic rings which may be condensed with an unsubstituted or at least mono-substituted mono- or bicyclic ring system may preferably be selected from the group consisting of imidazolyl, pyrazolyl, triazolyl, (4,5,6,7)-tetrahydro-2H-indazolyl, indazolyl and benzimidazolyl.

Suitable saturated or unsaturated, optionally at least one heteroatom as ring member containing cycloaliphatic radicals, C₃₋₉ cycloalkyl radicals or C₄₋₉ cycloalkenyl radicals which are condensed with an unsubstituted or at least mono-substituted mono- or bicyclic ring system may preferably be selected from the group consisting of indolinyl, isoindolinyl, decahydronaphthyl, (1,2,3,4)-tetrahydroquinolinyl, (1,2,3,4)-tetrahydroisoquinolinyl, (1,2,3,4)-tetrahydronaphthyl, octahydro-cyclopenta[c]pyrrolyl, (1,3,4,7,9a)-hexahydro-2H-quinolizinyl, (1,2,3,5,6,8a)-hexahydro-indolizinyl, decahydroquinolinyl, dodecahydrocarbazolyl, 9H-carbazolyl, decahydroisoquinolinyl, (6,7)-dihydro-4H-thieno[3,2-c]pyridinyl, (2,3)-dihydro-1H-benzo[de]isoquinolinyl, octahydropyrrolo[1,2-a]pyrazinyl, octahydro-1H-pyrido[1,2-a]pyrazinyl, (1,2,3,7,8,8a)-hexahydroindolizinyl, (2,6,7,8,9,9a)-hexahydro-1H-quinolizinyl, octahydroindolizinyl, octahydro-1H-quinolizinyl, (1,2,3,5,8,8a)-hexahydroindolizinyl, (4,6,7,8,9,9a)-hexahydro-1H-quinolizinyl, fluorenyl and (1,2,3,4)-tetrahydroquinoxalinyl.

If any of the substituents in any of the above defined formulae represents an alkylene group, preferably an C₁₋₆ alkylene group, an alkenylene group, preferably an C₂₋₆ alkenylene group or an alkinylene group, preferably an C₂₋₆, alkinylene group, which may be substituted, said alkylene group, C₂₋₆ alkylene group, alkenylene group, C₂₋₆ alkenylene group, alkinylene group or C₂₋₆ alkinylene group may be unsubstituted or substituted by one or more substituents, preferably unsubstituted or optionally substituted with 1, 2 or 3 substituent(s). Said substituent(s) may preferably be selected independently from the group consisting of -O-C₁₋₅-alkyl, -S-C₁₋₅-alkyl, -F, Cl, Br, I, - CN, -CF₃, -OCF₃, -SCF₃, -OH, -SH, -NH₂, -NH(C₁₋₅-alkyl) and -N(C₁₋₅-alkyl)₂, whereby in each occurrence C₁₋₅-alkyl may be linear or branched. An alkenylene group comprises at least one carbon-carbon double bond, an alkinylene group comprises at least one carbon-carbon triple bond.

Suitable alkylene groups include -(CH₂)-, -CH(CH₃)-, -CH(phenyl), -(CH₂)₂-, -(CH₂)₃-,-(CH₂)₄-,-(CH₂)₅ and -(CH₂)₆-, suitable alkenylene groups include -CH=CH-, -CH₂-CH=CH- and -CH=CH-CH₂- and suitable alkinylene groups include -C≡C- , -CH₂-C≡C- and -C≡C-CH₂-.

If any of the substituents in any of the above defined formulae represents or comprises an aryl radical, including a 6-membered aryl radical such as phenyl or a 10-membered aryl radical such as naphthyl or a 14-membered aryl radical such as anthracenyl, said aryl radical may - if not defined otherwise - be unsubstituted or substituted by one or more substituents, preferably unsubstituted or substituted with 1, 2, 3, 4 or 5 substituent(s). Said substituent(s) may preferably be selected independently from the group consisting of C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, -C₁-₅-alkylene-C(=O)-OH, -C₁₋₅-alkylene-C(=O)-O-C₁₋₅-alkyl, -NH-C(=O)-C₁₋₅-alkyl, -NH-S(=O)₂-C₁₋₅-alkyl, pyrrolidinyl, piperdinyl, morpholinyl, oxazolyl, isoxazolyl, pyridinyl, cyclopropyl, cyclobutyl, cyclopentyl, cyclohexyl, thiophenyl, furanyl, pyrolidin-2,5-dionyl, phenyl, phenoxy and benzyl; whereby in each occurrence C₁₋₅-alkyl may be linear or branched and whereby said cyclic substituent(s) may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃, -OCF₃, - SCF₃, -OH, -SH, -NH₂ and -NO₂.

More preferably said substituents may be selected independently from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, - C(=O)-O-C₂H₅, -C(=O)-O-CH₃-CH₃-CH₃, -C(=O)-O-CH(CH₃)₂, -C(=O)-O-C(CH₃)₃, - C(=O)-CH₃, -C(=O)-C₂H₅, -C(=O)-CH₃-CH₃-CH₃; C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, - C(=O)-N(C₂H₅)₂ and -S(=O)₂-CH₃.

Preferred aryl radicals, which may optionally be at least mono-substituted, are phenyl and naphthyl.

Suitable aryl radicals, which are condensed with an unsubstituted or at least mono-substituted saturated or unsaturated mono- or bicyclic ring system, may preferably be selected from the group consisting of indolinyl, isoindolinyl, (1,2,3,4)-tetrahydroquinolinyl, (1,2,3,4)-tetrahydroisoquinolinyl, (1,2,3,4)-tetrahydronaphthyl, (1,2,3,4)-tetrahydroquinoxalinyl, benzo[d]oxazol-2(3H)-onyl, benzo[d]thiazol-2(3H)-onyl, 2,3-dihydrobenzo[b][1,4]dioxinyl, benzo[d][1,3]dioxolyl, 3,4-dihydro-2H-benzo[b][1,4]oxazinyl, isochromanyl, chromanyl, 2,3-dihydrobenzofuranyl and 1H-benzo[b][1,4]diazepine-2,4(3H,5H)-dionyl.

If any of the substituents in any of the above defined formulae represents or comprises a heteroaryl radical, including a monocyclic 5- or 6-membered heteroaryl radical or a bi-or tricyclic 8-, 9-, 10-, 11-, 12-, 13- or 14 membered heteroaryl radical, said heteroaryl radical may - if not defined otherwise - be unsubstituted or substituted by one or more substituents, preferably unsubstituted or substituted with 1, 2, 3, 4 or 5 substituent(s). Said substituent(s) may preferably be selected independently from the group consisting of C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -SH, -NH₂, - NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, -C₁₋₅-alkylene-C(=O)-OH, -C₁₋₅-alkylene-C(=O)-O-C₁₋₅-alkyl, -NH-C(=O)-C₁₋₅-alkyl, -NH-S(=O)₂-C₁₋₅-alkyl, pyrrolidinyl, piperdinyl, morpholinyl, oxazolyl, isoxazolyl, pyridinyl, cyclopropyl, cyclobutyl, cyclopentyl, cyclohexyl, thiophenyl, furanyl, pyrrolidin-2,5-dionyl, phenyl, phenoxy and benzyl; whereby in each occurrence C₁₋₅-alkyl may be linear or branched and whereby said cyclic substituent(s) may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃, -OCF₃, -SCF₃, -OH, -SH, -NH₂, and -NO₂.

More preferably said substituents may be selected independently from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, - C(=O)-O-C₂H₅, -C(=O)-O-CH₃-CH₃-CH₃, -C(=O)-O-CH(CH₃)₂, -C(=O)-O-C(CH₃)₃, - C(=O)-CH₃, -C(=O)-C₂H₅, -C(=O)-CH₃-CH₃-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, - C(=O)-N(C₂H₅)₂ and -S(=O)₂-CH₃.

The heteroatom(s), which are present as ring member(s) in the heteroaryl radical, may, unless defined otherwise, independently be selected from the group consisting of nitrogen, oxygen and sulphur. Preferably the heteroaryl radical comprises 1, 2, 3 or 4 heteroatom(s).

Suitable bi- or tricyclic heteroaryl radicals, which may optionally be at least mono-substituted, may preferably be selected from the group consisting of indolyl, isoindolyl, quinolinyl, isoquinolinyl, benzo[b]furanyl, benzo[b]thiophenyl, benzimidazolyl, benzo[2,1,3]thiadiazolyl, [1,2,3]-benzothiadiazolyl, [2,1,3]-benzoxadiazolyl, [1,2,3]-benzoxadiazolyl, benzoxazolyl, benzothiazolyl, benzisoxazolyl, benzisothiazolyl, imidazo[2,1-b]thiazolyl, 2H-chromenyl, indazolyl and quinazolinyl.

Suitable mono-, bi- or tricyclic heteroaryl radicals, which are condensed with an unsubstituted or at least mono-substituted saturated or unsaturated mono- or bicyclic ring system, may preferably be selected from the group consisting of [1,3]-benzodioxolyl, [1,4]-benzodioxanyl, [1,2,3,4]-tetrahydronaphthyl, (2,3)-dihydro-1H-cyclopenta[b]indolyl,[1,2,3,4]-tetrahydroquinolinyl, [1,2,3,4]-tetrahydroisoquinolinyl, [1,2,3,4]-tetrahydroquinazolinyl and [3,4]-dihydro-2H-benzo[1,4]oxazinyl.

Suitable monocyclic heteroaryl radicals, which may optionally be at least mono-substituted, may preferably be selected from the group consisting of pyridinyl, furyl (furanyl), thiophenyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, pyrimidinyl, pyrazinyl and pyranyl.

A mono- or bicyclic ring system according to the present invention - if not defined otherwise - means a mono- or bicyclic hydrocarbon ring system that may be saturated, unsaturated or aromatic. Each of its different rings may show a different degree of saturation, i.e. it may be saturated, unsaturated or aromatic. Optionally each of the rings of the mono- or bicyclic ring system may contain one or more, preferably 1, 2 or 3, heteroatom(s) as ring member(s), which may be identical or different and which can preferably be selected from the group consisting of N, O and S. The rings of the mono-or bicyclic ring system are preferably 5-, 6- or 7-membered.

Preferably a mono-or bicyclic ring system according to the present invention is a phenyl or naphthyl ring system.

The term "condensed" according to the present invention means that a ring or ring system is attached to another ring or ring system, whereby the term's "annulated" or "annelated" are also used by those skilled in the art to designate this kind of attachment.

Such a mono- or bicyclic ring system may - if not defined otherwise - be unsubstituted or substituted by one or more substituents, preferably unsubstituted or substituted with 1, 2, 3, 4 or 5 substituent(s). Said substituents may preferably be selected independently from the group consisting of C₁₋₅-alkyl, -O-C₁₋₅-alkyl, -S-C₁₋₅-alkyl, -C(=O)-OH, oxo (=O), thioxo (=S), -C(=O)-O-C₁₋₅-alkyl, -O-C(=O)-C₁₋₅-alkyl, F, Cl, Br, I, -CN, -CF₃, - OCF₃, -SCF₃, -OH, -SH, -NH₂, -NH(C₁₋₅-alkyl), -N(C₁₋₅-alkyl)₂, -NO₂, -CHO, -CF₂H, - CFH₂, -C(=O)-NH)₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl; whereby in each occurrence C₁₋₅-alkyl may be linear or branched and whereby said cyclic substituents may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃, -OCF₃, - SCF, -OH, -SH, -NH₂ and -NO₂.

More preferably said substituents may be selected from the group consisting of oxo (=O), thioxo (=S), methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₁-CH₁-CH₃, -S-CH(CH₃)₂, -S-C(CH₃)₃, -C(=O)-OH, -C(=O)-O-CH₃, -C(=O)-O-C₂H₅, -C(=O)-O-CH₃-CH₃-CH₃, -C(=O)-O-CH(CH₃)₂, -C(=O)-O-C(CH₃)₃, -C(=O)-CH₃, -C(=O)-C₂H₅, -C(=O)-CH₃-CH₃-CH₃, -C(=O)-CH(CH₃)₂, - C(=O)-C(CH₃)₃, F, Cl, Br, I, -CN, -CF₃, -OCF₃, -SCF₃, -OH, -SH, -NH₂, -NH-CH₃, - NH-C₂H₅, -NH-CH₂-CH₂-CH₃, -NH-CH(CH₃)₂, -NH-C(CH₃)₃, -N(CH₃)₂, -N(C₂H₅)₂, - NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂, -S(=O)₂-CH₃, -S(=O)₂-phenyl, phenyl, phenoxy and benzyl; whereby in each occurrence said cyclic substituents may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃, -OCF₃, - SCF₃, -OH, -SH, -NH₂ and -NO₂.

If any of the substituents in any of the above defined formulae represents a saturated or unsaturated aliphatic radical, i.e. an alkyl radical, preferably an C₁₋₁₀ alkyl radical; an alkenyl radical, preferably an C₂₋₁₀ alkenyl radical or an alkinyl radical, preferably an C₂₋₁₀ alkinyl radical; said aliphatic radical may - if not defined otherwise - be unsubstituted or substituted by one or more substituents, preferably unsubstituted or substituted with 1, 2, 3, 4 or 5 substituent(s). Said substituent(s) may preferably be selected independently from the group consisting of -O-C₁₋₅-alkyl, -S-C₁₋₅-alkyl, F, Cl, Br, I, -CN, -CF₃, -OCF₃, -SCF₃, -OH, -SH, -NH₂, -NH(C₁₋₅-alkyl) and -N(C₁₋₅-alkyl)₂, whereby in each occurrence C₁₋₅-alkyl may be linear or branched. More preferably said substituent(s) may preferably be selected independently from the group consisting of - O-CH₃, -O-C₂H₅, -O-CH₂-CH₂-CH), -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, F, Cl, Br, I, -CN, -CF₃, -OCF₃, -SCF₃, -OH, - SH, -NH₂, NH-CH₃, -NH-C₂H₅, -NH-CH₂-CH₂-CH₃, -NH-CH(CH₃)₂, -NH-C(CH₃)₃, - N(CH₃)₂, -N(C₂H₅)₂.

An alkenyl radical comprises at least one carbon-carbon double bond, an alkinyl radical comprises at least one carbon-carbon triple bond.

Suitable alkyl radicals, which may be substituted by one or more substituents, may preferably be selected from the group consisting of methyl, ethyl, n-propyl, isopropyl, n-butyl, isobutyl, sec-butyl, tert-butyl, n-pentyl, n-hexyl, n-heptyl, n-octyl, n-nonyl and n-decyl.

Suitable alkenyl radicals, which may be substituted by one or more substituents, may preferably be selected from the group consisting of vinyl, 1-propenyl, 2-propenyl, 1-butenyl, 2-butenyl and 3-butenyl.

Suitable alkinyl radicals, which may be substituted by one or more substituents, may preferably be selected from the group consisting of ethinyl, 1-propinyl, 2-propinyl, 1-butinyl, 2-butinyl and 3-butinyl.

The NMDA receptor is a cell-surface protein complex, widely distributed in the mammalian central nervous system that belongs to the class of ionotropic-glutamate receptors. It is involved in excitatory-synaptic transmission and the regulation of neuronal growth. The structure comprises a ligand-gated/voltage-sensitive ion channel. The NMDA receptor is highly complex and is believed to contain at least five distinct binding (activation) sites: a glycine-binding site, a glutamate-binding site (NMDA-binding site); a PCP-binding site, a polyamine-binding site, and a zinc-binding site. In general, a receptor antagonist is a molecule that blocks or reduces the ability of an agonist to activate the receptor.

Memantine is present as component (B). Memantine (CAS Registry No. 41100-52-1), is an uncompetitive N-methyl-D-aspartate antagonist currently used for the treatment of dementia syndrome, spinal spasticity and Parkinson's disease. Chemically, memantine is 1-amino-3,5-dimethyladamantane of the adamantine class.

Further derivatives of memantine and nitromemantine can be prepared as outlined in U.S. patent application 2004/0122090.

The term "cognitive disorder" indicates disruptions in performance including one or more of the following signs:
1) memory deficits (impaired ability to learn new information or recall previously learned information;
2) one (or more) of the following disturbances:
   a) aphasia (language disturbance)
   b) apraxia (impaired ability to carry out motor activities despite intact motor function)
   c) agnosia (failure to recognize or identify objects despite in tact sensory function)
   d) disturbance in executive functioning (i.e. planning, organizing, sequencing, abstracting);
3) memory disturbances causing significant impairment in social or occupational functioning, and representing a significant decline from a previous level of functioning; and
4) impairment in cognitive functioning as evidenced by neuropsychological testing or quantified clinical assessment, accompanied by objective evidence of a systemic general medical condition or central nervous system dysfunction.

Cognitive disorders (or memory disorders) may include Alzheimer's disease, senile dementia process, learning disabilities caused by degenerative disorders, learning disabilities caused by non-degenerative disorders, memory or cognitive dysfunction such as mild cognitive impairment, age-related cognitive decline, cerebral senility, vascular dementia, AIDS-associated dementia, electric shock induced amnesia, memory impairment associated with depression or anxiety, cognitive defects in Parkinson's disease, Down's syndrome, stroke, traumatic brain injury, Huntington's disease, and attention deficit disorder; especially ADHD (attention deficit / hyperactivity disorder).

"Treatment" (e.g. of cognitive disorders, e.g. depression or e.g. of obesity and obesity-related disorders) refers to the administration of the compounds or combinations of the present invention to treat (in the case of cognitive disorders or dpression) the disorder or more often - the symptoms of these disorders; or (for obesity) reduce or maintain the body weight of an obese subject. One outcome of treatment may be ameliorating the symptoms of e.g. Alzheimer's disease or the clinical depression or may be reducing the body weight of an obese subject relative to that subject's body weight immediately before the administration of the compounds or combinations of the present invention. A preferred aspect of the treatment, especially for cognitive disorders or depression, also involves the prophylaxis against the disorder, thus preventing the occurrence of its symptoms. Another outcome of treatment may be preventing body weight regain of body weight previously lost as a result of diet, exercise, or pharmacotherapy.

Another outcome of treatment may be decreasing the occurrence of and/or the severity of obesity-related diseases. Another outcome of treatment may be to maintain weight loss. The treatment may suitably result in a reduction in food or calorie intake by the subject, including a reduction in total food intake, or a reduction of intake of specific components of the diet such as carbohydrates or fats; and/or the inhibition of nutrient absorption; and/or the inhibition of the reduction of metabolic rate; and in weight reduction in patients in need thereof. The treatment may also result in an alteration of metabolic rate, such as an increase in metabolic rate, rather than or in addition to an inhibition of the reduction of metabolic rate; and/or in minimization of the metabolic resistance that normally results from weight loss.

The term "depression" or especially "clinical depression" is referring to a state of sadness, melancholia or despair that has advanced to the point of being disruptive to an individual's social functioning and/or activities of daily living.

"Obesity" is a condition in which there is an excess of body fat. The operational definition of obesity is based on the Body Mass Index(BMI), which is calculated as body weight per height in meters squared (kg/m²). "Obesity" refers to a condition whereby an otherwise healthy subject has a Body Mass Index (BMI) greater than or equal to 30kg/m², or a condition whereby a subject with at least one co-morbidity has a BMI greater than or equal to 27kg/m². An "obese subject" is an otherwise healthy subject with a Body Mass Index (BMI) greater than or equal to 30 kg/m² or a subject with at least one co-morbidity with a BMI greater than or equal to 27 kg/m². A "subject at risk of obesity" is an otherwise healthy subject with a BMI of 25 kg/m² to less than 30 kg/m² or a subject with at least one co-morbidity with a BMI of 25 kg/m² to less than 27 kg/m².

The increased risks associated with obesity occur at a lower Body Mass Index(BMI) in Asians. In Asian countries, including Japan, "obesity" refers to a condition whereby a subject with at least one obesity-induced or obesity-related co-morbidity, that requires weight reduction or that would be improved by weight reduction, has a BMI greater than or equal to 25 kg/m². In Asian countries, including Japan, an "obese subject" refers to a subject with at least one obesity-induced or obesity-related co-morbidity that requires weight reduction or that would be improved by weight reduction, with a BMI greater than or equal to 25kg/m². In Asia-Pacific, a "subject at risk of obesity" is a subject with a BMI of greater than 23 kg/m² to less than 25 kg/m².

As used herein, the term "obesity" is meant to encompass all of the above definitions of obesity.

Obesity-induced or obesity-related co-morbidities include, but are not limited to, diabetes, non-insulin dependent diabetes mellitus-type II (2), impaired glucose tolerance, impaired fasting glucose, insulin resistance syndrome, dyslipidemia, hypertension, hyperuricacidemia, gout, coronary artery disease, myocardial infarction, angina pectoris, sleep apnea syndrome, Pickwickian syndrome, fatty liver; cerebral infarction, cerebral thrombosis, transient ischemic attack, orthopedic disorders, arthritis deformans, lumbodynia, emmeniopathy, and infertility.

In particular, co-morbidities include: hypertension, hyperlipidemia, dyslipidemia, glucose intolerance, cardiovascular disease, sleep apnea, diabetes mellitus, and other obesity-related conditions.

The term "Metabolic syndrome", also known as syndrome X, is defined in the Third Report of the National Cholesterol Education Program Expert Panel on Detection, Evaluation and Treatment of High Blood Cholesterol in Adults (ATP-E). E. S. Ford et al. , JAMA, Vol. 287 (3), Jan. 16, 2002, pp 356-359. Briefly, a person is defined as having Metabolic syndrome if the person has three or more of the following symptoms: abdominal obesity, hypertriglyceridemia, low HDL cholesterol, high blood pressure, and high fasting plasma glucose.

The terms "administration of" and or "administering a" compound should be understood to mean providing a compound of the invention or a prodrug of a compound of the invention to a subject in need of treatment.

The instant pharmaceutical composition includes administration of a single pharmaceutical dosage formulation which contains both the compound with 5-HT₆ receptor affinity, and at least one NMDA-receptor ligand, as well as administration of each active agent in its own separate pharmaceutical dosage formulation. Where separate dosage formulations are used, the individual components of the composition can be administered at essentially the same time, i. e., concurrently, or at separately staggered times, i. e. sequentially prior to or subsequent to the administration of the other component of the composition. The instant pharmaceutical composition is therefore to be understood to include all such regimes of simultaneous or alternating treatment, and the terms "administration" and "administering" are to be interpreted accordingly.

Administration in these various ways are suitable for the present compositions as long as the beneficial pharmaceutical effect of the combination of the compound with 5-HT₆ receptor affinity, and at least one NMDA-receptor ligand, is realised by the patient at substantially the same time.

Such beneficial effect is preferably achieved when the target blood level concentrations of each active drug are maintained at substantially the same time. It is preferred that the combination of the compound with 5-HT₆ receptor affinity, and at least one NMDA-receptor ligand, be co-administered concurrently on a once-a-day dosing schedule; however, varying dosing schedules, such as the compound with 5-HT₆ receptor affinity once a day and the NMDA-receptor ligand once, twice or more times per day, is also encompassed herein. A single oral dosage formulation comprised of both a compound with 5-HT₆ receptor affinity and a NMDA-receptor ligand is preferred. A single dosage formulation will provide convenience for the patient, which is an important consideration especially for patients with diabetes or obese patients who may be in need of multiple medications.

The term "subject" as used herein refers to an animal, preferably a mammal, most preferably a human, who has been the object of treatment, observation or experiment.

The administration of the composition of the present invention in order to practice the present methods of therapy is carried out by administering a therapeutically effective amount of the compounds in the composition to a subject in need of such treatment or prophylaxis. The need for a prophylactic administration according to the methods of the present invention is determined via the use of well known risk factors. The effective amount of an individual compound is determined, in the final analysis, by the physician in charge of the case, but depends on factors such as the exact disease to be treated, the severity of the disease and other diseases or conditions from which the patient suffers, the chosen route of administration, other drugs and treatments which the patient may concomitantly require, and other factors in the physician's judgement.

The term "therapeutically effective amount" as used herein means the amount of the active compounds in the composition that will elicit the biological or medical response in a tissue, system, subject, or human that is being sought by the researcher, veterinarian, medical doctor or other clinician, which includes alleviation of the symptoms of the disorder being treated. The novel methods of treatment of this invention are for disorders known to those skilled in the art.

The term "salt" as used herein is to be understood as meaning any form of the compounds in which they assume an ionic form or are charged and are coupled with a counter-ion (a cation or anion) or are in solution. By this are also to be understood complexes of the active compound with other molecules and ions, in particular complexes which are complexed via ionic interactions.

The term "physiologically acceptable salt" is understood in particular, in the context of this invention, as salt (as defined above) formed either with a physiologically tolerated acid, that is to say salts of the particular active compound with inorganic or organic acids which are physiologically tolerated - especially if used on humans and/or mammals - or with at least one, preferably inorganic, cation which are physiologically tolerated - especially if used on humans and/or mammals. Examples of physiologically tolerated salts of particular acids are salts of: hydrochloric acid, hydrobromic acid, sulfuric acid, hydrobromide, monohydrobromide, monohydrochloride or hydrochloride, methiodide, methanesulfonic acid, formic acid, acetic acid, oxalic acid, succinic acid, malic acid, tartaric acid, mandelic acid, fumaric acid, lactic acid, citric acid, glutamic acid, hippuric acid, picric acid and/or aspartic acid. Examples of physiologically tolerated salts of particular bases are salts of alkali metals and alkaline earth metals and with NH₄.

Solvates, preferably hydrates, of the compounds of the present invention and in each case of corresponding stereoisomers may also be obtained by standard procedures known to those skilled in the art.

The term "solvate" according to this invention is to be understood as meaning any form of the compounds in which they have attached to it via non-covalent binding another molecule (most likely a polar solvent) especially including hydrates and alcoholates, e.g. methanolate.

The active substance combination according to this invention comprises preferably 1-99% by weight of the component (A) and 99-1 % by weight of the component (B), more preferably 10-80% by weight of the component (A) and 80-20% by weight of the component (B), these percentages referring to the total weight of both components (A) and (B).

Another aspect of the present invention is a medicament, which comprises an inventive active substance combination and optionally one or more pharmacologically acceptable adjuvants.

Said medicament is suitable for simultaneous NMDA-receptor inhibition and 5-HT₆-receptor regulation.

Said medicament is particularly preferably suitable for the prophylaxis and/or treatment of disorders related to food ingestion, preferably for prophylaxis and/or treatment of obesity, anorexia, cachexia, bulimia, diabetes, preferably type II diabetes (non-insulin-dependent diabetes mellitus), or for prophylaxis and/or treatment of gastrointestinal tract disorders, preferably of the irritable bowel syndrome, for prophylaxis and/or treatment of Metabolic Syndrome, Peripheral Nervous System Disorders, Central Nervous System Disorders, arthritis, epilepsy, anxiety, panic, depression, cognitive disorders, memory disorders, cardiovascular diseases, senile dementia processes, such as Alzheimer's, Parkinson's and/or Huntington's Disease, schizophrenia, psychosis, infantile hyperkinesia (ADHD, attention deficit / hyperactivity disorder), pain, hypertensive syndrome, inflammatory diseases, immunologic diseases or for improvement of cognition.

Said medicament is more particularly preferably suitable for the prophylaxis and/or treatment of cognitive disorders or memory disorders like Alzheimer's disease, senile dementia process, learning disabilities caused by degenerative disorders, learning disabilities caused by non-degenerative disorders, memory or cognitive dysfunction such as mild cognitive impairment, age-related cognitive decline, cerebral senility, vascular dementia, AIDS-associated dementia, electric shock induced amnesia, memory impairment associated with depression or anxiety, cognitive defects in Parkinson's disease, Down's syndrome, stroke, traumatic brain injury, Huntington's disease, and attention deficit disorder; especially ADHD (attention deficit / hyperactivity disorder). Said medicament is more particularly preferably also suitable for the prophylaxis and/or treatment of depression as well as anxiety and panic.

Said medicament is more particularly preferably also suitable for the regulation of appetite, for maintenance, increase or reduction of body weight, for prophylaxis and/or treatment of disorders related to food ingestion, preferably for prophylaxis and/or treatment of obesity, anorexia, cachexia, bulimia, diabetes, preferably type II diabetes (non-insulin-dependent diabetes mellitus), or for prophylaxis and/or treatment of gastrointestinal tract disorders, preferably of the irritable bowel syndrome.

Said medicament is even more particularly preferably suitable for the the prophylaxis and/or treatment of obesity.

Said medicament is also particularly preferably suitable for the prophylaxis and/or treatment of obesity-related disorders such as elevated plasma insulin concentrations and insulin resistance, dyslipidemias, hyperlipidemia, endometrial, breast, prostate and colon cancer, osteoarthritis, obstructive sleep apnea, cholelithiasis, gallstones, heart disease, abnormal heart rhythms and arrythmias, myocardial infarction, congestive heart failure, coronary heart disease, sudden death, stroke, polycystic ovary disease, craniopharyngioma, the Prader-Willi Syndrome and Frohlich's syndrome. Further examples of obesity-related disorders are reproductive hormone abnormalities, sexual and reproductive dysfunction, such as impaired fertility, infertility, hypogonadism in males and hirsutism in females, fetal defects associated with maternal obesity, gastrointestinal motility disorders, such as obesity-related gastro-esophageal reflux, respiratory disorders, such as obesity-related hypoventilation syndrome (Pickwickian syndrome), breathlessness, cardiovascular disorders, inflammation, such as systemic inflammation of the vasculature, arteriosclerosis, hypercholesterolemia, hyperuricaemia, lower back pain, gallbladder disease, gout, kidney cancer, and increased anesthetic risk.

Another aspect of the present invention is the use of an inventive active substance combination for the manufacture of a medicament for simultaneous NMDA-receptor inhibition and 5-HT₆-receptor regulation.

Another aspect of the present invention is the use of an inventive active substance combination for the manufacture of a medicament for the regulation of appetite, for maintenance, increase or reduction of body weight, for prophylaxis and/or treatment of disorders related to food ingestion, preferably for prophylaxis and/or treatment of obesity, anorexia, cachexia, bulimia, diabetes, preferably type II diabetes (non-insulin-dependent diabetes mellitus), or for prophylaxis and/or treatment of gastrointestinal tract disorders, preferably of the irritable bowel syndrome, for prophylaxis and/or treatment of Peripheral Nervous System Disorders, Central Nervous System Disorders, arthritis, epilepsy, anxiety, panic, depression, preferably bipolar disorders, cognitive disorders, memory disorders, cardiovascular diseases, senile dementia processes, neurodegenerative disorders, preferably Alzheimer's disease, Parkinson's diesease, Huntington's Disease and/or multiple sclerosis, schizophrenia, psychosis, infantile hyperkinesia (ADHD, attention deficit / hyperactivity disorder), pain, hypertensive syndrome, inflammatoric diseases, immunologic diseases or for improvement of cognition.

Particularly preferred is the use of an inventive active substance combination for the manufacture of a medicament for the prophylaxis and/or treatment of cognitive disorders or memory disorders like Alzheimer's disease, senile dementia process, learning disabilities caused by degenerative disorders, learning disabilities caused by non-degenerative disorders, memory or cognitive dysfunction such as mild cognitive impairment, age-related cognitive decline, cerebral senility, vascular dementia, AIDS-associated dementia, electric shock induced amnesia, memory impairment associated with depression or anxiety, cognitive defects in Parkinson's disease, Down's syndrome, stroke, traumatic brain injury, Huntington's disease, and attention deficit disorder; especially ADHD (attention deficit / hyperactivity disorder).

Also particularly preferred is the use of an inventive active substance combination for the manufacture of a medicament for the prophylaxis and/or treatment of depression as well as anxiety and panic.

Also particularly preferred is the use of an inventive active substance combination for the manufacture of a medicament for the regulation of appetite, for maintenance, increase or reduction of body weight, for prophylaxis and/or treatment of disorders related to food ingestion, preferably for prophylaxis and/or treatment of obesity, anorexia, cachexia, bulimia, diabetes, preferably type II diabetes (non-insulin-dependent diabetes mellitus), or for prophylaxis and/or treatment of gastrointestinal tract disorders, preferably of the irritable bowel syndrome.

Those skilled in the art understand that the components. (A) and (B) of the active substance combination according to the present invention may be administered simultaneously or sequentially to one another, whereby in each case components (A) and (B) may be administered via the same or different administration pathways, e.g. orally or parentally preferably both components (A) and (B) are administered simultaneously in one and the same administration form.

Yet another aspect of the present invention are pharmaceutical formulations in different pharmaceutical forms comprising an inventive active substance combination and optionally one or more pharmacologically acceptable adjuvants.

As well known to somebody skilled in the art the pharmaceutical formulations may - depending on their route of administration, also contain one or more auxiliary substances known to those skilled in the art.

The pharmaceutical formulations according to the present invention may be produced according to standard procedures known to those skilled in the art, e.g. from the tables of contents from "Pharmaceutics: the Science of Dosage Forms", Second Edition, Aulton, M.E. (Ed.) Churchill Livingstone, Edinburgh (2002); "Encyclopedia of Pharmaceutical Technology", Second Edition, Swarbrick, J. and Boylan J.C. (Eds.), Marcel Dekker, Inc. New York (2002); "Modem Pharmaceutics", Fourth Edition, Banker G.S. and Rhodes C.T. (Eds.) Marcel Dekker, Inc. New York 2002 and "The Theory and Practice of Industrial Pharmacy", Lachman L., Lieberman H. and Kanig J. (Eds.), Lea & Febiger, Philadelphia (1986). The respective descriptions are incorporated by reference and are part of the disclosure.

Preferred pharmaceutical formulation are solid pharmaceutical forms, preferably tablets, chewing tablets, chewing gums, dragées, capsules, suppositories, powder preparations, transdermal therapeutic systems, transmucosal therapeutic systems, preferably tablets or capsules.

Preferred pharmaceutical formulations are also liquid and semi-liquid pharmaceutical forms such as drops or such as juice, sirup, solution, emulsion, suspension, preferably drops or solutions.

In an additional preferred embodiment, the pharmaceutical formulations are in the form of multiparticulates, preferably microtablets, microcapsules, microspheroids, granules, crystals or pellets, optionally compacted in a tablet, filled in a capsule or suspended in a suitable liquid.

The pharmaceutical formulations according to the present invention are particularly preferably suitable for oral, intravenous, intramuscular, subcutaneous, intrathecal, epidural, buccal, sublingual, pulmonal, rectal, transdermal, nasal or intracerebroventricular application, more particularly for oral, intravenous or intraperitoneal application.

In one embodiment of the present invention the pharmaceutical formulation comprises at least one of the components (A) and (B) of the active substance combination at least partially in a sustained-release form.

By incorporating one or both of these components (A) and (B) at least partially or completely in a sustained-release form it is possible to extend the duration of their effect, allowing for the beneficial effects of such a sustained-release form, e.g. the maintenance of even concentrations in the blood.

Suitable sustained-release forms as well as materials and methods for their preparation are known to those skilled in the art, e.g. from the tables of contents from "Modified-Release Drug Delivery Technology", Rathbone, M.J. Hadgraft, J. and Roberts, M.S. (Eds.), Marcel Dekker, Inc., New York (2002); "Handbook of Pharmaceutical Controlled Release Technology", Wise, D.L. (Ed.), Marcel Dekker, Inc. New York, (2000);"Controlled Drug Delivery", Vol. I, Basic Concepts, Bruck, S.D. (Ed.), CRC Press Inc., Boca Raton (1983) and from Takada, K. and Yoshikawa, H., "Oral Drug delivery", Encyclopedia of Controlled Drug Delivery, Mathiowitz, E. (Ed.), John Wiley & Sons, Inc., New York (1999), Vol. 2, 728-742; Fix, J., "Oral drug delivery, small intestine and colon", Encylopedia of Controlled Drug Delivery, Mathiowitz, E. (Ed.), John Wiley & Sons, Inc., New York (1999), Vol. 2, 698-728. The respective descriptions are incorporated by reference and are part of the disclosure.

If the pharmaceutical formulation according to the present invention comprises at least one of the components (A) and (B) at least partially in a sustained-release form, said sustained release may preferably be achieved by the application of at least one coating or provision of a matrix comprising at least one sustained-release material.

The sustained-release material is preferably based on an optionally modified, water-insoluble, natural, semisynthetic or synthetic polymer, or a natural, semisynthetic or synthetic wax or fat or fatty alcohol or fatty acid, or on a mixture of at least two of these afore mentioned components.

The water-insoluble polymers used to produce a sustained-release material are preferably based on an acrylic resin, which is preferably selected from the group of poly(meth)acrylates, particularly preferably poly(C₁₋₄)alkyl (meth)acrylates, poly(C₁₋₄)dialkylamino(C₁₋₄)alkyl (meth)acrylates and/or copolymers or mixtures thereof, and very particularly preferably copolymers of ethyl acrylate and methyl methacrylate with a monomer molar ratio of 2:1 (Eudragit NE30D^{®}), copolymers of ethyl acrylate, methyl methacrylate and trimethylammonium ethyl methacrylate-chloride with a monomer molar ratio of 1:2:0.1 (Eudragit RS^{®}), copolymers of ethyl acrylate, methyl methacrylate and trimethylammonium ethyl methacrylate-chloride with a monomer molar ratio of 1:2:0.2. (Eudragit RL^{®}), or a mixture of at least two of the above-mentioned copolymers. These coating materials are commercially available as 30 wt.% aqueous latex dispersions, i.e. as Eudragit RS30D^{®}, Eudragit NE30D^{®} or Eudragit RL30D^{®}, and may also be used as such for coating purposes.

In another embodiment, the sustained-release material is based on water-insoluble cellulose derivatives, preferably alkyl celluloses, particularly preferably ethyl cellulose, or cellulose esters, e.g. cellulose acetate. Aqueous ethyl cellulose dispersions are commercially available, for example, under the trademarks Aquacoat^{®} or Surelease^{®}.

As natural, semisynthetic or synthetic waxes, fats or fatty alcohols, the sustained-release material may be based on carnauba wax, beeswax, glycerol monostearate, glycerol monobehenate, glycerol ditripalmitostearate, microcrystalline wax, cetyl alcohol, cetylstearyl alcohol or a mixture of at least two of these components.

The afored mentioned polymers of the sustained-release material may also comprise a conventional, physiologically acceptable plasticizer in amounts known to those skilled in the art.

Examples of suitable plasticizers are lipophilic diesters of a C₆-C₄₀ aliphatic or aromatic dicarboxylic acid and a C₁-C₈ aliphatic alcohol, e.g. dibutyl phthalate, diethyl phthalate, dibutyl sebacate or diethyl sebacate, hydrophilic or lipophilic citric acid esters, e.g. triethyl citrate, tributyl citrate, acetyltributyl citrate or acetyltriethyl citrate, polyethylene glycols, propylene glycol, glycerol esters, e.g. triacetin, Myvacet^{®} (acetylated mono- and diglycerides, C₂₃H₄₄O₅ to C₂₅H₄₇O₇), medium-chain triglycerides (Miglyol^{®}), oleic acid or mixtures of at least two of said plasticizers.

Aqueous dispersions of Eudragit RS^{®} and optionally Eudragit RL^{®} preferably contain triethyl citrate. The sustained-release material may comprise one or more plasticisers in amounts of, for example, 5 to 50 wt.% based on the amount of polymer(s) used.

The sustained-release material may also contain other conventional auxiliary substances known to those skilled in the art, e.g. lubricants, coloured pigments or surfactants.

The pharmaceutical formulation of the present invention may also comprise at least one of the components (A) and (B) covered by an enteric coating form which dissolves as a function of pH. Because of this coating, part or all of the pharmaceutical formulation can pass through the stomach undissolved and the components (A) and/or (B) are only released in the intestinal tract. The enteric coating preferably dissolves at a pH of between 5 and 7.5.

The enteric coating may be based on any enteric material known to those skilled in the art, e.g. on methacrylic acid/methyl methacrylate copolymers with a monomer molar ratio of 1:1 (Eudragit L^{®}), methacrylic acid/methyl methacrylate copolymers with a monomer molar ratio of 1:2 (Eudragit S^{®}), methacrylic acid/ethyl acrylate copolymers with a monomer molar ratio of 1:1 (Eudragit L30D-55^{®}), methacrylic acid/methyl acrylate/methyl methacrylate copolymers with a monomer molar ratio of 7:3:1 (Eudragit FS^{®}), shellac, hydroxypropyl methyl cellulose acetate-succinates, cellulose acetate-phthalates or a mixture of at least two of these components, which can optionally also be used in combination with the above-mentioned water-insoluble poly(meth)acrylates, preferably in combination with Eudragit NE30D^{®} and/or Eudragit RL^{®} and/or Eudragit RS^{®}.

The coatings of the pharmaceutical formulations of the present invention may be applied by the conventional processes known to those skilled in the art, e.g. from Johnson, J.L., "Pharmaceutical tablet coating", Coatings Technology Handbook (Second Edition), Satas, D. and Tracton, A.A. (Eds), Marcel Dekker, Inc. New York, (2001), 863-866; Carstensen, T., "Coating Tablets in Advanced Pharmaceutical Solids", Swarbrick, J. (Ed.), Marcel Dekker, Inc. New York (2001), 455-468; Leopold, C.S., "Coated dosage forms for colon-specific drug delivery", Pharmaceutical Science & Technology Today, 2(5), 197-204 (1999), Rhodes, C.T. and Porter, S.C., Coatings, in Encyclopedia of Controlled Drug Delivery. Mathiowitz, E. (Ed.), John Wiley & Sons, Inc., New York (1999), Vol. 1; 299-311. The respective descriptions are incorporated by reference and are part of the disclosure.

In another embodiment, the pharmaceutical formulation of the present invention contains one or both of components (A) and (B) not only in sustained-release form, but also in non-sustained-release form. By combination with the immediately released form, a high initial dose can be achieved for the rapid onset of the beneficial effect. The slow release from the sustained-release form then prevents the beneficial effect from diminishing. Such a pharmaceutical formulation is particularly useful for the treatment of acute health problems.

This may be achieved, for example, by a pharmaceutical formulation having at least one immediate-release coating comprising at least one of the components (A) and (B) to provide for rapid onset of the beneficial effect after administration to the patient.

The present invention also relates to the treatment the aforementioned disorders and/or diseases with a combination of at least one compound with 5-HT₆ receptor affinity and at least one NMDA-receptor ligand which may be administered separately, therefore the invention also relates to combining separate pharmaceutical compositions into a kit form. The kit, according to this invention, comprises two separate pharmaceutical compositions: a first unit dosage form comprising a prophylactically or therapeutically effective amount of at least one NMDA-receptor ligand, or a pharmaceutically acceptable salt or ester thereof, and a pharmaceutically acceptable carrier or diluent in a first unit dosage form, and a second unit dosage form comprising a prophylactically or therapeutically effective amount of at least one compound with 5-HT₆ receptor affinity, or a pharmaceutically acceptable salt or ester thereof, and a pharmaceutically acceptable carrier or diluent in a second unit dosage form. The kit further comprises a container. Such kits are especially suited for the delivery of solid oral forms such as tablets or capsules. Such a kit preferably includes a number of unit dosages. Such kits can include a card having the dosages oriented in the order of their intended use. An example of such a kit is a "blister pack". Blister packs are well known in the packaging industry and are widely used for packaging pharmaceutical unit dosage forms. If desired, a memory aid can be provided, for example in the form of numbers, letters, or other markings or with a calendar insert, designating the days or time in the treatment schedule in which the dosages can be administered.

### Example

### Example 1. Test of novel object discrimination in rats

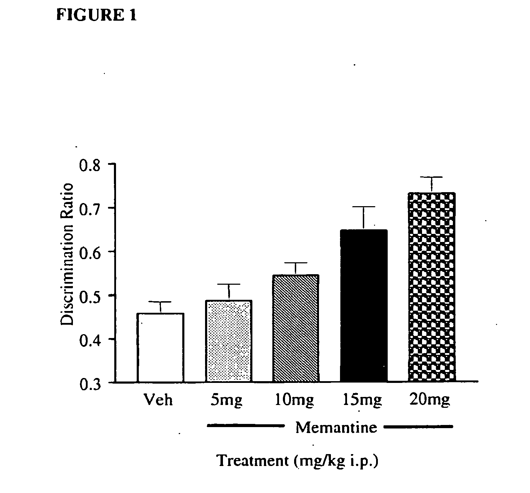

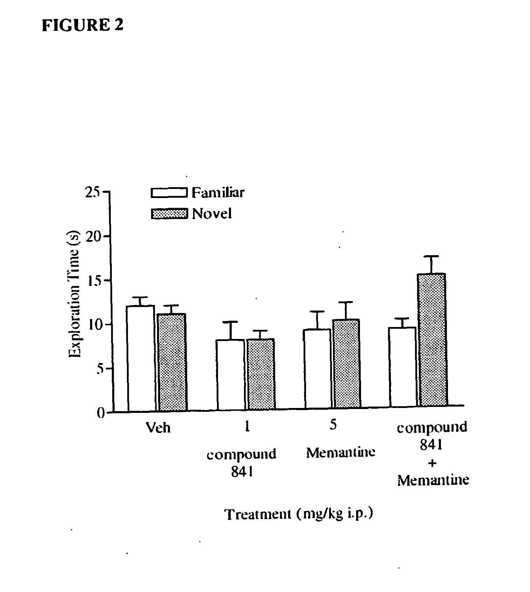

Adult male Lister Hooded rats (Charles River, UK) weighing 200-350g at the start of the experiment were housed in groups of four on a 12:12 h light:dark cycle (lights on at 07:00 h). Food and water were available ad libitum throughout the study, and the room temperature (21 ± 2°C) and relative humidity (45-65%) were kept constant. In one experiment, rats received i.p. injections (2ml/kg, -20 minutes prior to the familiarisation trial) of memantine (n=12) at 5, 10, 15 and 20mg/kg compared with vehicle (0.5% methylcellulose in saline, 2ml/kg), such that all rats received all doses of the compound in a random order with each behavioural test occurring at seven day intervals.
In the combination study, a group of rats (n=12 each) received injection of a sub-effective dose of compound 841 (1mg/kg i.p.) or vehicle (0.5% methylcellulose in saline, 2ml/kg), either alone or combined with memantine (5mg/kg). Each drug combination was administered to all rats in the group over a period of 4 weeks in a random order using a seven day behavioural test interval.
The two trial novel object discrimination paradigm utilised, was as described by Ennanceur and Delacour, (A new one-trial test for neurobiological studies of memory in rats. 1: Behavioral data. Behav Brain Res 31, 47-59, 1988) with minor modification (King et al., "5-ht6 receptor antagonists reverse delay-dependent deficits in novel object discrimination by enhancing consolidation-an effect sensitive to NMDA receptor antagonist", Neuropharmacol. 47, 195-204, 2004; Woolley et al., "Reversal of a cholinergic-induced deficit in a rodent model of recognition memory by the selective 5-ht6 receptor antagonist, Ro 04-6790", Psychopharmacol. 170, 358-367, 2003). The twelve open field test arenas used for object discrimination were clear perspex boxes (39 x 23.5 cm with 24.5 cm high walls) to which each rat was habituated for 60 minutes the day prior to test days. On the test day, for the first experiment, the administration was 20 minutes before acclimatisation. For the combination experiment, the first drug was administered -40 minutes and the second drug -20 minutes before the familiarisation trial. Therefore, 20 minutes after the injection each rat received 3 minutes acclimatisation to the perspex box in absence of objects which was then followed by the 3 minute familiarisation trial and a second 3 minute choice trial following a 4 hour inter-trial interval. During both trials, exploration of each object was defined as the time spent (s) sniffing (within 1 cm of it with active vibrissae), licking, chewing or touching the object with the nose.
The results obtained for the first trial were shown in Figure 1. As can be seen, when memantine is administered alone, high amounts of said compound are necessary for rats to discriminate a novel object against a familiar object. On the contrary, as showm in Figure 2 for the second trial, the combined administration of memantine and compound 841 in lower doses (5 and 1 mg/kg, respectively) makes the animal to spend more time exploring the novel/unknown object than when the object is familiar for him, thus proving a better ability to memorize or distinguish a novel from a known object.

## Claims

1. An active substance combination that comprises
(A) at least one compound which is selected from the group consisting of tetrahydroisoquinoline-derived sulfonamide compounds of general formula (If): 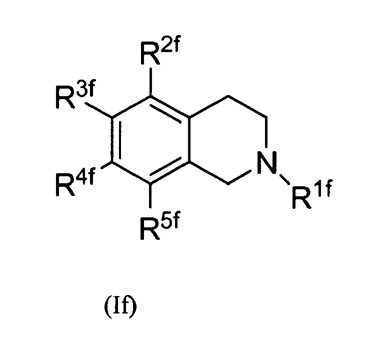 wherein
R^{1f} represents a hydrogen atom; a -C(=O)-OR^{37f} moiety; a radical selected from the group consisting of methyl, ethyl, n-propyl, isopropyl, n-butyl, sec-butyl, isobutyl, tert-butyl, n-pentyl, n-hexyl, -CH₂-NH₂, -CH₂-NH-CH₃, -CH₂-N(CH₃)₂, -CH₂-N(C₂H₅)₂, -CH₂-NH-C₂H₅, -CH₂-CH₂-NH₂, -CH₂-CH₂-NH-CH₃, -CH₂-CH₂-N(CH₃)₂, -CH₂-CH₂-N(C₂Hs)₂, -CH₂-CH₂-NH-C₂Hs, -CH₂-CH₂-CH₂-NH-CH₃, -CH₂-CH₂-CH₂-N(CH₃)₂, -CH₂-CH₂-CH₂-N(C₂H₅)₂ and -CH₂-CH₂-CH₂-NH-C₂H₅; or a (hetero)cycloaliphatic radical selected from the group consisting of imidazolidinyl, aziridinyl, azetidinyl, pyrrolidinyl, piperidinyl, morpholinyl, thiomorpholinyl, piperazinyl, pyrazolidinyl and azepanyl, which may be bonded via a -(CH₂)_{1, 2 or 3}- group and which may be unsubstituted or optionally substituted with 1, 2, 3, 4 or 5 substituent(s) independently selected from the group consisting of methyl, ethyl, n-propyl, isopropyl, n-butyl, tert-butyl, sec-butyl and isobutyl;
R^{2f}, R^{3f}, R^{4f} and R^{5f}, independently of one another, each represent a hydrogen atom; F, Cl, Br, I, -NO₂; -O-CH₃; -O-C₂H₅; -O-CF₃; -O-CFH₂; -O-CF₂H; -O-CH₂-CF₃; -O-CF₂-CF₃; -S-CH₃; -S-C₂H₅; -S-CF₃; -S-CFH₂; -S-CF₂H; -S-CH₂-CF₃; -S-CF₂-CF₃; -N(R^{11f})-S(=O)₂-R^{12f}; or a radical selected from the group consisting of methyl, ethyl, n-propyl, isopropyl, tert-butyl, -CF₃, -CFH₂, -CF₂H, -CH₂-CF₃ and -CF₂-CF₃;
with the proviso that at least one of the substituents R^{2f}, R^{3f}, R^{4f} and R^{5f} represents a -N(R^{11f})-S(=O)₂-R^{12f} moiety;
R^{11f} represents a hydrogen atom, -S(=O)₂-R^{12f} or an alkyl radical selected from the group consisting of methyl, ethyl, n-propyl, isopropyl, n-butyl, sec-butyl, isobutyl and tert-butyl;
R^{12f} represents a phenyl radical of general formula (Af), 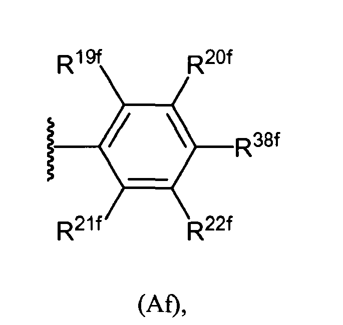 wherein
R¹⁹ ; R^{20f}, R^{21f}, R^{22f} and R^{38f}, independently of one another, each represent a hydrogen atom; F, Cl, Br, I, -NO₂; -NH₂; -SH; -OH; -CN; -C(=O)-OH; -C(=O)-H; -C(=O)-CH₃; -C(=O)-C₂H₅; -O-CH₃; -O-C₂H₅; -O-CF₃; -O-CFH₂; -O-CF₂H; - O-CH₂-CF₃; -O-CF₂-CF₃; -S-CH₃; -S-C₂H₅; -S-CF₃; -S-CFH₂; -S-CF₂H; -S-CH₂-CF₃; -S-CF₂-CF₃; -C(=O)-OCH₃; -C(=O)-OC₂H₅; methyl, ethyl, n-propyl, isopropyl, n-butyl, isobutyl, sec-butyl, tert-butyl, -CF₃, -CF₂H, -CFH₂, -CH₂-CF₃ and -CF₂-CF₃;
a radical selected from the group consisting of naphthyl, [1,3]-benzodioxolyl, [1,4]-benzodioxanyl, benzo[b]furanyl, benzo[b]thiophenyl, benzo[2,1,3]thiadiazolyl, [1,2,3]-benzothiadiazolyl, [2,1,3]-benzoxadiazolyl, [1,2,3]-benzoxadiazolyl, benzoxazolyl, benzothiazolyl, benzisoxazolyl, benzisothiazolyl and imidazo[2,1-b]thiazolyl, which may be unsubstituted or optionally substituted with 1, 2, 3, 4 or 5 substituent(s) independently selected from the group consisting of methyl, ethyl, n-propyl, isopropyl, n-butyl, tert-butyl, sec-butyl, isobutyl, n-pentyl, -O-CH₃, -O-C₂Hs, F, Cl, Br, I, -CN, -CF₃, - OCF₃, -SCF₃, -CF₂H and -CFH₂;
or a radical selected from the group consisting of pyridinyl, pyrrolyl, imidazolyl, pyrazolyl, triazolyl, pyridazinyl, pyrimidinyl and pyrazinyl, which may be unsubstituted or optionally substituted with 1, 2, 3, 4 or 5 substituent(s) independently selected from the group consisting of F, Cl, Br, I, -NO₂; methyl, ethyl, n-propyl, isopropyl, n-butyl, isobutyl, sec-butyl, tert-butyl, -CF₃, -CF₂H, - CFH₂, -CH₂-CF₃ and -CF₂-CF₃;
and R^{37f} represents a radical selected from the group consisting of methyl, ethyl, n-propyl, isopropyl, n-butyl, sec-butyl, isobutyl, tert-butyl, n-pentyl, n-hexyl, fluorenyl, fluorenylmethyl, phenyl, benzyl and naphthyl;
optionally in form of one of its stereoisomers, preferably enantiomers or diastereomers, a racemate or in form of a mixture of at least two of its stereoisomers, preferably enantiomers and/or diastereomers, in any mixing ratio, or a salt thereof, or a corresponding solvate thereof;
and compounds of general formula (Ih) 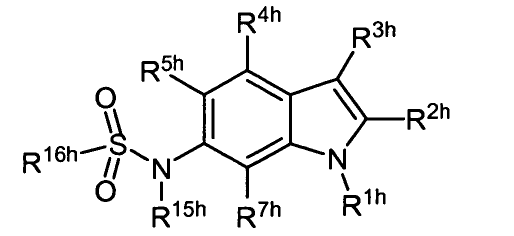 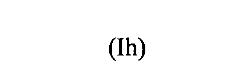 wherein
R^{1h} represents a hydrogen atom or a -(CH₂)ₘₕ-NR^{13h}R^{14h} radical,
R^{2h} and R^{7h} each represent hydrogen,
R^{3h} represents a hydrogen atom, 1-methyl-piperidin-4-yl or a -(CH₂)ₙₕ-NR^{13h}R^{14h} moiety with nh = 0, 1 or 2,
R^{4h} represents chlorine, bromine or a hydrogen atom,
R^{5h} represents -C(=O)-O-C₂H₅ or a hydrogen atom,
R^{15h} represents hydrogen or a -S(=O)₂-R^{16h} moiety,
R^{13h} and R^{14h}, identical or different, each represent methyl, ethyl, isopropyl or n-propyl, more preferably methyl,
or
R^{13h} and R^{14h}, together with the bridging nitrogen atom form a 5- or 6-membered heterocyclic ring, more preferably form a pyrrolidine ring or a piperidine ring,
and
R^{16h} represents an aryl or heteroaryl radical selected from the group consisting of phenyl, naphthyl, thiophenyl, benzo[b]furanyl, benzo[b]thiophenyl and imidazo[2,1-b]thiazolyl which may be substituted by 1, 2 or 3 substituents selected from the group consisting of chlorine, methyl, phenyl and -O-phenyl and/or which may be bonded via a C₁₋₂ alkylene group,
and mh is 0, 1 or 2,
optionally in form of one of its stereoisomers, preferably enantiomers or diastereomers, a racemate or in form of a mixture of at least two of its stereoisomers, preferably enantiomers and/or diastereomers, in any mixing ratio, or a salt thereof, preferably a corresponding, physiologically acceptable salt thereof, or a corresponding solvate thereof;
and compounds of general formula (Ik) 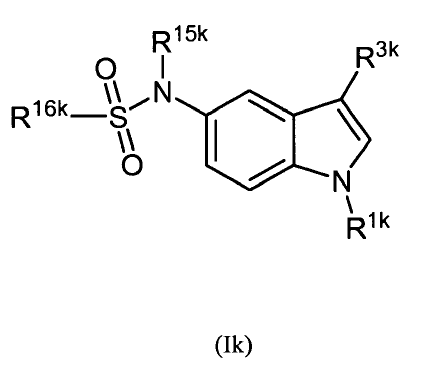 wherein
nk represents 0, 1, 2, 3 or 4;
R^{1k} represents hydrogen,
R^{3k} represents a -NR^{13k}R^{14k} moiety or a moiety selected from the group consisting of 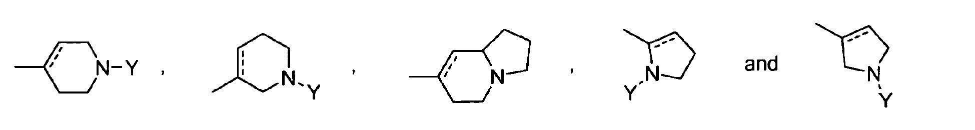
wherein, if present, the dotted line represents an optional chemical bond and Y represents hydrogen, a methyl group or an ethyl group,
R^{15k} represents hydrogen, a methyl group or an ethyl group,
R^{13k} and R^{14k}, identical or different, represent a methyl group, an ethyl group, an n-propyl group, an isopropyl group, an n-butyl group, an isobutyl group, a sec-butyl group, or a tert-butyl group, or
R^{13k} and R^{14k} together with the bridging nitrogen atom form a moiety selected from the group consisting of 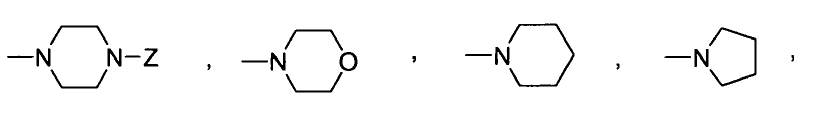 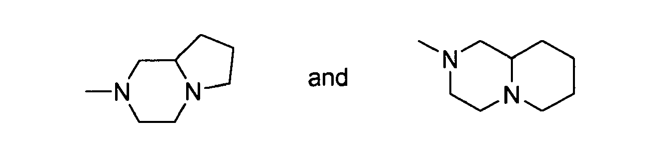
wherein Z represents hydrogen, a methyl group or an ethyl group,
R^{16k} represents a moiety selected from the group consisting of 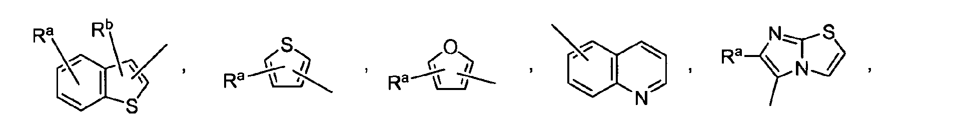 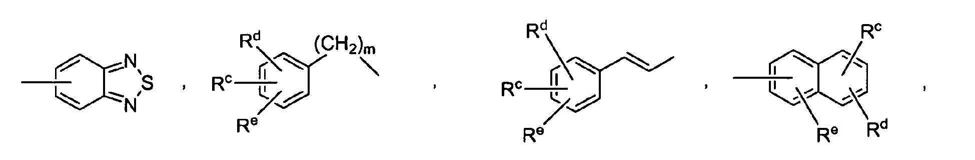 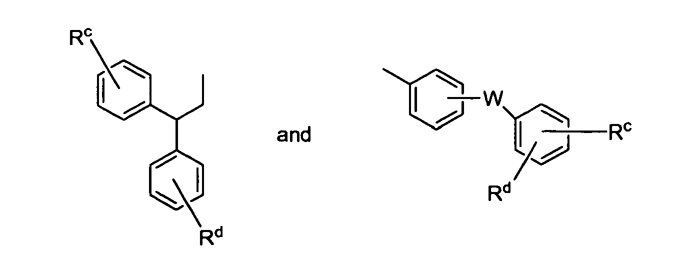
wherein
R^{a} and R^{b} are each independently selected from the group consisting of hydrogen, fluorine, chlorine, bromine, methyl, ethyl, pyridinyl, thiophenyl and furyl,
R^{c}, R^{d} and R^{e} are each independently selected from the group consisting of hydrogen, fluorine, chlorine, bromine, methyl, ethyl, methoxy, ethoxy and -CF₃,
W represents a single chemical bond between the two rings, a CH₂-group, O, S or a NR^{f}-moiety, wherein R^{f} is hydrogen, methyl or ethyl,
m is 0, 1, 2, 3 or 4;
optionally in form of one of its stereoisomers, preferably enantiomers or diastereomers, a racemate or in form of a mixture of at least two of its stereoisomers, preferably enantiomers and/or diastereomers, in any mixing ratio, or a salt thereof, preferably a corresponding, physiologically acceptable salt thereof, or a corresponding solvate thereof,
and
(B) memantine.

2. The combination according to claim 1, **characterized in that** the binding of compounds present as component (A) to the 5-HT₆-receptor is determined by a Kᵢ value of less than 7000 nM, preferably of less than 200 nM, more preferably of less than 100 nM.

3. The combination according to any of claims 1 to 2, **characterized in that** as component (A) at least one compound is present which is selected from
[788] N-[1-(2-Dimethylaminoethyl)-lH-indol-6-yl]-5-chloro-3-methylbenzo[b]thiophene-2-sulfonamide,
[789] N-[1-(2-Dimethylaminoethyl)-1H-indol-6-yl]-naphthalene-2-sulfonamide,
[790] N-[1-(2-Dimethylaminoethyl)-1H-indol-6-yl]-naphthalene-1-sulfonamide,
[791] N-[1-(2-Dimethylaminoethyl)-1H-indol-6-yl]-6-chloroimidazo[2,1-b]thiazole-5-sulfonamide,
[792] N-[1-(2-Dimethylaminoethyl)-1H-indol-6-yl]-4-phenylbenzenesulfonamide,
[793] N-[1-(2-Dimethylaminoethyl)-1H-indol-6-yl]-2-(naphthalene-1-yl)-ethanesulfonamide,
[794] N-[1-(2-Dimethylaminoethyl)-1H-indol-6-yl]-4-phenoxybenzenesulfonamide,
[795] N-[1-(2-Dimethylaminoethyl)-1H-indol-6-yl]-3,5-dichlorobenzenesulfonamide,
[796] 5-Chloro-3-methyl-N-[1-[2-(pyrrolidin-1-yl)ethyl-1H-indol-6-y1]-benzo[b]thiophene-2-sulfonamide,
[797] N-(1-[2-(Pyrrolidin-1-yl)ethyl]-1H-indol-6-yl]-naphthyl-2-sulfonamide,
[798] N-[1-[2-Pyrrolidin-1-yl]ethyl]-1H-indol-6-yl]-naphthalene-1-sulfonamide,
[799] 6-Chloro-N-[1-[2-(pyrrolidin-1-yl)ethyl]-1H-indol-6-yl]-imidazo[2,1-b]thiazole-5-sulfonamide,
[800] 4-Phenyl-N-(1-(2-(pyrrolidin-1-yl)ethyl)-1H-indol-6-yl)-benzenesulfonamide,
[801] 2-(Naphth-1-yl)-N-(1-(2-(pyrrolidin-1-yl)ethyl)-1H-indol-6-yl)-ethansulfonamide,
[802] 4-Phenoxy-N-(1-(2-(pyrrolidin-1-yl)ethyl)-1H-indol-6-yl)-benzenesulfonamide,
[803] 3,5-Dichloro-N-(1-(2-(pyrrolidin-1-yl)-1H-indol-6-yl)-benzenesulfonamide,
[804] 5-chloro-N-(3-(2-(diethylamino)ethyl)-1H-indol-6-yl)-3-methylbenzo[b]thiophene-2-sulfonamide,
[805] N-(3-(2-(diethylamino)ethyl)-1H-indol-6-yl)naphthalene-2-sulfonamide,
[806] N-(3-(2-(diethylamino)ethyl)-1H-indol-6-yl)naphthalene-1-sulfonamide,
[807] 6-chloro-N-(3-(2-(diethylamino)ethyl)-1H-indol-6-yl)imidazo[2,1-b]thiazole-5-sulfonamide,
[808] N-(3-(2-(diethylamino)ethyl)-1H-indol-6-yl)-4-phenylbenzenesulfonamide,
[809] N-(3-(2-(diethylamino)ethyl)-1H-indol-6-yl)-4-phenoxybenzenesulfonamide,
[810] 3,5-dichloro-N-(3-(2-(diethylamino)ethyl)-1H-indol-6-yl)benzenesulfonamide,
[811] 4,5-dichloro-N-(3-(2-(diethylamino)ethyl)-1H-indol-6-yl)thiophene-2-sulfonamide,
[812] 5-chloro-N-(3-(2-(diethylamino)ethyl)-1H-mdol-6-yl)naphthalene-1-sulfonamide,
[813] 5-chloro-N-(3-(2-(dimethylamino)ethyl)-1H-indol-6-yl)-3-methylbenzo[b]thiophene-2-sulfonamide,
[814] N-(3-(2-(dimethylamino)ethyl)-1H-indol-6-yl)naphthalene-2-sulfonamide,
[815] N-(3-(2-(dimethylamino)ethyl)-1H-indol-6-yl)naphthalene-1-sulfonamide,
[816] 6-chloro-N-(3-(2-(dimethylamino)ethyl)-1H-indol-6-yl)imidazo[2,1-b]thiazole-5-sulfonamide,
[817] N-(3-(2-(dimethylamino)ethyl)-1H-indol-6-yl)-4-phenylbenzenesulfonamide,
[818] N-(3-(2-(dimethylamino)ethyl)-1H-indol-6-yl)-2-(naphthalen-1-yl)ethanesulfonamide,
[819] N-(3-(2-(dimethylamino)ethyl)-1H-indol-6-yl)-4-phenoxybenzenesulfonamide,
[820] 3,5-dichloro-N-(3-(2-(dimethylamino)ethyl)-1H-indol-6-yl)benzenesulfonamide,
[821] 4,5-dichloro-N-(3-(2-(dimethylamino)ethyl)-1H-indol-6-yl)thiophene-2-sulfonamide,
[822] 5-chloro-N-(3-(2-(dimethylamino)ethyl)-1H-indol-6-yl)naphthalene-1-sulfonamide,
[823] 6-bis(6-chloroimidazo[2,1-b]thiazol-5-ylsulfonyl)amino-3-(2-(dimethylamino)ethyl)-1H-indole,
[824] 6-bis(3,5-dichlorobenzenesulfonyl)amino-3-(2-(dimethylamino)ethyl)-1H-indole,
[825] 6-bis(4,5-dichlorothiophene-2-sulfonyl)amino-3-(2-(dimethylamino)ethyl)-1H-indo le,
[826] 6-bis(5-chloro-3-methylbenzo[b]thiophene-2-sulfonyl)amino-3-(2-(dimethylamino)-1-ethoxyethyl)-1H-indole,
[827] Ethyl 6-(5-chloro-3-methylbenzo[b]thiophene-2-sulfonamido)-3-(1-methylpiperidin-4-yl)-1H-indole-5-carboxylate,
[828] N-(4-bromo-3-(1-methylpiperidin-4-yl)-1H-indol-6-yl)naphthalene-1-sulfonamide,
[829] N-(7-bromo-3-(2-(dimethylamino)ethyl)-1H-indol-5-yl)benzofuran-2-sulfonamide,
[830] N-(7-methoxy-3-(2-(pyrrolidin-1-yl)ethyl)-1H-indol-5-yl)benzo[c][1,2,5]thiadiazole-4-sulfonamide,
[831] N-(7-methoxy-3-(2-(pyrrolidin-1-yl)ethyl)-1H-indol-5-yl)naphthalene-2-sulfonamide,
[832] 6-chloro-N-(7-methoxy-3-(2-(pyrrolidin-1-yl)ethyl)-1H-indol-5-yl)imidazo[2,1-b]thiazole-5-sulfonamide,
[833] N-[3-(2-diethylaminoethyl)-1H-indol-5-yl]-5-chloro-3-methylbenzo[b]thiophene-2-sulphonamide,
[834] N-[3-(2-diethylaminoethyl)-1H-indol-5-yl]naphthalene-1-sulphonamide,
[835] Hydrochloride N-[3-(2-diethylaminoethyl)-1*H*-indol-5-yl]naphthalene-1-sulphonamide,
[836] N-[3-(2-diethylaminoethyl)-1*H*-indol-5-yl]-3,5-dichlorobenzenesulphonamide,
[837] N-[3-(2-diethylaminoethyl)-1*H*-indol-5-yl]-4-phenylbenzenesulphonamide,
[838] N-[3-(2-diethylaminoethyl)-1*H*-indol-5-yl]-5-chlorothiophene-2-sulphonamide;
[839] N-[3-(2-dimethylaminoethyl)-1*H*-indol-5-yl]-5-chloro-3-methylbenzo[b]thiophene-2-sulphonamide,
[840] N-[3-(2-dimethylaminoethyl)-1*H*-indol-5-yl]naphthalene-1-sulphonamide,
[841] N-[3-(2-dimethylamino-ethyl)-1*H*-indol-5-yl]-6-chloroimidazo[2,1-b]thiazol-5-sulphonamide,
[842] N-[3-(1-methylpiperidin-4-yl)-1*H*-indol-5-yl]-5-chloro-3-methylbenzo[b]thiophene-2-sulphonamide,
[843] N-[3-(1-methylpiperidin-4-yl)-1*H*-indol-5-yl]-5-chloro-3-methylbenzo[b]thiophene-2-sulphonamide hydrochloride,
[844] N-[3-(1-methylpiperidin-4-yl)-1*H*-indol-5-yl]naphthalene-1-sulphonamide,
[845] N-[3-(1-methylpiperidin-4-yl)-1*H*-indol-5-yl]naphthalene-1-sulphonamide hydrochloride,
[846] N-[3-(1-methylpiperidin-4-yl)-1*H*-indol-5-yl]-5-chlorothiophene-2-sulphonamide,
[847] N-[3-(1-methylpiperidin-4-yl)-1*H*-indol-5-yl]-4-phenylbenzenesulphonamide,
[848] N-[3-(1-methylpiperidin-4-yl)-1*H*-indol-5-yl]quinoline-8-sulphonamide,
[849] N-[3-(2-diethylaminoethyl)-1*H*-indol-5-yl]naphthalene-2-sulphonamide,
[850] N-[3-(1-methyl-1,2,3,6-tetrahydropyridin-4-yl)-1*H*-indol-5-yl]naphthalene-1-sulphonamide,
[851] N-[3-(4-methylpiperazin-1-yl)methyl-1*H*-indol-5-yl]-5-chloro-3-methylbenzo[b]thiophene-2-sulphonamide,
[852] N-[3-(2-dimethylaminoethyl)-1*H*-indol-5-yl]-5-(2-pyridil)thiophene-2-sulphonamide,
[853] N-[3-(2-dimethylaminoethyl)-1*H*-indol-5-yl]-2,1,3-benzothiadiazol-4-sulphonamide,
[854] N-[3-(2-dimethylaminoethyl)-1*H*-indol-5-yl]quinoline-8-sulphonamide,
[855] N-[3-(2-dimethylaminoethyl)-1*H*-indol-5-yl]-5-chloronaphthalene-2-sulphonamide,
[856] N-[3-(2-dimethylaminoethyl)-1*H*-indol-5-yl]-4-phenoxybenzenesulphonamide,
[857] N-[3-(2-dimethylaminoethyl)-1*H*-indol-5-yl]-4-phenylbenzenesulphonarnide,
[858] N-[3-(2-diethylaminoethyl)-1*H*-indol-5-yl]-N-ethyl-naphthalene-2-sulphonamide,
[859] N-{3-[2-(morpholin-4-yl)ethyl]-1H-indol-5-yl}-5-chloro-3-methylbenzo[b]thiophene-2-sulphonamide,
[860] N-{3-[2-(morpholin-4-yl)ethyl]-1H-indol-5-yl}naphthalene-1-sulphonamide,
[861] N-[3-(2-diethylaminoethyl)-1H-indol-5-yl]naphthalene-2-sulphonamide,
[862] N-[3-dimethylaminomethyl-1H-indol-5-yl]-5-chloro-3-methylbenzo[b]thiophene-2-sulphonamide,
[863] N-[3-(2-dipropylaminoethyl)-1H-indol-5-yl]naphthalene-1-sulphonamide,
[864] N-[3-(2-dipropylaminoethyl)-1H-indol-5-yl]-5-chloro-3-methylbenzo[b]thiophene-2-sulphonamide,
[865] N-[3-(2-dibutylaminoethyl)-1H-indol-5-yl]-5-chloro-3-methylbenzo[b]thiophene-2-sulphonamide,
[866] N-[3-(2-dibutylaminoethyl)-1H-indol-5-yl]naphthalene-1-sulphonamide,
[867] N-[3-(2-diethylaminoethyl)-1H-indol-5-yl]-5-chloronaphthalene-1-sulphonamide,
[868] N-[3-(2-diethylaminoethyl)-1H-indol-5-yl]-trans-β-styrenesulphonamide,
[869] N-[3-(4-methylpiperazin-1-yl)methyl-1*H*-indol-5-yl]-trans-β-styrenesulphonamide,
[870] N-[3-(octahydroindolizin-7-yl)-1H-indol-5-yl]-5-chloro-3-methylbenzo[b]thiophene-2-sulphonamide,
[871] N-[3-(2-diethylaminoethyl)-1*H*-indol-5-yl]-6-chloroimidazo[2,1-b]thiazol-5-sulphonamide,
[872] N-{3-[2-(morpholin-4-yl)ethyl]-1H-indol-5-yl}naphthalene-2-sulphonamide,
[873] N-[3-(4-methylpiperazin-1-yl)methyl-1*H*-indol-5-yl]-α-toluenesulphonamide,
[874] N-[3-(3-diethylaminopropyl)-1H-indol-5-yl]naphthalene-2-sulphonamide,
[875] N-[3-(3-diethylaminopropyl)-1H-indol-5-yl]-5-chloro-3-methylbenzo[b]thiophene-2-sulphonamide,
[876] N-{3-[2-(pyrrolidin-1-yl)ethyl]-1H-indol-5-yl}-5-chloro-3-methylbenzo[b]thiophene-2-sulphonamide,
[877] N-(3-[2-(pyrrolidin-1-yl)ethyl]-1H-indol-5-yl}naphthalene-1-sulphonamide,
[878] N-{3-[2-(pyrrolidin-1-yl)ethyl]-1H-indol-5-yl}naphthalene-2-sulphonamide,
[879] N-[3-(2-dipropylaminoethyl)-1H-indol-5-yl]naphthalene-2-sulphonamide,
[880] N-[3-(2-dimethylaminoethyl)-1H-indol-5-yl]-5-chloronaphthalene-1-sulphonamide,
[881] N-[3-(2-dimethylaminoethyl)-1H-indol-5-yl]naphthalene-2-sulphonamide,
[882] N-{3-[2-(morpholin-4-yl)ethyl]-lH-indol-5-yl}quinoline-8-sulphonamide,
[883] N-{3-[2-(morpholin-4-yl)ethyl]-1H-indol-5-yl}-4-phenylbenzenesulphonamide,
[884] N-[3-(4-methylpiperazin-1-yl)ethyl-1*H*-indol-5-yl]naphthalene-2-sulphonamide,
[885] N-[3-(4-methylpiperazin-1-yl)ethyl-1*H*-indol-5-yl]-5-chloronaphthalene-1-sulphonamide,
[1114] N-(1,2,3,4-tetrahydroisoquinolin-6-yl)naphthalene-1-sulfonamide hydrochloride,
[1115] 2,2-dimethyl-6-(N-methylnaphthalene-1-sulfonamido)-1,2,3,4-tetrahydroisoquinolinium iodide,
[1116] N-(2-methyl-1,2,3,4-tetrahydroisoquinolin-6-yl)naphthalene-1-sulfonamide hydrochloride,
[1117] 5-chloro-3-methyl-N-(1,2,3,4-tetrahydroisoquinolin-6-yl)benzo[b]thiophene-2-sulfonamide hydrochloride,
[1118] 5-chloro-3-methyl-N-(2-methyl-1,2,3,4-tetrahydroisoquinolin-6-yl)benzo[b]thiophene-2-sulfonamide hydrochloride,
[1119] 4-methyl-N-(1,2,3,4-tetrahydroisoquinolin-6-yl)naphthalene-1-sulfonamide hydrochloride,
[1120] 4-methyl-N-(2-methyl-1,2,3,4-tetrahydroisoquinolin-6-yl)naphthalene-1-sulfonamide hydrochloride,
[1121] N-(1,2,3,4-tetrahydroisoquinolin-6-yl)naphthalene-2-sulfonamide hydrochloride,
[1122] N-(2-methyl-1,2,3,4-tetrahydroisoquinolin-6-yl)naphthalene-2-sulfonamide hydrochloride,
[1123] 6-chloro-N-(1,2,3,4-tetrahydroisoquinolin-6-yl)imidazo[2,1-b]thiazole-5-sulfonamide hydrochloride,
[1124] 2-methoxy-5-methyl-N-(1,2,3,4-tetrahydroisoquinolin-6-yl)benzenesulfonamide hydrochloride,
[1125] N-(1,2,3,4-tetrahydroisoquinolin-6-yl)pyridine-3-sulfonamide dihydrochloride,
[1126] 6-(naphthalene-1-sulfonylamino)-3,4-dihydro-1H-isoquino line-2-carboxylic acid tert-butyl ester,
[1127] 6-(5-chloro-3-methyl-benzo[b]thiophene-2-sulfonylamino)-3,4-dihydro-1H-isoquinoline-2-carboxylic acid tert-butyl ester,
[1128] 6-(4-methyl-naphthalene-1-sulfonylamino)-3,4-dihydro-1H-isoquinoline-2-carboxylic acid tert-butyl ester,
[1129] 6-(naphthalene-2-sulfonylamino)-3,4-dihydro-1H-isoquinoline-2-carboxylic acid tert-butyl ester,
[1130] 6-(2-methoxy-5-methyl-benzenesulfonylamino)-3,4-dihydro-1H-isoquinoline-2-carboxylic acid tert-butyl ester and
[1131] 6-(pyridine-3-sulfonylamino)-3,4-dihydro-1H-isoquinoline-2-carboxylic acid tert-butyl ester;
optionally in form of one of its stereoisomers, preferably enantiomers or diastereomers, a racemate or in form of a mixture of at least two of its stereoisomers, preferably enantiomers and/or diastereomers, in any mixing ratio, or a salt thereof, or a corresponding solvate thereof.

4. The combination according to claim 3, **characterized in that** as component (A) at least one compound is present which is selected from
[816] 6-chloro-N-(3-(2-(dimethylamino)ethyl)-1H-indol-6-yl)imidazo[2,1-b]thiazole-5-sulfonamide,
[841] N-[3-(2-dimethylamino-ethyl)-1H-indol-5-yl]-6-chloroimidazo[2,1-b]thiazol-5-sulphonamide, and
[1123] 6-chloro-N-(1,2,3,4-tetrahydroisoquinolin-6-yl)imidazo[2,1-b]thiazole-5-sulfonamide hydrochloride;
optionally in form of one of its stereoisomers, preferably enantiomers or diastereomers, a racemate or in form of a mixture of at least two of its stereoisomers, preferably enantiomers and/or diastereomers, in any mixing ratio, or a salt thereof, or a corresponding solvate thereof.

5. The combination according to one or more of claims 1 to 4, **characterized in that** it comprises 1 - 99% by weight of component (A) and 99 - 1% by weight of component (B), more preferably 10 - 80% by weight of component (A) and 80 - 20% by weight of component (B), in each case referring to the total weight of both components (A) and (B).

6. A combination as defined in one or more of claims 1 to 5 for use as medicament.

7. A combination as defined in one or more of claims 1 to 5 for use as medicament for simultaneous NMDA-receptor inhibition and 5-HT₆-receptor regulation.

8. A combination as defined in one or more of claims 1 to 5 for use as medicament for the prophylaxis and/or treatment of disorders related to food ingestion, preferably for prophylaxis and/or treatment of obesity, anorexia, cachexia, bulimia, diabetes, preferably type II diabetes (non-insulin-dependent diabetes mellitus), or for prophylaxis and/or treatment of gastrointestinal tract disorders, preferably of the irritable bowel syndrome, for prophylaxis and/or treatment of Metabolic Syndrome, Peripheral Nervous System Disorders, Central Nervous System Disorders, arthritis, epilepsy, anxiety, panic, depression, cognitive disorders, memory disorders, cardiovascular diseases, senile dementia processes, such as Alzheimer's, Parkinson's and/or Huntington's Disease, schizophrenia, psychosis, infantile hyperkinesia (ADHD, attention deficit / hyperactivity disorder), pain, hypertensive syndrome, inflammatory diseases, immunologic diseases or for improvement of cognition.

9. A pharmaceutical formulation, **characterized in that** it comprises an active substance combination according to one or more of claims 1 to 5 and optionally one or more pharmacologically acceptable adjuvants.

10. The pharmaceutical formulation according to claim 9, **characterized in that** it is present in solid pharmaceutical forms such as tablets, chewing tablets, chewing gums, dragées, capsules, suppositories, powder preparations, transdermal therapeutic systems, transmucosal therapeutic systems, or in liquid and semi-liquid pharmaceutical forms such as drops or such as juice, sirup, solution, emulsion, suspension, preferably in form of tablets, capsules, drops or solution.

11. The pharmaceutical formulation according to claim 9, **characterized in that** it is present in form of multiple particles, preferably microtablets, microcapsules, microspheroids, granules, crystals or pellets, optionally compacted in a tablet, filled in a capsule or suspended in a suitable liquid.

12. The pharmaceutical formulation according to one or more of claims 9 to 11, **characterized in that** it is for oral, intravenous, intramuscular, subcutaneous, intrathecal, epidural, buccal, sublingual, pulmonal, rectal, transdermal, nasal or intracerebroventricular application, preferably oral or intravenous.

13. The pharmaceutical formulation according to one or more of claims 9 to 11, **characterized in that** at least one of the components of the active substance combination (A) or (B) is present at least partially in sustained-release form.

14. The pharmaceutical formulation according to claim 13, **characterized in that** the medicament has at least one coating or at least one matrix comprising at least one material, which sustains active substance release.

15. The pharmaceutical formulation according to claim 14, **characterized in that** the sustained-release material is based on optionally modified, water-insoluble, natural, semisynthetic or synthetic polymer, or a natural wax or fat or fatty alcohol or semisynthetic or synthetic fatty acid, or on a mixture of at least two of these afore mentioned components.

16. The pharmaceutical formulation according to claim 15, **characterized in that** the water-insoluble polymer is based on an acrylic resin, which is preferably selected from the group of poly(meth)acrylates, poly(C₁₋₄)dialkylamino(C₁₋₄)alkyl (meth)acrylates and/or copolymers thereof or a mixture of at least two of the afore-mentioned polymers.

17. The pharmaceutical formulation according to claim 15, **characterized in that** the water-insoluble polymers are cellulose derivatives, preferably alkyl cellulose and even more preferably ethyl cellulose, or cellulose esters.

18. The pharmaceutical formulation according to claim 15, **characterized in that** the wax is carnauba wax, beeswax, glycerol monostearate, glycerol monobehenate, glycerol ditripalmitostearate, microcrystalline wax or a mixture of at least two of these components.

19. The pharmaceutical formulation according to one or more of claims 15 to 18, **characterized in that** polymers have been used in combination with one or more plasticizers.

20. The pharmaceutical formulation according to one or more of claims 13 to 19, **characterized in that** besides the sustained-release form, at least one of the active substance components (A) or (B) is present in a non-sustained-release form.

## Patentansprüche

1. Eine Wirkstoffkombination, die umfasst
(A) mindestens eine Verbindung, die ausgewählt ist aus der Gruppe bestehend aus von Tetrahydroisochinolin abgeleiteten Sulfonamidverbindungen der allgemeinen Formel (If): 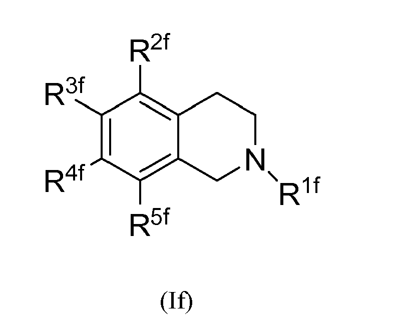 wobei
R^{1f} für ein Wasserstoffatom; eine -C(=O)-OR^{37f} Einheit; einen Rest, ausgewählt aus der Gruppe bestehend aus Methyl, Ethyl, n-Propyl, Isopropyl, n-Butyl, sec-Butyl, Isobutyl, tert-Butyl, n-Pentyl, n-Hexyl, -CH₂-NH₂, -CH₂-NH-CH₃, -CH₂-N(CH₃)₂, -CH₂-N(C₂H₅)₂, -CH₂-NH-C₂H₅, -CH₂-CH₂-NH₂, -CH₂-CH₂-NH-CH₃, -CH₂-CH₂-N(CH₃)₂, -CH₂-CH₂-N(C₂H₅)₂, -CH₂-CH₂-NH-C₂H₅, -CH₂-CH₂-CH₂-NH-CH₃, -CH₂-CH₂-CH₂-N(CH₃)₂, -CH₂-CH₂-CH₂-N(C₂H₅)₂ und -CH₂-CH₂-CH₂-NH-C₂H₅; oder einen (hetero)cycloaliphatischen Rest steht, ausgewählt aus der Gruppe bestehend aus Imidazolidinyl, Aziridinyl, Azetidinyl, Pyrrolidinyl, Piperidinyl, Morpholinyl, Thiomorpholinyl, Piperazinyl, Pyrazolidinyl und Azepanyl, der über eine -(CH₂)₁, ₂ oder ₃- Gruppe gebunden sein kann und der unsubstituiert oder gegebenenfalls mit 1, 2, 3, 4 oder 5 Substituenten substituiert sein kann, unabhängig ausgewählt aus der Gruppe bestehend aus Methyl, Ethyl, n-Propyl, Isopropyl, n-Butyl, tert-Butyl, sec-Butyl und Isobutyl;
R^{2f}, R^{3f}, R^{4f} und R^{5f} unabhängig voneinander jeweils für ein Wasserstoffatom; F, Cl, Br, I, -NO₂; -O-CH₃; -O-C₂H₅, -O-CF₃; -O-CFH₂; -O-CF₂H; -O-CH₂-CF₃; -O-CF₂-CF₃; -S-CH₃; -S-C₂H₅, -S-CF₃; -S-CFH₂; -S-CF₂H; -S-CH₂-CF₃; -S-CF₂-CF₃; -N(R^{11f})-S(=O)₂-R^{12f}; oder einen Rest stehen, ausgewählt aus der Gruppe bestehend aus Methyl, Ethyl, n-Propyl, Isopropyl, tert-Butyl, -CF₃, -CFH₂, -CF₂H, -CH₂-CF₃ und -CF₂-CF₃;
mit der Maßgabe, dass mindestens einer der Substituenten R^{2f}, R^{3f}, R^{4f} und R^{5f} für eine -N(R^{11f})-S(=O)₂-R^{12f} Einheit steht;
R^{11f} für ein Wasserstoffatom, -S(=O)₂-R^{12f} oder einen Alkylrest steht, ausgewählt aus der Gruppe bestehend aus Methyl, Ethyl, n-Propyl, Isopropyl, n-Butyl, sec-Butyl, Isobutyl und tert-Butyl;
R^{12f} für eine Phenylrest der allgemeinen Formel (Af) steht, 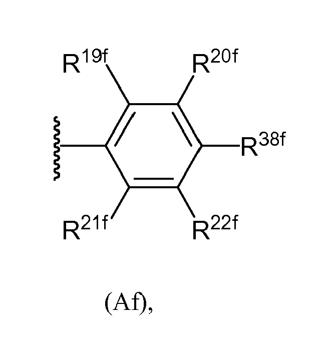 wobei
R^{19f}, R^{20f}, R^{21f}, R^{22f} und R^{38f} unabhängig voneinander jeweils für ein Wasserstoffatom; F, Cl, Br, I, -NO₂; -NH₂; -SH; -OH; -CN; -C(=O)-OH; - C(=O)-H; -C(=O)-CH₃; -C(=O)-C₂H₅; -O-CH₃; -O-C₂H₅, -O-CF₃; -O-CFH₂; -O-CF₂H; -O-CH₂-CF₃; -O-CF₂-CF₃; -S-CH₃; -S-C₂H₅, -S-CF₃; -S-CFH₂; -S-CF₂H; -S-CH₂-CF₃; -S-CF₂-CF₃; -C(=O)-OCH₃; -C(=O)-OC₂H₅; Methyl, Ethyl, n-Propyl, Isopropyl, n-Butyl, Isobutyl, sec-Butyl, tert-Butyl, -CF₃, -CF₂H, -CFH₂, -CH₂-CF₃ und -CF₂-CF₃;
einen Rest, ausgewählt aus der Gruppe bestehend aus Naphthyl, [1,3]-Benzodioxolyl, [1,4]-Benzodioxanyl, Benzo[b]furanyl, Benzo[b]thiophenyl, Benzo[2,1,3]thiadiazolyl, [1,2,3]-Benzothiadiazolyl, [2,1,3]-Benzoxadiazolyl, [1,2,3]-Benzoxadiazolyl, Benzoxazolyl, Benzothiazolyl, Benzisoxazolyl, Benzisothiazolyl und Imidazo[2,1-b]thiazolyl, der unsubstituiert oder gegebenenfalls mit 1, 2, 3, 4 oder 5 Substituenten substituiert sein kann, unabhängig ausgewählt aus der Gruppe bestehend aus Methyl, Ethyl, n-Propyl, Isopropyl, n-Butyl, tert-Butyl, sec-Butyl, Isobutyl,
n-Pentyl, -O-CH₃, -O-C₂H₅, F, Cl, Br, I, -CN, -CF₃, -OCF₃, -SCF₃, -CF₂H und -CFH₂;
oder einen Rest stehen, ausgewählt aus der Gruppe bestehend aus Pyridinyl, Pyrrolyl, Imidazolyl, Pyrazolyl, Triazolyl, Pyridazinyl, Pyrimidinyl und Pyrazinyl, der unsubstituiert oder gegebenenfalls mit 1, 2, 3, 4 oder 5 Substituenten substituiert sein kann, unabhängig ausgewählt aus der Gruppe bestehend aus F, Cl, Br, I, -NO₂; Methyl, Ethyl, n-Propyl, Isopropyl, n-Butyl, Isobutyl, sec-Butyl, tert-Butyl, -CF₃, - CF₂H,
-CFH₂, -CH₂-CF₃ und -CF₂-CF₃;
und R^{37f} für einen Rest steht, ausgewählt aus der Gruppe bestehend aus Methyl, Ethyl, n-Propyl, Isopropyl, n-Butyl, sec-Butyl, Isobutyl, tert-Butyl, n-Pentyl, n-Hexyl, Fluorenyl, Fluorenylmethyl, Phenyl, Benzyl und Naphthyl;
gegebenenfalls in Form eines ihrer Stereoisomere, bevorzugt Enantiomere oder Diastereomere, eines Racemats oder in Form eines Gemisches aus mindestens zwei ihrer Stereoisomere, bevorzugt Enantiomere und/oder Diastereomere, in jeglichem Mischungsverhältnis, oder eines Salzes davon oder eines entsprechenden Solvats davon;
und Verbindungen der allgemeinen Formel (Ih) 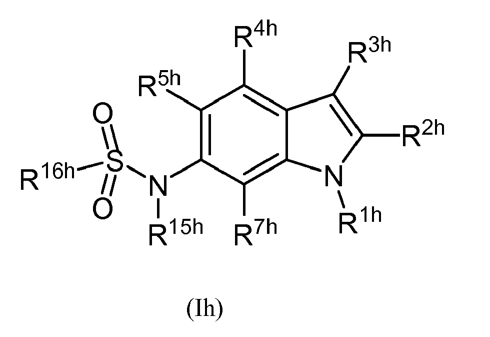 wobei
R^{1h} für ein Wasserstoffatom oder einen -(CH₂)ₘₕ-NR^{13h}R^{14h} Rest steht,
R^{2h} und R^{7h} jeweils für Wasserstoff stehen;
R^{3h} für ein Wasserstoffatom, 1-Methyl-piperidin-4-yl oder eine -(CH₂)ₙₕ-NR^{13h}R^{14h} Einheit steht mit nh = 0, 1 oder 2,
R^{4h} für Chlor, Brom oder ein Wasserstoffatom steht,
R^{5h} für -C(=O)-O-C₂H₅ oder ein Wasserstoffatom steht,
R^{15h} für Wasserstoff oder eine -S(=O)₂-R^{16h} Einheit steht,
R^{13h} und R^{14h}, gleich oder verschieden, jeweils für Methyl, Ethyl, Isopropyl oder n-Propyl, stärker bevorzugt Methyl, stehen,
oder
R^{13h} und R^{14h}, zusammen mit dem Brücken-Stickstoffatom, einen 5- oder 6-gliedrigen heterocyclischen Ring bilden, stärker bevorzugt einen Pyrrolidinring oder einen Piperidinring bilden,
und
R^{16h} für einen Aryl- oder Heteroarylrest steht, ausgewählt aus der Gruppe bestehend aus Phenyl, Naphthyl, Thiophenyl, Benzo[b]furanyl, Benzo[b]thiophenyl und Imidazo[2,1-b]thiazolyl, der mit 1, 2 oder 3 Substituenten substituiert sein kann, ausgewählt aus der Gruppe bestehend aus Chlor, Methyl Phenyl und -O-Phenyl und/oder der über einen C₁₋₂-Alkyleneinheit gebunden sein kann,
und mh gleich 0, 1 oder 2 ist,
gegebenenfalls in Form eines ihrer Stereoisomere, bevorzugt Enantiomere oder Diastereomere, eines Racemats oder in Form eines Gemisches aus mindestens zwei ihrer Stereoisomere, bevorzugt Enantiomere und/oder Diastereomere, in jeglichem Mischungsverhältnis, oder eines Salzes davon, bevorzugt eines entsprechenden, physiologisch verträglichen Salzes davon, oder eines entsprechenden Solvats davon;
und Verbindungen der allgemeinen Formel (Ik) 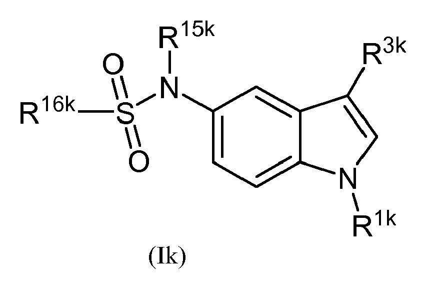 wobei
nk für 0, 1, 2, 3 oder 4 steht;
R^{1k} für Wasserstoff steht,
R^{3k} für eine -NR^{13k}R^{14k} Einheit oder eine Einheit, ausgewählt aus der Gruppe bestehend aus 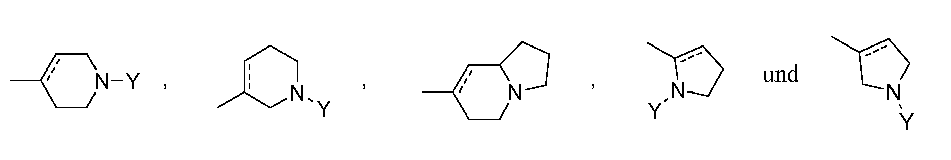 steht, wobei, falls vorhanden, die gestrichelte Linie für eine optionale chemische Bindung steht und Y für Wasserstoff, eine Methylgruppe oder eine Ethylgruppe steht,
R^{15k} für Wasserstoff, eine Methylgruppe oder eine Ethylgruppe steht,
R^{13k} und R^{14k}, gleich oder verschieden, für eine Methylgruppe, eine Ethylgruppe, eine n-Propylgruppe, eine Isopropylgruppe, eine n-Butylgruppe, eine Isobutylgruppe, eine sec-Butylgruppe oder eine tert-Butylgruppe stehen oder
R^{13k} und R^{14k} zusammen mit dem Brücken-Stickstoffatom eine Einheit, ausgewählt aus der Gruppe bestehend aus 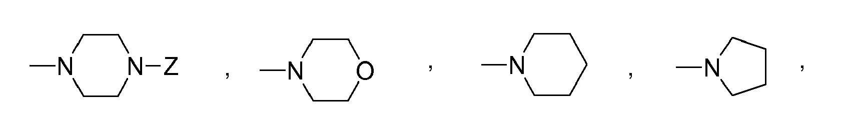 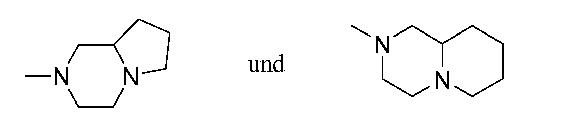 bilden, wobei Z für Wasserstoff, eine Methylgruppe oder eine Ethylgruppe steht,
R^{16k} für eine Einheit, ausgewählt aus der Gruppe bestehend aus 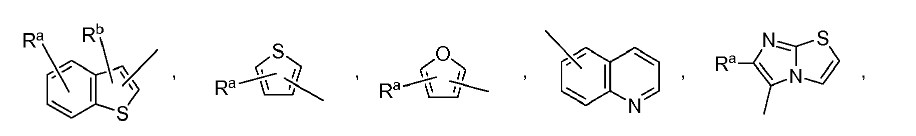 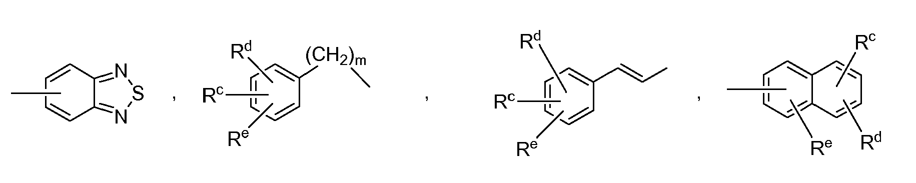 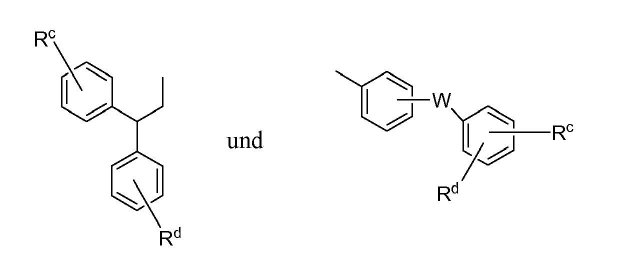 steht, wobei
R^{a} und R^{b} jeweils unabhängig ausgewählt sind aus der Gruppe bestehend aus Wasserstoff, Fluor, Chlor, Brom, Methyl, Ethyl, Pyridinyl, Thiophenyl und Furyl, R^{c}, R^{d} und R^{e} jeweils unabhängig ausgewählt sind aus der Gruppe bestehend aus Wasserstoff, Fluor, Chlor, Brom, Methyl, Ethyl, Methoxy, Ethoxy und -CF₃,
W für eine einfache chemische Bindung zwischen den zwei Ringen, eine CH₂-Gruppe, O, S oder eine NR^{f}-Einheit steht, wobei R^{f} Wasserstoff, Methyl oder Ethyl ist,
m gleich 0, 1, 2, 3 oder 4 ist;
gegebenenfalls in Form eines ihrer Stereoisomere, bevorzugt Enantiomere oder Diastereomere, eines Racemats oder in Form eines Gemisches aus mindestens zwei ihrer Stereoisomere, bevorzugt Enantiomere und/oder Diastereomere, in jeglichem Mischungsverhältnis, oder eines Salzes davon, bevorzugt eines entsprechenden, physiologisch verträglichen Salzes davon, oder eines entsprechenden Solvats davon,
und
(B) Memantin.

2. Die Kombination gemäß Anspruch 1, **dadurch gekennzeichnet, dass** die Bindung der Verbindungen, die als Komponente (A) vorliegen, an den 5-HT₆-Rezeptor durch einen Kᵢ-Wert von unter 7000 nM, bevorzugt unter 200 nM, stärker bevorzugt unter 100 nM, bestimmt ist.

3. Die Kombination gemäß einem der Ansprüche 1 bis 2, **dadurch gekennzeichnet, dass** als Komponente (A) mindestens eine Verbindung vorliegt, die ausgewählt ist aus
[788] N-[1-(2-Dimethylaminoethyl)-1H-indol-6-yl]-5-chlor-3-methylbenzo[b]thiophen-2-sulfonamid,
[789] N-[1-(2-Dimethylaminoethyl)-1H-indol-6-yl]-naphthalin-2-sulfonamid,
[790] N-[1-(2-Dimethylaminoethyl)-1H-indol-6-yl]-naphthalin-1-sulfonamid,
[791] N-[1-(2-Dimethylaminoethyl)-1H-indol-6-yl]-6-chlorimidazo[2,1-b]thiazol-5-sulfonamid,
[792] N-[1-(2-Dimethylaminoethyl)-1H-indol-6-yl]-4-phenylbenzolsulfonamid,
[793] N-[1-(2-Dimethylaminoethyl)-1H-indol-6-yl]-2-(naphthalin-1-yl)-ethansulfonamid,
[794] N-[1-(2-Dimethylaminoethyl)-1H-indol-6-yl]-4-phenoxybenzolsulfonamid,
[795] N-[1-(2-Dimethylaminoethyl)-1H-indol-6-yl]-3,5-dichlorbenzolsulfonamid,
[796] 5-Chlor-3-methyl-N-[1-[2-(pyrrolidin-1-yl)ethyl-1H-indol-6-yl]-benzo [b]thiophen-2-sulfonamid,
[797] N-(1-[2-(Pyrrolidin-1-yl)ethyl]-lH-indol-6-yl]-naphthyl-2-sulfonamid,
[798] N-[1-[2-Pyrrolidin-1-yl]ethyl]-1H-indol-6-yl]-naphthalin-1-sulfonamid,
[799] 6-Chlor-N-[1-[2-(pyrrolidin-1-yl)ethyl]-1H-indol-6-yl]-imidazo[2,1-b]thiazol-5-sulfonamid,
[800] 4-Phenyl-N-(1-(2-(pyrrolidin-1-yl)ethyl)-1H-indol-6-yl)-benzolsulfonamid,
[801] 2-(Naphth-1-yl)-N-(1-(2-(pyrrolidin-1-yl)ethyl)-1H-indol-6-yl)-ethansulfonamid,
[802] 4-Phenoxy-N-(1-(2-(pyrrolidin-1-yl)ethyl)-1H-indol-6-yl)-benzolsulfonamid,
[803] 3,5-Dichlor-N-(1-(2-(pyrrolidin-1-yl)-1H-indol-6-yl)-benzolsulfonamid,
[804] 5-Chlor-N-(3-(2-(diethylamino)ethyl)-1H-indol-6-yl)-3-methylbenzo[b]thiophen-2-sulfonamid,
[805] N-(3-(2-(Diethylamino)ethyl)-1H-indol-6-yl)naphthalin-2-sulfonamid,
[806] N-(3-(2-(Diethylamino)ethyl)-1H-indol-6-yl)naphthalin-1-sulfonamid,
[807] 6-Chlor-N-(3-(2-(diethylamino)ethyl)-1H-indol-6-yl)imidazo[2,1-b]thiazol-5-sulfonamid,
[808] N-(3-(2-(Diethylamino)ethyl)-1H-indol-6-yl)-4-phenylbenzolsulfonamid,
[809] N-(3-(2-(Diethylamino)ethyl)-1H-indol-6-yl)-4-phenoxybenzolsulfonamid,
[810] 3,5-Dichlor-N-(3-(2-(diethylamino)ethyl)-1H-indol-6-yl)benzolsulfonamid,
[811] 4,5-Dichlor-N-(3-(2-(diethylamino)ethyl)-1H-indol-6-yl)thiophen-2-sulfonamid,
[812] 5-Chlor-N-(3-(2-(diethylamino)ethyl)-1H-indol-6-yl)naphthalin-1-sulfonamid,
[813] 5-Chlor-N-(3-(2-(dimethylamino)ethyl)-1H-indol-6-yl)-3-methylbenzo[b]thiophen-2-sulfonamid,
[814] N-(3-(2-(Dimethylamino)ethyl)-1H-indol-6-yl)naphthalin-2-sulfonamid,
[815] N-(3-(2-(Dimethylamino)ethyl)-1H-indol-6-yl)naphthalin-1-sulfonamid,
[816] 6-Chlor-N-(3-(2-(dimethylamino)ethyl)-1H-indol-6-yl)imidazo[2,1-b]thiazol-5-sulfonamid,
[817] N-(3-(2-(Dimethylamino)ethyl)-1H-indol-6-yl)-4-phenylbenzolsulfonamid,
[818] N-(3-(2-(Dimethylamino)ethyl)-1H-indol-6-yl)-2-(naphthalin-1-yl)ethansulfonamid,
[819] N-(3-(2-(Dimethylamino)ethyl)-1H-indol-6-yl)-4-phenoxybenzolsulfonamid,
[820] 3,5-Dichlor-N-(3-(2-(dimethylamino)ethyl)-1H-indol-6-yl)benzolsulfonamid,
[821] 4,5-Dichlor-N-(3-(2-(dimethylamino)ethyl)-1H-indol-6-yl)thiophen-2-sulfonamid,
[822] 5-Chlor-N-(3-(2-(dimethylamino)ethyl)-1H-indol-6-yl)naphthalin-1-sulfonamid,
[823] 6-Bis(6-chlorimidazo[2,1-b]thiazol-5-ylsulfonyl)amino-3-(2-(dimethylamino)ethyl)-1H-indol,
[824] 6-Bis(3,5-dichlorbenzolsulfonyl)amino-3-(2-(dimethylamino)ethyl)-1H-indol,
[825] 6-Bis(4,5-dichlorthiophen-2-sulfonyl)amino-3-(2-(dimethylamino)ethyl)-1H-indol,
[826] 6-Bis(5-chlor-3-methylbenzo[b]thiophen-2-sulfonyl)amino-3-(2-(dimethylamino)-1-ethoxyethyl)-1H-indol,
[827] Ethyl-6-(5-chlor-3-methylbenzo[b]thiophen-2-sulfonamid)-3-(1-methylpiperidin-4-yl)-1H-indol-5-carboxylat,
[828] N-(4-Brom-3-(1-methylpiperidin-4-yl)-1H-indol-6-yl)naphthalin-1-sulfonamid,
[829] N-(7-Brom-3-(2-(dimethylamino)ethyl)-1H-indol-5-yl)benzofuran-2-sulfonamid,
[830] N-(7-Methoxy-3-(2-(pyrrolidin-1-yl)ethyl)-1H-indol-5-yl)benzo[c][1,2,5]thiadiazol-4-sulfonamid,
[831] N-(7-Methoxy-3-(2-(pyrrolidin-1-yl)ethyl)-1H-indol-5-yl)naphthalin-2-sulfonamid,
[832] 6-Chlor-N-(7-methoxy-3-(2-(pyrrolidin-1-yl)ethyl)-1H-indol-5-yl)imidazo[2,1-b]thiazol-5-sulfonamid,
[833] N-[3-(2-Diethylaminoethyl)-1H-indol-5-yl]-5-chlor-3-methylbenzo[b]thiophen-2-sulfonamid,
[834] N-[3-(2-Diethylaminoethyl)-1H-indol-5-yl]naphthalin-1-sulfonamid,
[835] N-[3-(2-Diethylaminoethyl)-1*H*-indol-5-yl]naphthalin-1-sulfonamid-hydrochlorid,
[836] N-[3-(2-Diethylaminoethyl)-1*H* indol-5-yl]-3,5-dichlorbenzolsulfonamid,
[837] N-[3-(2-Diethylaminoethyl)-1*H*-indol-5-yl]-4-phenylbenzolsulfonamid,
[838] N-[3-(2-Diethylaminoethyl)-1*H*-indol-5-yl]-5-chlorthiophen-2-sulfonamid,
[839] N-[3-(2-Dimethylaminoethyl)-1*H*-indol-5-yl]-5-chlor-3-methylbenzo[b]thiophen-2-sulfonamid,
[840] N-[3-(2-Dimethylaminoethyl)-1*H*-indol-5-yl]naphthalin-1-sulfonamid,
[841] N-[3-(2-Dimethylamino-ethyl)-1*H*-indol-5-yl]-6-chlorimidazo[2,1-b]thiazol-5-sulfonamid,
[842] N-[3-(1-Methylpiperidin-4-yl)-1*H*-indol-5-yl]-5-chlor-3-methylbenzo[b]thiophen-2-sulfonamid,
[843] N-[3-(1-Methylpiperidin-4-yl)-1*H*-indol-5-yl]-5-chlor-3-methylbenzo[b]thiophen-2-sulfonamid-hydrochlorid,
[844] N-[3-(1-Methylpiperidin-4-yl)-1*H*-indol-5-yl]naphthalin-1-sulfonamid,
[845] N-[3-(1-Methylpiperidin-4-yl)-1*H*-indol-5-yl]naphthalin-1-sulfonamid-hydrochlorid,
[846] N-[3-(1-Methylpiperidin-4-yl)-1*H*-indol-5-yl]-5-chlorthiophen-2-sulfonamid,
[847] N-[3-(1-Methylpiperidin-4-yl)-1*H* indol-5-yl]-4-phenylbenzolsulfonamid,
[848] N-[3-(1-Methylpiperidin-4-yl)-1*H*indol-5-yl]chinolin-8-sulfonamid,
[849] N-[3-(2-Diethylaminoethyl)-1*H*-indol-5-yl]naphthalin-2-sulfonamid,
[850] N-[3-(1-Methyl-1,2,3,6-tetrahydropyridin-4-yl)-1*H*-indol-5-yl]naphthalin-l-sulfonamid,
[851] N-[3-(4-Methylpiperazin-1-yl)methyl-1*H*-indol-5-yl]-5-chlor-3-methylbenzo[b]thiophen-2-sulfonamid,
[852] N-[3-(2-Dimethylaminoethyl)-1*H*-indol-5-yl]-5-(2-pyridil)thiophen-2-sulfonamid,
[853] N-[3-(2-Dimethylaminoethyl)-1*H*-indol-5-yl]-2,1,3-benzothiadiazol-4-sulfonamid,
[854] N-[3-(2-Dimethylaminoethyl)-1*H*-indol-5-yl]chinolin-8-sulfonamid,
[855] N-[3-(2-Dimethylaminoethyl)-1*H*-indol-5-yl]-5-chlornaphthalin-2-sulfonamid,
[856] N-[3-(2-Dimethylaminoethyl)-1*H*-indol-5-yl]-4-phenoxybenzolsulfonamid,
[857] N-[3-(2-Dimethylaminoethyl)-1*H*-indol-5-yl]-4-phenylbenzolsulfonamid,
[858] N-[3-(2-Diethylaminoethyl)-1*H*-indol-5-yl]-N-ethyl-naphthalin-2-sulfonamid,
[859] N-{3-[2-(Morpholin-4-yl)ethyl]-1H-indol-5-yl}-5-chlor-3-methylbenzo[b]thiophen-2-sulfonamid,
[860] N-{3-[2-(Morpholin-4-yl)ethyl]-1H-indol-5-yl}naphthalin-1-sulfonamid,
[861] N-[3-(2-Diethylaminoethyl)-1H-indol-5-yl]naphthalin-2-sulfonamid,
[862] N-[3-Dimethylaminomethyl-1H-indol-5-yl]-5-chlor-3-methylbenzo[b]thiophen-2-sulfonamid,
[863] N-[3-(2-Dipropylaminoethyl)-1H-indol-5-yl]naphthalin-1-sulfonamid,
[864] N-[3-(2-Dipropylaminoethyl)-1H-indol-5-yl]-5-chlor-3-methylbenzo[b]thiophen-2-sulfonamid,
[865] N-[3-(2-Dibutylaminoethyl)-1H-indol-5-yl]-5-chlor-3-methylbenzo[b]thiophen-2-sulfonamid,
[866] N-[3-(2-Dibutylaminoethyl)-1H-indol-5-yl]naphthalin-l-sulfonamid,
[867] N-[3-(2-Diethylaminoethyl)-1H-indol-5-yl]-5-chlornaphthalin-1-sulfonamid,
[868] N-[3-(2-Diethylaminoethyl)-1H-indol-5-yl]-trans-β-styrolsulfonamid,
[869] N-[3-(4-Methylpiperazin-1-yl)methyl-1*H*-indol-5-yl]-trans-β-styrolsulfonamid,
[870] N-[3-(Octahydroindolizin-7-yl)-1*H*-indol-5-yl]-5-chlor-3-methylbenzo[b]thiophen-2-sulfonamid,
[871] N-[3-(2-Diethylaminoethyl)-1*H* indol-5-yl]-6-chlorimidazo[2,1-b]thiazol-5-sulfonamid,
[872] N-{3-[2-(Morpholin-4-yl)ethyl]-1H-indol-5-yl}naphthalin-2-sulfonamid,
[873] N-[3-(4-Methylpiperazin-1-yl)methyl-1*H*-indol-5-yl]-α-toluolsulfonamid,
[874] N-[3-(3-Diethylaminopropyl)-1H-indol-5-yl]naphthalin-2-sulfonamid,
[875] N-[3-(3-Diethylaminopropyl)-1H-indol-5-yl]-5-chlor-3-methylbenzo[b]thiophen-2-sulfonamid,
[876] N-{3-[2-(Pyrrolidin-1-yl)ethyl]-lH-indol-5-yl}-5-chlor-3-methylbenzo[b]thiophen-2-sulfonamid,
[877] N- {3-[2-(Pyrrolidin-1-yl)ethyl]-1H-indol-5-yl}naphthalin-1-sulfonamid,
[878] N-{3-[2-(Pyrrolidin-1-yl)ethyl]-1H-indol-5-yl}naphthalin-2-sulfonamid,
[879] N-[3-(2-Dipropylaminoethyl)-1H-indol-5-yl]naphthalin-2-sulfonamid,
[880] N-[3-(2-Dimethylaminoethyl)-1H-indol-5-yl]-5-chlornaphthalin-1-sulfonamid,
[881] N-[3-(2-Dimethylaminoethyl)-1H-indol-5-yl]naphthalin-2-sulfonamid,
[882] N-{3-[2-(Morpholin-4-yl)ethyl]-1H-indol-5-yl}chinolin-8-sulfonamid,
[883] N-{3-[2-(Morpholin-4-yl)ethyl]-1H-indol-5-yl}-4-phenylbenzolsulfonamid,
[884] N-[3-(4-Methylpiperazin-1-yl)ethyl-1*H*-indol-5-yl]naphthalin-2-sulfonamid,
[885] N-[3-(4-Methylpiperazin-1-yl)ethyl-1*H*-indol-5-yl]-5-chlornaphthalin-1-sulfonamid,
[1114] N-(1,2,3,4-Tetrahydroisochinolin-6-yl)naphthalin-1-sulfonamid-hydrochlorid,
[1115] 2,2-Dimethyl-6-(N-methylnaphthalin-1-sulfonamid)-1,2,3,4-tetrahydroiso chino linium-io did,
[1116] N-(2-Methyl-1,2,3,4-tetrahydroisochinolin-6-yl)naphthalin-1-sulfonamid-hydrochlorid,
[1117] 5-Chlor-3-methyl-N-(1,2,3,4-tetrahydroisochinolin-6-yl)benzo[b]thiophen-2-sulfonamid-hydrochlorid,
[1118] 5-Chlor-3-methyl-N-(2-methyl-1,2,3,4-tetrahydroisochinolin-6-yl)benzo[b]thiophen-2-sulfonamid-hydrochlorid,
[1119] 4-Methyl-N-(1,2,3,4-tetrahydroisochinolin-6-yl)naphthalin-1-sulfonamid-hydrochlorid,
[1120] 4-Methyl-N-(2-methyl-1,2,3,4-tetrahydroisochinolin-6-yl)naphthalin-1-sulfonamid-hydrochlorid,
[1121] N-(1,2,3,4-Tetrahydroisochinolin-6-yl)naphthalin-2-sulfonamid-hydrochlorid,
[1122] N-(2-Methyl-1,2,3,4-tetrahydroisochinolin-6-yl)naphthalin-2-sulfonamid-hydrochlorid,
[1123] 6-Chlor-N-(1,2,3,4-tetrahydroisochinolin-6-yl)imidazo[2,1-b]thiazol-5-sulfonamid-hydrochlorid,
[1124] 2-Methoxy-5-methyl-N-(1,2,3,4-tetrahydroisochinolin-6-yl)benzolsulfonamid-hydrochlorid,
[1125] N-(1,2,3,4-Tetrahydroisochinolin-6-yl)pyridin-3-sulfonamid-dihydrochlorid,
[1126] 6-(Naphthalin-1-sulfonylamino)-3,4-dihydro-1H-isochinolin-2-carbonsäure-tert-butylester,
[1127] 6-(5-Chlor-3-methyl-benzo[b]thiophen-2-sulfonylamino)-3,4-dihydro-1H-isochinolin-2-carbonsäure-tert-butylester,
[1128] 6-(4-Methyl-naphthalin-1-sulfonylamino)-3,4-dihydro-1H-isochinolin-2-carbonsäure-tert-butylester,
[1129] 6-(Naphthalin-2-sulfonylamino)-3,4-dihydro-1H-isochinolin-2-carbonsäure-tert-butylester,
[1130] 6-(2-Methoxy-5-methyl-benzolsulfonylamino)-3,4-dihydro-1H-isochinolin-2-carbonsäure-tert-butylester und
[1131] 6-(Pyridin-3-sulfonylamino)-3,4-dihydro-1H-isochinolin-2-carbonsäure tert-butylester; gegebenenfalls in Form eines ihrer Stereoisomere, bevorzugt Enantiomere oder Diastereomere, eines Racemats oder in Form eines Gemisches aus mindestens zwei ihrer Stereoisomere, bevorzugt Enantiomere und/oder Diastereomere, in jeglichem Mischungsverhältnis, oder eines Salzes davon oder eines entsprechenden Solvats davon.

4. Die Kombination gemäß Anspruch 3, **dadurch gekennzeichnet, dass** als Komponente (A) mindestens eine Verbindung vorliegt, die ausgewählt ist aus
[816] 6-Chlor-N-(3-(2-(dimethylamino)ethyl)-1H-indol-6-yl)imidazo[2,1-b]thiazol-5-sulfonamid,
[841] N-[3-(2-Dimethylamino-ethyl)-1H-indol-5-yl]-6-chlorimidazo[2,1-b]thiazol-5-sulfonamid und
[1123] 6-Chlor-N-(1,2,3,4-tetrahydroisochinolin-6-yl)imidazo[2,1-b]thiazol-5-sulfonamid-hydrochlorid;
gegebenenfalls in Form eines ihrer Stereoisomere, bevorzugt Enantiomere oder Diastereomere, eines Racemats oder in Form eines Gemisches aus mindestens zwei ihrer Stereoisomere, bevorzugt Enantiomere und/oder Diastereomere, in jeglichem Mischungsverhältnis, oder eines Salzes davon oder eines entsprechenden Solvats davon.

5. Die Kombination gemäß einem oder mehreren der Ansprüche 1 bis 4, **dadurch gekennzeichnet, dass** sie 1 - 99 Gew.-% der Komponente (A) und 99 - Gew.-% der Komponente (B) umfasst, stärker bevorzugt 10 - 80 Gew.-% der Komponente (A) und 80 - 20 Gew.-% der Komponente (B), jeweils bezogen auf das Gesamtgewicht der beiden Komponenten (A) und (B).

6. Eine Kombination wie in einem oder mehreren der Ansprüche 1 bis 5 definiert zur Verwendung als Medikament.

7. Eine Kombination wie in einem oder mehreren der Ansprüche 1 bis 5 definiert zur Verwendung als Medikament zum gleichzeitigen Inhibieren des NMDA-Rezeptors und Regulieren des 5-HT₆-Rezeptors.

8. Eine Kombination wie in einem oder mehreren der Ansprüche 1 bis 5 definiert zur Verwendung als Medikament zur Vorbeugung und/oder Behandlung von Störungen im Zusammenhang mit Nahrungsaufnahme, bevorzugt zur Vorbeugung und/oder Behandlung von Adipositas, Anorexie, Kachexie, Bulimie, Diabetes, bevorzugt Typ-II-Diabetes (nicht insulinabhängiger Diabetes Mellitus), oder zur Vorbeugung und/oder Behandlung von Störungen des Verdauungstrakts, bevorzugt des Reizdarmsyndroms, zur Vorbeugung und/oder Behandlung des metabolischen Syndroms, Störungen des peripheren Nervensystems, Störungen des Zentralnervensystems, Arthritis, Epilepsie, Angst, Panik, Depression, kognitiven Störungen, Erinnerungsstörungen, Herz-Kreislauf-Erkrankungen, senilen Demenzprozessen wie Alzheimer-, Parkinson- und/oder Huntington-Krankheit, Schizophrenie, Psychose, infantiler Hyperkinesie (ADHS, Aufmerksamkeitsdefizit/Hyperaktivitätsstörung), Schmerz, Hypertoniesyndrom, Entzündungserkrankungen, immunologischen Erkrankungen oder zur Verbesserung der Wahrnehmung.

9. Eine pharmazeutische Formulierung, **dadurch gekennzeichnet, dass** sie eine Wirkstoffkombination gemäß einem oder mehreren der Ansprüche 1 bis 5 und gegebenenfalls einen oder mehrere pharmakologisch verträgliche Adjuvans umfasst.

10. Die pharmazeutische Formulierung gemäß Anspruch 9, **dadurch gekennzeichnet, dass** sie in festen pharmazeutischen Formen wie Tabletten, Tabletten, Kautabletten, Kaugummis, Dragees, Kapseln, Suppositorien, Pulverpräparaten, transdermalen therapeutischen Systemen, transmucosalen therapeutischen Systemen, oder in flüssigen und halbflüssigen pharmazeutischen Formen wie Tropfen oder wie Saft, Sirup, Lösung, Emulsion, Suspension, bevorzugt in Form von Tabletten, Kapseln, Tropfen oder Lösung, vorliegt.

11. Die pharmazeutische Formulierung gemäß Anspruch 9, **dadurch gekennzeichnet, dass** sie in Form mehrfacher Teilchen, bevorzugt Mikrotabletten, Mikrokapseln, Mikrokugeln, Granulatkörnern, Kristallen oder Pellets, gegebenenfalls zu einer Tablette verdichtet, in eine Kapsel gefüllt oder in einer geeigneten Flüssigkeit suspendiert, vorliegt.

12. Die pharmazeutische Formulierung gemäß einem oder mehreren der Ansprüche 9 bis 11, **dadurch gekennzeichnet, dass** sie für orale, intravenöse, intramuskuläre, subkutane, intrathekale, epidurale, bukkale, sublinguale, pulmonale, rektale, transdermale, nasale oder intracerebroventikulare, bevorzugt orale oder intravenöse, Applikation ist.

13. Die pharmazeutische Formulierung gemäß einem oder mehreren der Ansprüche 9 bis 11, **dadurch gekennzeichnet, dass** mindestens eine der Komponenten der Wirkstoffkombination (A) oder (B) mindestens teilweise in Retardform vorliegt.

14. Die pharmazeutische Formulierung gemäß Anspruch 13, **dadurch gekennzeichnet, dass** das Medikament mindestens eine Beschichtung oder mindestens eine Matrix aufweist, umfassend mindestens ein Material, das die Wirkstofffreisetzung aufrechterhält.

15. Die pharmazeutische Formulierung gemäß Anspruch 14, **dadurch gekennzeichnet, dass** das Retardmaterial auf gegebenenfalls modifiziertem, wasserunlöslichem, natürlichem, semisynthetischem oder synthetischem Polymer oder einem natürlichen Wachs oder Fett oder Fettalkohol oder semisynthetischer oder synthetischer Fettsäure oder auf einem Gemisch aus mindestens zwei dieser vorstehend genannten Komponenten basiert.

16. Die pharmazeutische Formulierung gemäß Anspruch 15, **dadurch gekennzeichnet, dass** das wasserunlösliche Polymer auf einem Acrylharz, das bevorzugt ausgewählt ist aus der Gruppe aus Poly(meth)acrylaten, Poly(C₁₋₄)dialkylamino(C₁₋₄)alkyl(meth)acrylaten und/oder Copolymeren davon oder einem Gemisch aus mindestens zwei der vorstehend genannten Polymere basiert.

17. Die pharmazeutische Formulierung gemäß Anspruch 15, **dadurch gekennzeichnet, dass** die wasserunlöslichen Polymere Cellulosederivate, bevorzugt Alkylcellulose und noch mehr bevorzugt Ethylcellulose, oder Celluloseester sind.

18. Die pharmazeutische Formulierung gemäß Anspruch 15, **dadurch gekennzeichnet, dass** das Wachs s Carnaubawachs, Bienenwachs, Glycerinmonostearat, Glycerinmonobehenat, Glycerinditripalmitostearat, microkristallines Wachs oder ein Gemisch aus mindestens zwei dieser Komponenten ist.

19. Die pharmazeutische Formulierung gemäß einem oder mehreren der Ansprüche 15 bis 18, **dadurch gekennzeichnet, dass** Polymere in Kombination mit einem oder mehreren Weichmachern verwendet worden sind.

20. Die pharmazeutische Formulierung gemäß einem oder mehreren der Ansprüche 13 bis 19, **dadurch gekennzeichnet, dass** neben der Retardform mindestens eine der Wirkstoffkomponenten (A) oder (B) in nicht Retardform vorliegt.

## Revendications

1. Combinaison de substances actives qui comprend
(A) au moins un composé qui est sélectionné dans le groupe constitué par les composés de sulfonamide dérivés de la tétrahydroisoquinoline de formule générale (If) : 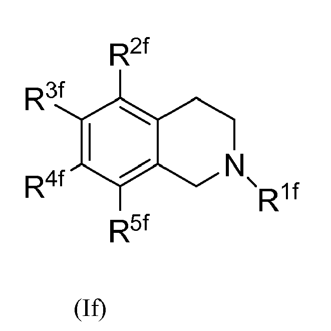 dans laquelle
R^{1f} représente un atome d'hydrogène ; un fragment -C(=O)-OR^{37f}; un radical sélectionné dans le groupe constitué par méthyle, éthyle, n-propyle, isopropyle, n-butyle, sec-butyle, isobutyle, tert-butyle, n-pentyle, n-hexyle, -CH₂-NH₂, - CH₂-NH-CH₃, -CH₂-N(CH₃)₂, -CH₂-N(C₂H₅)₂, -CH₂-NH-C₂H₅, -CH₂-CH₂-NH₂, -CH₂-CH₂-NH-CH₃, -CH₂-CH₂-N(CH₃)₂, -CH₂-CH₂-N(C₂H₅)₂, -CH₂-CH₂-NH-C₂H₅, -CH₂-CH₂-CH₂-NH-CH₃, -CH₂-CH₂-CH₂-N(CH₃)₂, -CH₂-CH₂-CH₂-N(C₂H₅)₂ et -CH₂-CH₂-CH₂-NH-C₂H₅ ; ou un radical (hétéro)cycloaliphatique sélectionné dans le groupe constitué par imidazolidinyle, aziridinyle, azétidinyle, pyrrolidinyle, pipéridinyle, morpholinyle, thiomorpholinyle, pipérazinyle, pyrazolidinyle et azépanyle, qui peut être lié via un groupe -(CH₂)₁, ₂ ou ₃- et qui peut être non substitué ou facultativement substitué par 1, 2, 3, 4 ou 5 substituants indépendamment sélectionnés dans le groupe constitué par méthyle, éthyle, n-propyle, isopropyle, n-butyle, tert-butyle, sec-butyle et isobutyle ;
R^{2f}, R^{3f}, R^{4f} et R^{5f}, indépendamment les uns des autres, représentent chacun un atome d'hydrogène ; F, Cl, Br, 1, -NO₂ ; -O-CH₃; -O-C₂H₅ ; -O-CF₃ ; -O-CFH₂ ; -O-CF₂H ; -O-CH₂-CF₃ ; -O-CF₂-CF₃ ; -S-CH₃ ; -S-C₂H₅ ; -S-CF₃ ; -S-CFH₂ ; - S-CF₂H ; -S-CH₂-CF₃ ; -S-CF₂-CF₃ ; -N(R^{11f})-S(=O)₂-R^{12f}; ou un radical sélectionné dans le groupe constitué par méthyle, éthyle, n-propyle, isopropyle, tert-butyle, -CF₃, -CFH₂, -CF₂H, -CH₂-CF₃ et -CF₂-CF₃ ;
à condition qu'au moins l'un des substituants R^{2f}, R^{3f}, R^{4f} et R^{5f} représente un groupement -N(R^{11f})-S(=O)₂-R^{12f} ;
R^{11f} représente un atome d'hydrogène, -S(=O)₂-R^{12f} ou un radical alkyle sélectionné dans le groupe constitué par méthyle, éthyle, n-propyle, isopropyle, n-butyle, sec-butyle, isobutyle et tert-butyle;
R^{12f} représente un radical phényle de formule générale (Af), 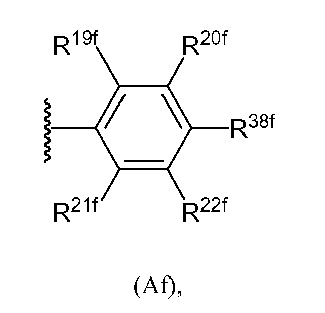 dans laquelle
R^{19f}, R^{20f}, R^{21f}, R^{22f} et R^{38f}, indépendamment les uns des autres, représentent chacun un atome d'hydrogène ; F, Cl, Br, I, -NO₂ ; -NH₂ ; -SH ; -OH ; -CN ; - C(=O)-OH ; -C(=O)-H ; -C(=O)-CH₃ ; -C(=O)-C₂H₅ ; -O-CH₃ ; -O-C₂H₅ ; -O-CF₃; -O-CFH₂; -O-CF₂H; -O-CH₂-CF₃; -O-CF₂-CF₃; -S-CH₃; -S-C₂H₅; -S-CF₃; -S-CFH₂; -S-CF₂H; -S-CH₂-CF₃; -S-CF₂-CF₃; -C(=O)-OCH₃ ; -C(=O)-OC₂H₅; méthyle, éthyle, n-propyle, isopropyle, n-butyle, isobutyle, sec-butyle, tert-butyle, -CF₃, -CF₂H, -CFH₂, -CH₂-CF₃ et -CF₂-CF₃;
un radical sélectionné dans le groupe constitué par naphtyle, [1,3]-benzodioxolyle, [1,4]-benzodioxanyle, benzo[b]furanyle, benzo[b]thiophényle, benzo[2,1,3]thiadiazolyle, [1,2,3]-benzothiadiazolyle, [2,1,3]-benzoxadiazolyle, [1,2,3]-benzoxadiazolyle, benzoxazolyle, benzothiazolyle, benzisoxazolyle, benzisothiazolyle et imidazo[2,1-b]thiazolyle, qui peut être non substitué ou facultativement substitué par 1, 2, 3, 4 ou 5 substituants indépendamment sélectionnés dans le groupe constitué par méthyle, éthyle, n-propyle, isopropyle, n-butyle, tert-butyle, sec-butyle, isobutyle, n-pentyle, -O-CH₃, -O-C₂H₅, F, Cl, Br, I, -CN, -CF₃, -OCF₃, -SCF₃, -CF₂H et -CFH₂ ;
ou un radical sélectionné dans le groupe constitué par pyridinyle, pyrrolyle, imidazolyle, pyrazolyle, triazolyle, pyridazinyle, pyrimidinyle et pyrazinyle, qui peut être non substitué ou facultativement substitué par 1, 2, 3, 4 ou 5 substituants sélectionnés indépendamment dans le groupe constitué par F, Cl, Br, I, -NO₂ ; méthyle, éthyle, n-propyle, isopropyle, n-butyle, isobutyle, sec-butyle, tert-butyle, -CF₃, -CF₂H, -CFH₂, -CH₂-CF₃ et -CF₂-CF₃ ;
et R^{37f} représente un radical sélectionné dans le groupe constitué par méthyle, éthyle, n-propyle, isopropyle, n-butyle, sec-butyle, isobutyle, tert-butyle, n-pentyle, n-hexyle, fluorényle, fluorénylméthyle, phényle, benzyle et naphtyle ;
facultativement sous la forme de l'un de ses stéréoisomères, de préférence les énantiomères ou les diastéréoisomères, d'un racémate ou sous la forme d'un mélange d'au moins deux de ses stéréoisomères, de préférence les énantiomères et/ou les diastéréoisomères, dans un quelconque rapport de mélange, ou d'un sel de ceux-ci, ou d'un produit de solvatation correspondant de ceux-ci ;
et des composés de formule générale (Ih) 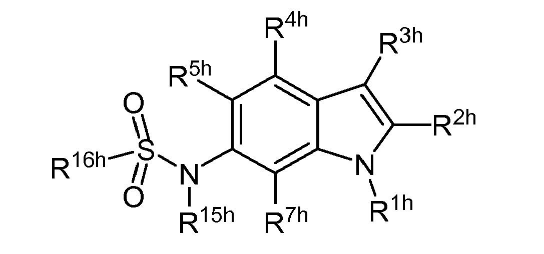 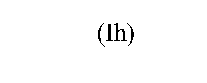 dans laquelle
R^{1h} représente un atome d'hydrogène ou un radical -(CH₂)ₘₕ-NR^{13h}R^{14h},
R^{2h} et R^{7h} représentent chacun l'hydrogène,
R^{3h} représente un atome d'hydrogène, 1-méthyl-pipéridin-4-yle ou un groupement -(CH₂)ₙₕ-NR^{13h}R^{14h} avec nh = 0, 1 ou 2,
R^{4h} représente le chlore, le brome ou un atome d'hydrogène,
R^{5h} représente -C(=O)-O-C₂H₅ ou un atome d'hydrogène,
R^{15h} représente l'hydrogène ou un groupement -S(=O)₂-R^{16h},
R^{13h} et R^{14h}, identiques ou différents, représentent chacun méthyle, éthyle, isopropyle ou n-propyle, plus préférablement méthyle,
ou
R^{13h} et R^{14h}, conjointement avec l'atome d'azote pontant, forment un cycle hétérocyclique à 5 ou 6 chaînons, plus préférablement forment un cycle pyrrolidine ou un cycle pipéridine,
et
R^{16h} représente un radical aryle ou hétéroaryle sélectionné dans le groupe constitué par phényle, naphtyle, thiophényle, benzo[b]furanyle, benzo[b]thiophényle and imidazo[2,1-b]thiazolyle qui peut être substitué par 1, 2 ou 3 substituants sélectionnés dans le groupe constitué par le chlore, méthyle, phényle et -O-phényle et/ou qui peut être lié via un groupe alkylène en C₁₋₂,
et mh vaut 0, 1 ou 2,
facultativement sous la forme de l'un de ses stéréoisomères, de préférence les énantiomères ou les diastéréoisomères, d'un racémate ou sous la forme d'un mélange d'au moins deux de ses stéréoisomères, de préférence les énantiomères et/ou les diastéréoisomères, dans un quelconque rapport de mélange, ou d'un sel de ceux-ci, de préférence un sel physiologiquement acceptable correspondant de ceux-ci, ou d'un produit de solvatation correspondant de ceux-ci ;
et des composés de formule générale (Ik) 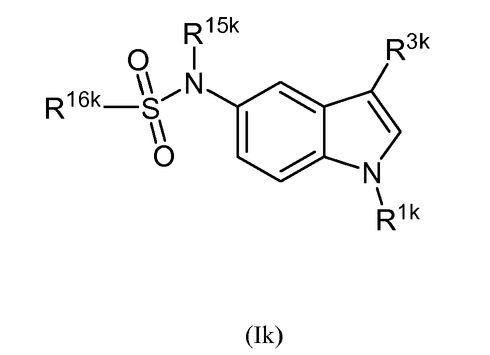 dans laquelle
nk représente 0, 1, 2, 3 ou 4 ;
R^{1k} représente l'hydrogène,
R^{3k} représente un groupement NR^{13k}R^{14k} ou un groupement sélectionné dans le groupe constitué par 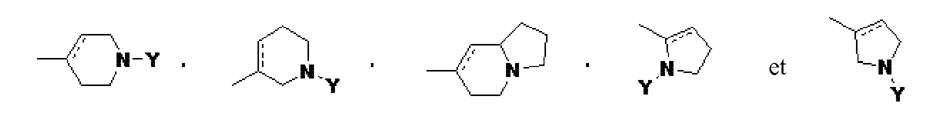
dans lequel, si elle est présente, la ligne en pointillés représente une liaison chimique facultative et Y représente l'hydrogène, un groupe méthyle ou un groupe éthyle,
R^{15k} représente l'hydrogène, un groupe méthyle ou un groupe éthyle,
R^{13k} et R^{14k}, identiques ou différents, représentent un groupe méthyle, un groupe éthyle, un groupe n-propyle, un groupe isopropyle, un groupe n-butyle, un groupe isobutyle, un groupe sec-butyle, ou un groupe tert-butyle, ou
R^{13k} et R^{14k}, conjointement avec l'atome d'azote pontant, forment un groupement sélectionné dans le groupe constitué par 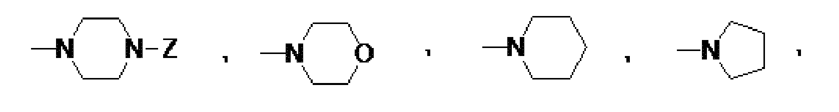 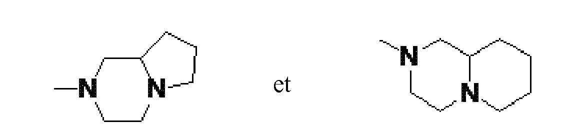 où Z représente l'hydrogène, un groupe méthyle ou un groupe éthyle,
R^{16k} représente un fragment sélectionné dans le groupe constitué par 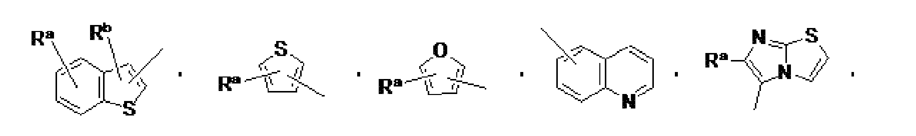 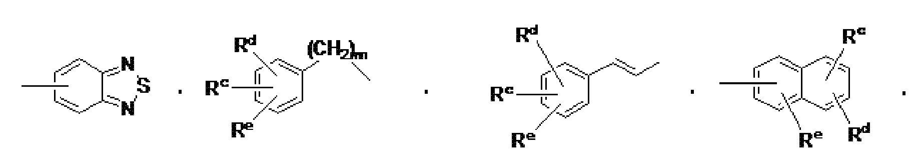 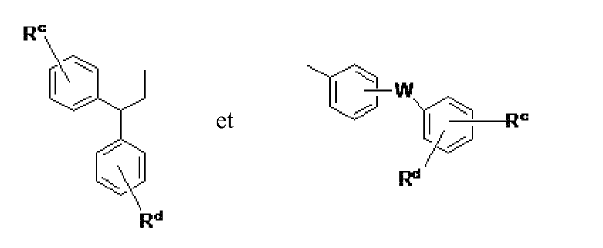
dans lequel
R^{a} et R^{b} sont chacun indépendamment sélectionnés dans le groupe constitué par l'hydrogène, le fluor, le chlore, le brome, méthyle, éthyle, pyridinyle, thiophényle et furyle,
R^{c}, R^{d} et R^{e} sont chacun indépendamment sélectionnés dans le groupe constitué par l'hydrogène, le fluor, le chlore, le brome, méthyle, éthyle, méthoxy, éthoxy et -CF₃,
W représente une liaison chimique simple entre les deux cycles, un groupe CH₂-, O, S ou un groupement NR^{f}-, dans lequel R^{f} est hydrogène, méthyle ou éthyle,
m vaut 0, 1, 2, 3 ou 4 ;
facultativement sous la forme de l'un de ses stéréoisomères, de préférence les énantiomères ou les diastéréoisomères, d'un racémate ou sous la forme d'un mélange d'au moins deux de ses stéréoisomères, de préférence les énantiomères et/ou les diastéréoisomères, dans un quelconque rapport de mélange, ou d'un sel de ceux-ci, de préférence un sel physiologiquement acceptable correspondant de ceux-ci, ou d'un produit de solvatation correspondant de ceux-ci,
et
(B) la mémantine.

2. Combinaison selon la revendication 1, **caractérisée en ce que** la liaison des composés présents en tant que composant (A) au récepteur 5-HT₆ est déterminée par une valeur Kᵢ inférieure à 7000 nM, de préférence inférieure à 200 nM, plus préférablement inférieure à 100 nM.

3. Combinaison selon l'une quelconque des revendications 1 à 2, **caractérisée en ce qu'**en tant que composant (A) au moins un composé est présent qui est sélectionné dans
[788] le N-[1-(2-diméthylaminoéthyl)-1H-indol-6-yl]-5-chloro-3-méthylbenzo[b]thiophène-2-sulfonamide,
[789] le N-[1-(2-diméthylaminoéthyl)-1H-indol-6-yl]-naphtalène-2-sulfonamide,
[790] le N-[1-(2-diméthylaminoéthyl)-1H-indol-6-yl]-naphtalène-1-sulfonamide,
[791] le N-[1-(2-diméthylaminoéthyl)-1H-indol-6-yl]-6-chloroimidazo[2,1-b]thiazole-5-sulfonamide,
[792] le N- 1-(2-diméthylaminoéthyl)-1H-indol-6-yl]-4-phénylbenzènesulfonamide,
[793] le N-[1-(2-diméthylaminoéthyl)-1H-indol-6-yl]-2-(naphtalène-1-yl)-éthanesulfonamide,
[794] le N-[1-(2-diméthylaminoéthyl)-1H-indol-6-yl]-4-phénoxybenzènesulfonamide,
[795] le N-[1-(2-diméthylaminoéthyl)-1H-indol-6-yl]-3,5-dichlorobenzènesulfonamide,
[796] le 5-chloro-3-méthyl-N-[1-[2-(pyrrolidin-1-yl)éthyl-1H-indol-6-yl]-benzo[b]thiophène-2-sulfonamide,
[797] le N-(1-[2-(pyrrolidin-1-yl)éthyl]-1H-indol-6-yl]-naphtyl-2-sulfonamide,
[798] le N-[1-[2-pyrrolidin-1-yl]éthyl]-1H-indol-6-yl]-naphtalène-1-sulfonamide,
[799] le 6-chloro-N-[1-[2-(pyrrolidin-1-yl)éthyl]-1H-indol-6-yl]-imidazo[2,1-b]thiazole-5-sulfonamide,
[800] le 4-phényl-N-(1-(2-(pyrrolidin-1-yl)éthyl)-1H-indol-6-yl)-benzènesulfonamide,
[801] le 2-(napht-1-yl)-N-(1-(2-(pyrrolidin-1-yl)éthyl)-1H-indol-6-yl)-éthanesulfonamide,
[802] le 4-phénoxy-N-(1-(2-(pyrrolidin-1-yl)éthyl)-1H-indol-6-yl)-benzènesulfonamide,
[803] le 3,5-dichloro-N-(1-(2-(pyrrolidin-1-yl)-1H-indol-6-yl)-benzènesulfonamide,
[804] le 5-chloro-N-(3-(2-(diéthylamino)éthyl)-1H-indol-6-yl)-3-méthylbenzo[b]thiophène-2-sulfonamide,
[805] le N-(3-(2-(diéthylamino)éthyl)-1H-indol-6-yl)naphtalène-2-sulfonamide,
[806] le N-(3-(2-(diéthylamino)éthyl)-1H-indol-6-yl)naphtalène-1-sulfonamide,
[807] le 6-chloro-N-(3-(2-(diéthylamino)éthyl)-1H-indol-6-yl)imidazo[2,1-b]thiazole-5-sulfonamide,
[808] le N-(3-(2-(diéthylamino)éthyl)-1H-indol-6-yl)-4-phénylbenzènesulfonamide,
[809] le N-(3-(2-(diéthylamino)éthyl)-1H-indol-6-yl)-4-phénoxybenzènesulfonamide,
[810] le 3,5-dichloro-N-(3-(2-(diéthylamino)éthyl)-1H-indol-6-yl)benzènesulfonamide,
[811] le 4,5-dichloro-N-(3-(2-(diéthylamino)éthyl)-1H-indol-6-yl)thiophène-2-sulfonamide,
[812] le 5-chloro-N-(3-(2-(diéthylamino)éthyl)-1H-indol-6-yl)naphtalène-1-sulfonamide,
[813] le 5-chloro-N-(3-(2-(diméthylamino)éthyl)-1H-indol-6-yl)-3-méthylbenzo[b]thiophène-2-sulfonamide,
[814] le N-(3-(2-(diméthylamino)éthyl)-1H-indol-6-yl)naphtalène-2-sulfonamide,
[815] le N-(3-(2-(diméthylamino)éthyl)-1H-indol-6-yl)naphtalène-1-sulfonamide,
[816] le 6-chloro-N-(3-(2-(diméthylamino)éthyl)-1H-indol-6-yl)imidazo[2,1-b]thiazole-5-sulfonamide,
[817] le N-(3-(2-(diméthylamino)éthyl)-1H-indol-6-yl)-4-phénylbenzènesulfonamide,
[818] le N-(3-(2-(diméthylamino)éthyl)-1H-indol-6-yl)-2-(naphtalène-1-yl)éthanesulfonamide,
[819] le N-(3-(2-(diméthylamino)éthyl)-1H-indol-6-yl)-4-phénoxybenzènesulfonamide,
[820] le 3,5-dichloro-N-(3-(2-(diméthylamino)éthyl)-1H-indol-6-yl)benzènesulfonamide,
[821] le 4,5-dichloro-N-(3-(2-(diméthylamino)éthyl)-1H-indol-6-yl)thiophène-2-sulfonamide,
[822] le 5-chloro-N-(3-(2-(diméthylamino)éthyl)-1H-indol-6-yl)naphtalène-1-sulfonamide,
[823] le 6-bis(6-chloroimidazo[2,1-b]thiazol-5-ylsulfonyl)amino-3-(2-(diméthylamino)éthyl)-1H-indole,
[824] le 6-bis(3,5-dichlorobenzènesulfonyl)amino-3-(2-(diméthylamino)éthyl)-1H-indole,
[825] le 6-bis(4,5-dichlorothiophène-2-sulfonyl)amino-3-(2-(diméthylamino)éthyl)-1H-indole,
[826] le 6-bis(5-chloro-3-méthylbenzo[b]thiophène-2-sulfonyl)amino-3-(2-(diméthylamino)-1-éthoxyéthyl)-1H-indole,
[827] le 6-(5-chloro-3-méthylbenzo[b]thiophène-2-sulfonamido)-3-(1-méthylpipéridin-4-yl)-1H-indole-5-carboxylate d'éthyle,
[828] le N-(4-bromo-3-(1-méthylpipéridin-4-yl)-1H-indol-6-yl)naphtalène-1-sulfonamide,
[829] le N-(7-bromo-3-(2-(diméthylamino)éthyl)-1H-indol-5-yl)benzofuran-2-sulfonamide,
[830] le N-(7-méthoxy-3-(2-(pyrrolidin-1-yl)éthyl)-1H-indol-5-yl)benzo[c][1,2,5]thiadiazole-4-sulfonamide,
[831] le N-(7-méthoxy-3-(2-(pyrrolidin-1-yl)éthyl)-1H-indol-5-yl)naphtalène-2-sulfonamide,
[832] le 6-chloro-N-(7-méthoxy-3-(2-(pyrrolidin-1-yl)éthyl)-1H-indol-5-yl)imidazo[2,1-b]thiazole-5-sulfonamide,
[833] le N-[3-(2-diéthylaminoéthyl)-1H-indol-5-yl]-5-chloro-3-méthylbenzo[b]thiophène-2-sulfonamide,
[834] le N-[3-(2-diéthylaminoéthyl)-1H-indol-5-yl]naphtalène-1-sulfonamide,
[835] le chlorhydrate de N-[3-(2-diéthylaminoéthyl)-1H-indol-5-yl]naphtalène-1-sulfonamide,
[836] le N-[3-(2-diéthylaminoéthyl)-1H-indol-5-yl]-3,5-dichlorobenzènesulfonamide,
[837] le N-[3-(2-diéthylaminoéthyl)-1H-indol-5-yl]-4-phénylbenzènesulfonamide,
[838] le N-[3-(2-diéthylaminoéthyl)-1H-indol-5-yl]-5-chlorothiophène-2-sulfonamide,
[839] le N-[3-(2-diméthylaminoéthyl)-1H-indol-5-yl]-5-chloro-3-méthylbenzo[b]thiophène-2-sulfonamide,
[840] le N-[3-(2-diméthylaminoéthyl)-1H-indol-5-yl]naphtalène-1-sulfonamide,
[841] le N-[3-(2-diméthylamino-éthyl)-1H-indol-5-yl]-6-chloroimidazo[2,1-b]thiazol-5-sulfonamide,
[842] le N-[3-(1-méthylpipéridin-4-yl)-1H-indol-5-yl]-5-chloro-3-méthylbenzo[b]thiophène-2-sulfonamide,
[843] le chlorhydrate de N-[3-(1-méthylpipéridin-4-yl)-1H-indol-5-yl]-5-chloro-3 méthylbenzo[b]thiophène-2-sulfonamide,
[844] le N-[3-(1-méthylpipéridin-4-yl)-1H-indol-5-yl]naphtalène-1-sulfonamide,
[845] le chlorhydrate de N-[3-(1-méthylpipéridin-4-yl)-1H-indol-5-yl]naphtalène-1-sulfonamide,
[846] le N-[3-(1-méthylpipéridin-4-yl)-1H-indol-5-yl]-5-chlorothiophène-2-sulfonamide,
[847] le N-[3-(1-méthylpipéridin-4-yl)-1H-indol-5-yl]-4-phénylbenzènesulfonamide,
[848] le N-[3-(1-méthylpipéridin-4-yl)-1H-indol-5-yl]quinoline-8-sulfonamide,
[849] le N-[3-(2-diéthylaminoéthyl)-1H-indol-5-yl]naphtalène-2-sulfonamide,
[850] le N-[3-(1-méthyl-1,2,3,6-tétrahydropyridin-4-yl)-1H-indol-5-yl]naphtalène-1-sulfonamide,
[851] le N-[3-(4-méthylpipérazin-1-yl)méthyl-1H-indol-5-yl]-5-chloro-3-méthylbenzo[b]thiophène-2-sulfonamide,
[852] le N-[3-(2-diméthylaminoéthyl)-1H-indol-5-yl]-5-(2-pyridil)thiophène-2-sulfonamide,
[853] le N-[3-(2-diméthylaminoéthyl)-1H indol-5-yl]-2,1,3-benzothiadiazol-4-sulfonamide,
[854] le N-[3-(2-diméthylaminoéthyl)-1H-indol-5-yl]quinoline-8-sulfonamide,
[855] le N-[3-(2-diméthylaminoéthyl)-1H-indol-5-yl]-5-chloronaphtalène-2-sulfonamide,
[856] le N-[3-(2-diméthylaminoéthyl)-1H-indol-5-yl]-4-phénoxybenzènesulfonamide,
[857] le N-[3-(2-diméthylaminoéthyl)-1H-indol-5-yl]-4-phénylbenzènesulfonamide,
[858] le N-[3-(2-diéthylaminoéthyl)-1H-indol-5-yl]-N-éthyl-naphtalène-2-sulfonamide,
[859] le N-{3-[2-(morpholin-4-yl)éthyl]-1H-indol-5-yl}-5-chloro-3-méthylbenzo[b]thiophène-2-sulfonamide,
[860] le N- {3-[2-(morpholin-4-yl)éthyl]-1H-indol-5-yl}naphtalène-1-sulfonamide,
[861] le N-[3-(2-diéthylaminoéthyl)-1H-indol-5-yl]naphtalène-2-sulfonamide,
[862] le N-[3-diméthylaminométhyl-1H-indol-5-yl]-5-chloro-3-méthylbenzo[b]thiophène-2-sulfonamide,
[863] le N-[3-(2-dipropylaminoéthyl)-1H-indol-5-yl]naphtalène-1-sulfonamide,
[864] le N-[3-(2-dipropylaminoéthyl)-1H-indol-5-yl]-5-chloro-3-méthylbenzo[b]thiophène-2-sulfonamide,
[865] le N-[3-(2-dibutylaminoéthyl)-1H-indol-5-yl]-5-chloro-3-méthylbenzo[b]thiophène-2-sulfonamide,
[866] le N-[3-(2-dibutylaminoéthyl)-1H-indol-5-yl]naphtalène-1-sulfonamide,
[867] le N-[3-(2-diéthylaminoéthyl)-1H-indol-5-yl]-5-chloronaphtalène-1-sulfonamide,
[868] le N-[3-(2-diéthylaminoéthyl)-1H-indol-5-yl]-trans-β-styrènesulfonamide,
[869] le N-[3-(4-méthylpipérazin-1-yl)méthyl-1H-indol-5-yl]-trans-β-styrènesulfonamide,
[870] le N-[3-(octahydroindolizin-7-yl)-1H-indol-5-yl]-5-chloro-3-méthylbenzo[b]thiophène-2-sulfonamide,
[871] le N-[3-(2-diéthylaminoéthyl)-1H-indol-5-yl]-6-chloroimidazo[2,1-b]thiazol-5-sulfonamide,
[872] le N-{3-[2-(morpholin-4-yl)éthyl]-1H-indol-5-yl}naphtalène-2-sulfonamide,
[873] le N-[3-(4-méthylpipérazin-1-yl)méthyl-1H-indol-5-yl]-a-toluènesulfonamide,
[874] le N-[3-(3-diéthylaminopropyl)-1H-indol-5-yl]naphtalène-2-sulfonamide,
[875] le N-[3-(3-diéthylaminopropyl)-1H-indol-5-yl]-5-chloro-3-méthylbenzo[b]thiophène-2-sulfonamide,
[876] le N- {3-[2-(pyrrolidin-1-yl)éthyl]-1H-indol-5-yl}-5-chloro-3-méthylbenzo[b]thiophène-2-sulfonamide,
[877] le N- {3-[2-(pyrrolidin-1-yl)éthyl]-1H-indol-5-yl}naphtalène-1-sulfonamide,
[878] le N- {3-[2-(pyrrolidin-1-yl)éthyl]-1H-indol-5-yl}naphtalène-2-sulfonamide,
[879] le N-[3-(2-dipropylaminoéthyl)-1H-indol-5-yl]naphtalène-2-sulfonamide,
[880] le N-[3-(2-diméthylaminoéthyl)-1H-indol-5-yl]-5-chloronaphtalène-1-sulfonamide,
[881] le N-[3-(2-diméthylaminoéthyl)-1H-indol-5-yl]naphtalène-2-sulfonamide,
[882] le N- {3-[2-(morpholin-4-yl)éthyl]-1H-indol-5-yl}quinoline-8-sulfonamide,
[883] le N- {3-[2-(morpholin-4-yl)éthyl]-1H-indol-5-yl}-4-phénylbenzènesulfonamide,
[884] le N-[3-(4-méthylpipérazin-1-yl)éthyl-1*H*-indol-5-yl]naphtalène-2-sulfonamide,
[885] le N-[3-(4-méthylpipérazin-1-yl)éthyl-1*H*-indol-5-yl]-5-chloronaphtalène-1-sulfonamide,
[1114] le chlorhydrate de N-(1,2,3,4-tétrahydroisoquinolin-6-yl)naphtalène-1-sulfonamide,
[1115] l'iodure de 2,2-diméthyl-6-(N-méthylnaphtalène-1-sulfonamido)-1,2,3,4-tétrahydroisoquinolinium,
[1116] le chlorhydrate de N-(2-méthyl-1,2,3,4-tétrahydroisoquinolin-6-yl)naphtalène-1-sulfonamide,
[1117] le chlorhydrate de 5-chloro-3-méthyl-N-(1,2,3,4-tétrahydroisoquinolin-6-yl)benzo[b]thiophène-2-sulfonamide,
[1118] le chlorhydrate de 5-chloro-3-méthyl-N-(2-méthyl-1,2,3,4-tétrahydroisoquinolin-6-yl)benzo[b]thiophène-2-sulfonamide,
[1119] le chlorhydrate de 4-méthyl-N-(1,2,3,4-tétrahydroisoquinolin-6-yl)naphtalène-1-sulfonamide,
[1120] le chlorhydrate de 4-méthyl-N-(2-méthyl-1,2,3,4-tétrahydroisoquinolin-6-yl)naphtalène-1-sulfonamide,
[1121] le chlorhydrate de N-(1,2,3,4-tétrahydroisoquinolin-6-yl)naphtalène-2-sulfonamide,
[1122] le chlorhydrate de N-(2-méthyl-1,2,3,4-tétrahydroisoquinolin-6-yl)naphtalène-2-sulfonamide,
[1123] le chlorhydrate de 6-chloro-N-(1,2,3,4-tétrahydroisoquinolin-6-yl)imidazo[2,1-b]thiazole-5-sulfonamide,
[1124] le chlorhydrate de 2-méthoxy-5-méthyl-N-(1,2,3,4-tétrahydroisoquinolin-6-yl)benzènesulfonamide,
[1125] le chlorhydrate de N-(1,2,3,4-tétrahydroisoquinolin-6-yl)pyridine-3-sulfonamide,
[1126] l'ester tert-butylique d'acide 6-(naphtalène-1-sulfonylamino)-3,4-dihydro-1H-isoquinoline-2-carboxylique,
[1127] l'ester tert-butylique d'acide 6-(5-chloro-3-méthyl-benzo[b]thiophène-2-sulfonylamino)-3,4-dihydro-1H-isoquinoline-2-carboxylique,
[1128] l'ester tert-butylique d'acide 6-(4-méthyl-naphtalène-1-sulfonylamino)-3,4-dihydro-1H-isoquinoline-2-carboxylique,
[1129] l'ester tert-butylique d'acide 6-(naphtalène-2-sulfonylamino)-3,4-dihydro-1H-isoquinoline-2-carboxylique,
[1130] l'ester tert-butylique d'acide 6-(2-méthoxy-5-méthyl-benzènesulfonylamino)-3,4-dihydro-1H-isoquinoline-2-carboxylique et
[1131] l'ester tert-butylique d'acide 6-(pyridine-3-sulfonylamino)-3,4-dihydro-1H-isoquinoline-2-carboxylique;
facultativement sous la forme de l'un de ses stéréoisomères, de préférence les énantiomères ou les diastéréoisomères, d'un racémate ou sous la forme d'un mélange d'au moins deux de ses stéréoisomères, de préférence les énantiomères et/ou les diastéréoisomères, dans un quelconque rapport de mélange, ou d'un sel de ceux-ci, ou d'un produit de solvatation correspondant de ceux-ci.

4. Combinaison selon la revendication 3, caractérisée en qu'en tant que composant (A) au moins un composé est présent qui est sélectionné dans
[816] le 6-chloro-N-(3-(2-(diméthylamino)éthyl)-1H-indol-6-yl)imidazo[2,1-b]thiazole-5-sulfonamide,
[841] le N-[3-(2-diméthylamino-éthyl)-1H-indol-5-yl]-6-chloroimidazo[2,1-b]thiazol-5-sulfonamide, et
[1123] le chlorhydrate de 6-chloro-N-(1,2,3,4-tétrahydroisoquinolin-6-yl)imidazo[2,1-b]thiazole-5-sulfonamide ;
facultativement sous la forme de l'un de ses stéréoisomères, de préférence les énantiomères ou les diastéréoisomères, d'un racémate ou sous la forme d'un mélange d'au moins deux de ses stéréoisomères, de préférence les énantiomères et/ou les diastéréoisomères, dans un quelconque rapport de mélange, ou d'un sel de ceux-ci, ou d'un produit de solvatation correspondant de ceux-ci.

5. Combinaison selon l'une ou plusieurs des revendications 1 à 4, **caractérisée en ce qu'**elle comprend 1 - 99 % en poids du composant (A) et 99 - 1 % en poids du composant (B), plus préférablement 10 - 80 % en poids du composant (A) et 80 - 20 % en poids du composant (B), faisant dans chaque cas référence au poids total des deux composants (A) et (B).

6. Combinaison selon l'une ou plusieurs des revendications 1 à 5 destinée à être utilisée en tant que médicament.

7. Combinaison selon l'une ou plusieurs des revendications 1 à 5 destinée à être utilisée en tant que médicament pour l'inhibition des récepteurs NMDA et la régulation du récepteur 5-HT₆ simultanées.

8. Combinaison selon l'une ou plusieurs des revendications 1 à 5 destinée à être utilisée en tant que médicament pour la prophylaxie et/ou le traitement de troubles en rapport avec l'ingestion de nourriture, de préférence pour la prophylaxie et/ou le traitement de l'obésité, de l'anorexie, de la cachexie, de la boulimie, du diabète, de préférence le diabète type II (diabète non insulinodépendant), ou pour la prophylaxie et/ou le traitement de troubles gastro-intestinaux, de préférence du syndrome du côlon irritable, pour la prophylaxie et/ou le traitement du syndrome métabolique, des troubles du système nerveux périphérique, des troubles du système nerveux central, de l'arthrite, de l'épilepsie, de l'anxiété, de la panique, de la dépression, des troubles cognitifs, des troubles de la mémoire, des maladies cardiovasculaires, des processus de démence sénile, tels que la maladie d'Alzheimer, de Parkinson et/ou de Huntington, de la schizophrénie, de la psychose, de l'hyperkinésie infantile (TDAH, trouble de déficit d'attention avec hyperactivité), de la douleur, du syndrome d'hypertension, des maladies inflammatoires, des maladies immunologiques ou pour l'amélioration de la cognition.

9. Formulation pharmaceutique, **caractérisée en ce qu'**elle comprend une combinaison de substances actives selon l'une ou plusieurs des revendications 1 à 5 et facultativement un ou plusieurs adjuvants pharmacologiquement acceptables.

10. Formulation pharmaceutique selon la revendication 9, **caractérisée en ce qu'**elle se présente sous des formes pharmaceutiques solides telles que les comprimés, les comprimés à mâcher, les gommes à mâcher, les dragées, les gélules, les suppositoires, les préparations en poudre, les systèmes thérapeutiques transdermiques, les systèmes thérapeutiques transmuqueux, ou sous des formes pharmaceutiques liquides et semi-liquides telles que les gouttes ou telles qu'un jus, un sirop, une solution, une émulsion, une suspension, de préférence sous la forme de comprimés, de gélules, de gouttes ou d'une solution.

11. Formulation pharmaceutique selon la revendication 9, **caractérisée en ce qu'**elle se présente sous la forme de multiples particules, de préférence des microcomprimés, des microcapsules, des microsphéroïdes, des granulés, des cristaux ou des pastilles, facultativement compactées dans un comprimé, versées dans une gélule ou mises en suspension dans un liquide approprié.

12. Formulation pharmaceutique selon l'une ou plusieurs des revendications 9 à 11, **caractérisée en ce qu'**elle est destinée à une application orale, intraveineuse, intramusculaire, sous-cutanée, intrathécale, épidurale, buccale, sublinguale, pulmonaire, rectale, transdermique, nasale ou intracérébroventriculaire, de préférence orale ou intraveineuse.

13. Formulation pharmaceutique selon l'une ou plusieurs des revendications 9 à 11, **caractérisée en ce qu'**au moins l'un des composants de la combinaison de substances actives (A) ou (B) se présente au moins partiellement sous une forme à libération prolongée.

14. Formulation pharmaceutique selon la revendication 13, **caractérisée en ce que** le médicament a au moins un enrobage ou au moins une matrice comprenant au moins une matière qui supporte une libération de substances actives.

15. Formulation pharmaceutique selon la revendication 14, **caractérisée en ce que** la matière à libération prolongée est à base d'un polymère semi-synthétique ou synthétique naturel insoluble dans l'eau facultativement modifié, ou d'une cire naturelle ou d'une graisse ou d'un alcool gras ou d'un acide gras semi-synthétique ou synthétique, ou d'un mélange d'au moins deux des composants mentionnés ci-dessus.

16. Formulation pharmaceutique selon la revendication 15, **caractérisée en ce que** le polymère insoluble dans l'eau est à base d'une résine acrylique, qui est de préférence sélectionnée dans le groupe des poly(méth)acrylates, des poly(C₁₋₄)dialkylamino(C₁₋₄)alkyl(méth)acrylates et/ou des copolymères de ceux-ci ou d'un mélange d'au moins deux des polymères mentionnés ci-dessus.

17. Formulation pharmaceutique selon la revendication 15, **caractérisée en ce que** les polymères insolubles dans l'eau sont les dérivés de cellulose, de préférence la cellulose d'alkyle et encore plus préférablement la cellulose d'éthyle, ou les esters de cellulose.

18. Formulation pharmaceutique selon la revendication 15, **caractérisée en ce que** la cire est la cire de carnauba, la cire d'abeille, le monostéarate de glycérol, le monobéhénate de glycérol, le ditripalmitostéarate de glycérol, la cire microcristalline ou un mélange d'au moins deux de ces composants.

19. Formulation pharmaceutique selon l'une ou plusieurs des revendications 15 à 18, **caractérisée en ce que** des polymères ont été utilisés en association avec un ou plusieurs plastifiants.

20. Formulation pharmaceutique selon l'une ou plusieurs des revendications 13 à 19, **caractérisée en ce que**, outre la forme à libération prolongée, au moins l'un des composants à substances actives (A) ou (B) est présent sous une forme qui n'est pas à libération prolongée.
